(19) Europäisches Patentamt
European Patent Office
Office européen des brevets

(11) EP 1 671 962 A1

(12) **EUROPEAN PATENT APPLICATION**
published in accordance with Art. 158(3) EPC

(43) Date of publication:
**21.06.2006 Bulletin 2006/25**

(21) Application number: **04792210.9**

(22) Date of filing: **08.10.2004**

(51) Int Cl.:
***C07D 311/08** (1985.01)*
***C07D 487/04** (1985.01)*
***A61K 45/00** (1985.01)*
***A61K 31/519** (2000.01)*
***A61P 43/00** (2000.01)*
***A61P 3/10** (2000.01)*
***A61P 29/00** (2000.01)*
***A61P 19/08** (2000.01)*
***A61P 19/02** (2000.01)*

(86) International application number:
**PCT/JP2004/014941**

(87) International publication number:
**WO 2005/035516 (21.04.2005 Gazette 2005/16)**

(84) Designated Contracting States:
**AT BE BG CH CY CZ DE DK EE ES FI FR GB GR HU IE IT LI LU MC NL PL PT RO SE SI SK TR**

(30) Priority: **10.10.2003 JP 2003352585**
**21.01.2004 JP 2004013672**

(71) Applicant: **ONO PHARMACEUTICAL CO., LTD.**
**Osaka-shi,**
**Osaka 541-8526 (JP)**

(72) Inventors:
- **YOSHIZAWA, T.;**
**c/o ONO PHARMACEUTICAL CO., LTD.**
**Mishima-gun, Osaka 6188585 (JP)**
- **OGAWA, Koji;**
**c/o ONO PHARMACEUTICAL CO., LTD.**
**Mishima-gun, Osaka 6188585 (JP)**
- **FUJITA, Setsuko;**
**c/o ONO PHARMACEUTICAL CO., LTD.**
**Mishima-gun, Osaka 6188585 (JP)**
- **INOHARA, Takeo;**
**c/o ONO PHARMACEUTICAL CO., LTD.**
**Mishima-gun,Os aka 6188585 (JP)**
- **OHMOTO, K.;**
**c/o ONO PHARMACEUTICAL CO., LTD.**
**Mishima-gun, Osaka 6188585 (JP)**

(74) Representative: **Teipel, Stephan et al**
**Lederer & Keller**
**Patentanwälte**
**Prinzregentenstrasse 16**
**80538 München (DE)**

# (54) NOVEL FUSED HETEROCYCLIC COMPOUND AND USE THEREOF

(57) The compound represented by the general formula (I):

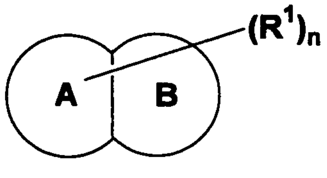

(I)

wherein, a fused ring AB represents a 5- to 10-membered fused heterocyclic ring; $R^1$ represents (1) a hydrogen atom, (2) a halogen atom, (3) a cyano group, (4) an oxo group, (5) an optionally protected hydroxyl group, (6) an optionally protected carboxyl group, (7) an optionally protected amino group, (8) a cyclic group which may have a substituent (s), (9) an aliphatic hydrocarbon group which may have a substituent (s), or (10) an optionally protected thiol group; n represents 0 or an integer of 1 to 8; provided that n represents an integer of not less than 2, plural $R^1$ are the same or different; a salt thereof, a solvate thereof or a prodrug thereof has a kinase(especially c-Jun N-terminalkinase) inhibitory activity and an inhibitory activity of a function of AP-1 as a transcription factor, it is useful as a preventive and/or therapeutic agent for a for example, a diabetes of metabolic disease, etc., a rheumatoid arthritis of inflammatory, etc.

EP 1 671 962 A1

## Description

## TECHNICAL FIELD

The present invention relates to a kinase inhibitor, especially c-Jun N-Terminal kinase inhibitor (hereinafter, this word sometimes may be abbreviated to JNK) which is useful as a medicament and use thereof.

## BACKGROUND ART

Protein kinase is an enzyme that catalyzes transfer of a phosphate group from adenosine triphosphate (ATP) to an amino-acid residue, for example, tyrosine, serine, threonine, and/or histidine in protein. More than 400 protein kinases have been identified until now. These protein kinases are essential for wide-ranging cell function, such as cell division, cell differentiation, cell death (apoptosis), alteration of cell motility and cytoskeleton structure, control of DNA replication, translation, splicing and translation, protein transportation from endoplasmic reticulum or Golgi apparatus to membrane or extracellular space, nuclear migration and nuclear exportation of protein, metabolic reaction.

Eukaryotic protein kinases can be classified into five groups based on their structural and functional features: (1) AGC group, such as cyclicnucleotide-dependent and phospholipid-dependent protein kinases , ribosome S6 kinase like PKA, PKG, PKC, (2) CaMK group, such as $Ca^{2+}$/calmodulin kinase like CaMK, (3) CMGC group, such as cyclin-dependent protein kinase like CDK, MAPK, GSK-3, (4) PTK group, such as protein tyrosine kinase like SRC, EGFR, (5) others, such as other kinase like MEK, Raf, TGFR, LIMK.

Above each group is classified based on its tropism for a substrate. For example, AGC and CaMK groups phosphorylate serine or threonine residue near arginine or lysine, and CMGC group phosphorylate serine/threonine residue in proline-rich domain. PTK group phosphorylates tyrosine residue. It is known that PTK group has two types, one is the receptor type which is a receptor of growth factor and the other is non-receptor type which is represented by oncogene product that is coded by RNA tumor virus. Another group can phosphorylate either serine/threonine residue or tyrosine residue, and some of them may be called as dual-specificity kinase because of their ability to phosphorylate both serine/ threonin and tyrosine residue.

As identified above, tyrosine protein kinase (PTK) can be classified as receptor PTKs and non-receptor PTKs. Sometimes receptor PTKs is called as receptor tyrosine kinase (RTK). Growth factor, such as Epidermal Growth Factor (EGF), transducts growth signals into intracellular by binding extracellular domain of RTKs to activate the RTKs. Overexpression of EGF and RTKs belonging to Epidermal Growth Factor Receptor (EGFR), such as EGF-r, erbB-2, erbB-3, erbB-4 is known to relate to genesis and progress of cancer [Rusch, V., Cytokine Growth Factor Rev. , 7, 133 (1996); Davies, D. E., Biochem. Pharmacol., 51, 1101 (1996) ; Modjtahedi, E. , Int. J. Oncol., 4, 277 (1994)] . As more concrete example, it has been reported that overexpression of erbB-2 gene product can be seen in breast and uterus cancer [Slamon, D. J. , Science, 244, 707 (1989) ; Slamon, D.J., Science, 235, 177 (1987)], increased activity of EGFR kinase can be seen in epidermoid carcinoma, breast cancer and tumor in other major tissue [Reiss, M., Cancer Res., 51, 6254 (1991); Macias, A., Anticancer Res. , 7, 459 (1987) ; Gullick, W. J. , Brit. Med. Bull. , 47, 87 (1991)] and the like. EGFR is suggested to relate to the cause of proliferation of epithelial cyst in polycystic kidney disease [Du, J., Amer. J. Physiol., 269 (2 Pt 1), 487 (1995); Nauta, J., Pediatric Res., 37 (6), 755 (1995); Gattone, V. H., Developmental Biology, 169 (2), 504 (1995); Wilson, P. D., Eur. J. Cell Biol., 61 (1), 131 (1993)].

RTKs also includes such as FGFR that is a receptor of Fibroblast Growth Factor, flk-1/KDR and flt-1 that is a receptor of Vascular Endothelial Growth Factor (VEGF), PDGFR that is a receptor of Platelet-Derived Growth Factor (PDGF). Tumor proliferation needs a new blood vessel formation, known as a vasculogenesis and also known that FGF and VEGF stimulate vasculogenesis. Tie-1, Tie-2, also a member of RTK involves vasculogenesis [Sato, T. N., Nature, 376, 70 (1995)] . Acompound that inhibits the kinase activity of PDGFR is said to be effective for therapy of tumor, because it is known that overexpression of PDGF and PDGFR can be seen in various human glioma [Nister, M., J. Biol. Chem., 266, 16755 (1991); Strawn, L. M., J. Biol. Chem., 269, 21215 (1994)]. It is known that examples of RTKs include insulin receptor, colon stimulated factor receptor, Nerve Growth Factor receptor (trkA, trkB and trkC), Insulin-like Growth Factor, Hepatocyte growth factor, erythropoietin-producing, hepatoma and the like beside above.

Non-receptor PTKs exist in cytoplasm and cell nucleus and play a role in various signal pathways. It is known that non-receptor PTKs include various kinds pf factors such as Abl, Jak, Fak, Syk, Zap-70, Csk. Among these, 8 kinds of Src family, that is, Src, Fyn, Lyn, Yes, Lck, Fgr, Hck, Blk are known for the most important signaling factors [Schwartzberg, P.L., Oncogene, 17, 1463 (1998)]. It has been shown that the expression of Src family kinases has elevated in tumor of breast, colon (~90%), spleen (>90%), and liver (>90%) and antisense oligonucleotide of Src inhibits colon tumor cell growth in nude mice [Staley, C.A., Cell Growth Differentiation, 8, 269 (1997)]. Furthermore, it has been reported that Src has a role in osteoclast function [Soriano, P., Cell, 64, 693 (1991); Boyce, B. F., J. Clin.,Invest., 90, 1622 (1992); Missbach, M., Bone, 24, 437 (1999)].

Lck and ZAP-70 are expressed mainly in T cell and natural killar (NK) cell and it is suggested that these non-receptor PTKs are essential for immune system [Myers, M., Current Pharm. Design, 3, 473 (1997)].

Protein kinases which phosphorylate serine/threonine residue include mitogen-activated protein (MAP) kinases. MAP kinase consists of two groups; stress-activated protein kinase (SAPK) and mitogen-activated protein kinase (MAPK). These kinases have roles in cell reaction caused by various kinds of stimulations, such as growth factors, chemical drugs, osmotic shock, radiation, bacterial infection, various cytokines. Above two groups of MAP kinases are activated by dual phosphorylation of Thr and Tyr site on Thr-Xaa-Tyr motif, which is catalyzed by upstream kinases like MAP kinase kinases and MEKs (MEKs) . MEKs are activated by more upstream kinases, MAP kinase kinase kinases that include more than 30 kinds. Twelve different kinds of MAP kinases have been identified in mammalian cells and these homologs are discovered in all eukaryotic cells. Among MAP kinases of mammalian cells, ERK1/2, p38 (SAPK), and c-Jun N terminal kinases are investigated most earnestly.

Cyclin-dependent kinases (cdks), such as cdc2/cyclin B, cdk2/cyclin A, cdk2/cyclin E and cdk4/cyclin D are serine/threonine kinase that controls cell-division of mammalian cell. It has been reported that the activation of these kinases is frequently observed in human tumor cell [Garrett, M. D., Current Opin. Genetics Devel., 9, 104 (1999); Webster, K. R., Exp. Opin. Invest. Drugs, 7, 865 (1998)].

Protein kinase A, B, and C are also important serine/threonine kinase. These kinases are sometimes called as PKA/cyclic AMP dependent protein kinase, PKB/Akt, PKC in order of mention.

As identified above, it is well known that malfunction of protein phosphorylation has a role in the etiology of many diseases, such as tumor, immunological disorder, nervous affection, metabolic disease. For example, it can be seen in many tumor cell that overexpression of RTK, such as EGFR and PDGFR or mutation of threonine kinase that can produce activated type constitutively [Druker, Nat. Med., 2, 561 (1996)]. Furthermore, defect of protein serine/threonine kinase gene is related to several kinds of diseases such as myotonic dystrophy, cancer, Alzheimer's disease [Sanpei, Biochem. Biophys. Res. Commun., 212, 341 (1995); Sperber, Neurosci. Lett., 197, 149 (1995) ; Grammas, Neurobiol. Aging, 16, 563 (1995); Govoni, Ann. N. Y. Acad. Sci., 777, 332 (1996)].

Therefore, protein kinase inhibitor is considered to be useful for therapy of disease which is based on disorder of protein phosphorylation as mentioned above. Among protein kinases, MAP kinases are related to various kinds of immunoreactions and inflammatory reaction in particular. Therefore, a drug that can inhibit activation of MAP kinases has possibility to be a therapeutic agent for very wide-ranging diseases.

As mentioned previously, it is known that a kind of MAP kinase, JNK is activated according to extracellular stimulation and transfer signal to downstream factor by phosphorylation of substrate protein such as c-Jun or ATF-2. In addition, c-Jun is known for a configuration factor of transcription factor AP-1, whose N terminal is phosphorylated specifically by JNK. Moreover, it is also known that this phosphorylation of c-Jun N terminal promotes transcriptional activity of AP-1 downstream gene [EMBO J., 15, 2760 (1996) ; Biochemica et Biophysica Acta, 1333, F85 (1997)]. It commands more attention in these days that substrates of JNK include not only these transcription factor but also important intracellular signaling factors. For example, it is reported that the activity of JNK is increased in liver, adipose skeletal muscle and the like of mouse made obese by a high-fat diet and ob/ob mouse and has a role in regulation of insulin reaction via serine phosphorylation of IRS-1 [Nature, 420(6913), 333 (2002)]. In addition, the same literature reported the result that JNK1 knockout mice are not easily to become obesity with high-fat diet.

Consequently, a compound that inhibits JNK activity is expected to be useful as a preventive and/or therapeutic agent for metabolic diseases [for example, diabetes mellitus (insulin-resistant diabetes mellitus or non-insulin-resistant diabetes mellitus), hyperlipemia, other insulin-resistant diseases, and the like], inflammatory diseases [for example, rhinitis, pharyngitis, bronchitis, pneumonia, pleuritis, bronchial asthma, chronic pulmonary emphysema, pulmonary fibrosis, inflammatory bowel disease, acute pancreatitis, chronic pancreatitis, acute respiratory distress syndrome, chronic thyroiditis, autoimmune gastritis and the like], scleroderma, deep lupus erythematosus, Graves' disease, autoimmune neutropenia, thrombocytopenia, myasthenia gravis, multiple myeloma, acute myeloblasticleukemia, chronic sarcoma, chronic myelocytic leukemia, metastatic melanoma, Kaposi's sarcoma, debilitating disease, Huntington's disease, ischemic/reperfusion disorders of stroke, myocardial ischemic symptom, ischemic heart disease, renal ischemia, neovascular glaucoma, infantile hemangioma, vascular proliferation, cardiac hypertrophy, abnormal immune response, pyrexia, cellular senescence, apoptosis-related diseases and the like.

In addition, a compound that inhibits JNK activity is expected to be useful as a preventive and/or therapeutic agent for TNF-$\alpha$ -related diseases, because JNK is considered to has a role in TNF-$\alpha$ production [Proc. Natl. Acad. Sci. USA., 98 (24), 13681 (2001)].

On the other hand, it was described that a compound represented by formula (H):

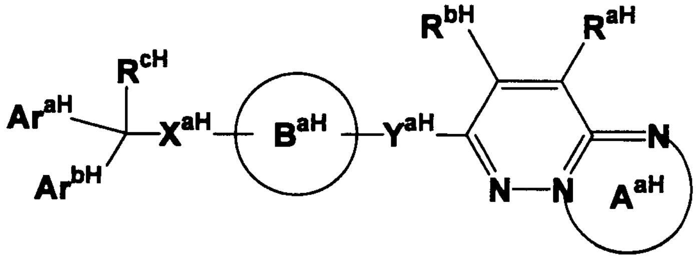

(H)

wherein, $Ar^{aH}$ and $Ar^{bH}$ each independently represent aromatic group which may have substituent(s), etc.; ring $B^{aH}$ represents heterocyclic ring containing nitrogen atom which may have substituent(s), etc.; $X^{aH}$ and $Y^{aH}$ each independently represent a bond or oxygen atom, etc.; ring $A^{aH}$ represents 5-membered cyclic group which may have substituent (s) ; $R^{aH}$ and $R^{aH}$ each independently represent halogen atom or hydrocarbon group, etc.; $R^{CH}$ represents hydroxy group, carboxyl group, etc.; with the proviso that only required symbols in the group are extracted; is useful for JNK inhibitor (see reference such as patent literature 1).

Furthermore, it was described that a compound represented by formula (Q) :

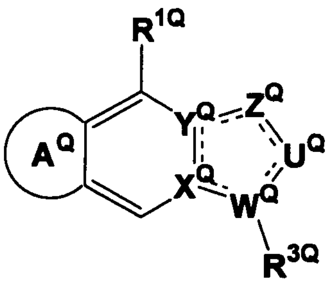

(Q)

wherein, $X^{Q}$ and $Y^{Q}$ each independently represent a carbon atom or nitrogen atom (in this regard, however these two don't represent a nitrogen atom at the same time); $W^{Q}$ represents a carbon atom or sulfur atom; $U^{Q}$ and $Z^{Q}$ each independently represent $CR^2$, $NR^{13}$, a nitrogen atom, oxygen atom, sulfur atom, etc.; ring $A^{Q}$ represents carbocyclic ring or heterocyclic ring; $R^{1Q}$ represents carbon chain with/without substituent (s) , etc. ; $R^{3Q}$ represents C5-10 monocyclic or bicyclic carbocyclic ring or 5-10 monocyclic or bicyclic heterocyclic ring; with the proviso that only required symbols in the group are extracted; is useful as a Corticotropin releasing factor receptor antagonist (see reference such as patent literature 2).

[Patent literature] JP2003-137785

[Patent literature] WO02/53565

## DISCLOSURE OF THE INVENTION

A preventive and/or therapeutic agent for metabolic disorder, inflammatory disease and the like which is useful as a medicament, and development of a compound having excellent oral absorption and safe kinase inhibitor, especially JNK inhibitor is desired.

The present inventors have made extensive studies to find a compound that can become a therapeutic agent for various diseases by inhibiting kinase activity of JNK, and as a result, have found that the object is achieved by the compound of the present invention represented by the formula (I), and then have completed the present invention.

Namely, the present invention relates to the followings:

1. a compound represented by the formula (I):

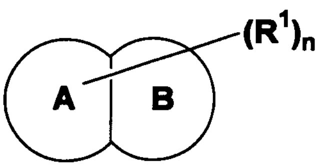

(I)

wherein

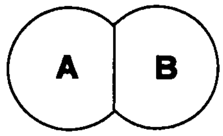

represents a 8- to 10-membered fused heterocyclic ring; $R^1$ represents (1) a hydrogen atom, (2) a halogen atom, (3) a cyano group, (4) an oxo group, (5) an optionally protected hydroxy group, (6) an optionally protected carboxyl group, (7) an optionally protected amino group, (8) a cyclic group which may have a substituent (s), (9) an aliphatic hydrocarbon group which may have a substituent (s), or (10) an optionally protected thiol group; n represents 0 or an integer of 1 to 8; provided that if n represents an integer of not less than 2, the plural $R^1$s are the same or different; or a salt thereof, a solvate thereof or a prodrug thereof,

2. the compound according to the above 1, wherein

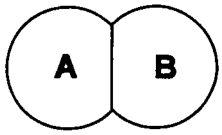

is

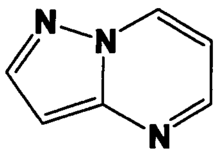 , 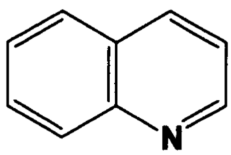 or 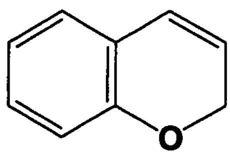 ,

3. The compound according to claim 1, wherein the formula

(I) is represented by the formula (I-1):

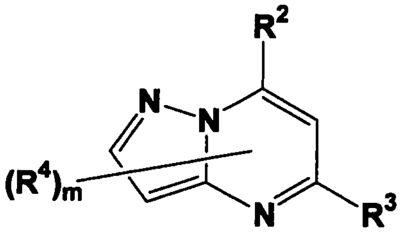

(I-1)

wherein $R^2$ represents an optionally protected amino group or a cyclic group which may have a substituent (s) ; $R^3$ represents a halogen atom, an optionally protected hydroxy group, an optionally protected thiol group or a cyclic group which may have a substituent (s) ; $R^4$ represents a hydrogen atom, a halogen atom, an optionally protected amino group, an optionally protected hydroxy group, an optionally protected thiol group, a cyclic group which may have a substituent (s) or an aliphatic hydrocarbon group which may have a substituent (s) ; m represents 0 or an integer of 1 to 3; provided that if m represents an integer of not less than 2, the plural $R^4$s are the same or different.

4. the compound according to the above 1, wherein the formula (I) is represented by the formula (1-2):

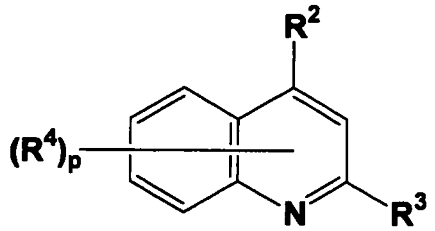

(I-2)

wherein p represents 0 or an integer of 1 to 5; the other symbols represent the same meanings as in claim 3; provided that if p represents an integer of not less than 2, the plural $R^4$s are the same or different,

5. the compound according to the above 1, wherein the formula (I) is represented by the formula (I-3) :

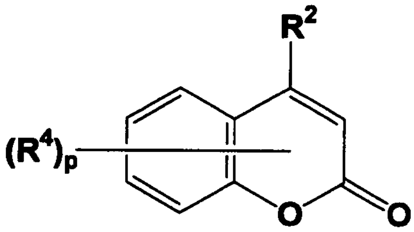

(I-3)

wherein all the symbols represent the same meanings as in the above 3 or 4,

6. the compound according to the above 3, 4, or 5, wherein $R^2$ is a protected amino group,

7. the compound according to the above 1 selected from the group consisting of

(1) N-(1,3-benzodioxol-5-ylmethyl)-5-chloropyrazolo[1,5-a]pyrimidin-7-amine,
(2) 5-chloro-N-(3-chlorophenyl)pyrazolo[1,5-a]pyrimidin-7-amine,
(3) 5-thien-3-yl-N-(3,4,5-trimethoxybenzyl)pyrazolo[1,5-a]pyrimidin-7-amine,
(4) N-(4-{7-[(4-methoxybenzyl)amino]pyrazolo[1,5-a]pyrimidin-5-yl}phenyl)acetamide,
(5) 2-{4-[(5-chloropyrazolo[1,5-a]pyrimidin-7-yl)amino]phenyl}ethanol,
(6) 5-(2-furyl)-N-(3,4,5-trimethoxybenzyl)pyrazolo[1,5-a]pyrimidin-7-amine,
(7) 5-(4-fluorophenyl)-N-(3,4,5-trimethoxybenzyl)pyrazolo[1,5-a]pyrimidin-7-amine,
(8) 5-(5-methylthien-2-yl)-N-(3,4,5-trimethoxybenzyl)pyrazolo[1,5-a]pyrimidin-7-amine,
(9) 5-(3,4-dimethylphenyl)-N-(pyridin-4-ylmethyl)pyrazolo[1,5-a]pyrimidin-7-amine,
(10) N-(pyridin-4-ylmethyl)-5-quinolin-3-ylpyrazolo[1,5-a]pyrimidin-7-amine,
(11) 5-(3-fluorophenyl)-N-(3,4,5-trimethoxybenzyl)pyrazolo[1,5-a]pyrimidin-7-amine,
(12) N-(pyridin-4-ylmethyl)-5-thien-3-ylpyrazolo[1,5-a]pyrimidin-7-amine,
(13) N-(4-methoxybenzyl)-5-thien-3-ylpyrazolo[1,5-a]pyrimidin-7-amine,
(14) 1-(3-{7-[(4-methoxybenzyl)amino]pyrazolo[1,5-a]pyrimidin-5-yl}phenyl)ethanone,
(15) 5-pyridin-4-yl-N-(3,4,5-trimethoxybenzyl)pyrazolo[1,5-a]pyrimidin-7-amine,
(16) $N^5$-[4-(dimethylamino)phenyl]-$N^7$-propylpyrazolo[1,5-a]pyrimidin-5,7-diamine,
(17) 5-(3-furyl)-N-(3,4,5-trimethoxybenzyl)pyrazolo[1,5-a]pyrimidin-7-amine,
(18) 5-(3-furyl)-N-(thien-2-ylmethyl)pyrazolo[1,5-a]pyrimidin-7-amine,
(19) 5-(3-furyl)-N-(3,4,5-trimethoxyphenyl)pyrazolo[1,5-a]pyrimidin-7-amine,
(20) 5-(4-methylphenyl)-N-(4-pyridinylmethyl)pyrazolo[1,5-a]pyrimidin-7-amine,
(21) 5-(3-methoxyphenyl)-N-(4-pyridinylmethyl)pyrazolo[1,5-a]pyrimidin-7-amine,
(22) 5-(3-furyl)-N-(4-pyridinylmethyl)pyrazolo[1,5-a]pyrimidin-7-amine,
(23) N-(4-methoxybenzyl)-5-(4-methylphenyl)pyrazolo[1,5-a]pyrimidin-7-amine,
(24) N-(4-methoxybenzyl)-5-(3-methoxyphenyl)pyrazolo[1,5-a]pyrimidin-7-amine,
(25) 5-(3-furyl)-N-(4-methoxybenzyl)pyrazolo[1,5-a]pyrimidin-7-amine,
(26) {1-[5-(3-methoxyphenyl)pyrazolo[1,5-a]pyrimidin-7-yl]-2-pyrrolidinyl}methanol,
(27) 5-(4-methyl-2-thienyl)-N-(4-pyridinylmethyl)pyrazolo[1,5-a]pyrimidin-7-amine,
(28) 4-[(3-chloro-4-fluorophenyl)amino]-6-methyl-2H-chromen-2-one,
(29) 4-[(3-chloro-4-fluorophenyl)amino]-8-methyl-2H-chromen-2-one,
(30) 4-[(3-chloro-4-fluorophenyl)amino]-2H-chromen-2-one,
(31) 5-chloro-N-(4-methoxybenzyl)pyrazolo[1,5-a]pyrimidin-7-amine,

(32) 5-chloro-N-(4-methoxybenzyl)-2-methylpyrazolo[1,5-a]pyrimidin-7-amine,
(33) 5-chloro-N-(4-methoxybenzyl)-3-methylpyrazolo[1,5-a]pyrimidin-7-amine,
(34) N-(4-methoxybenzyl)-5-methylpyrazolo[1,5-a]pyrimidin-7-amine,
(35) N-(4-methoxybenzyl)-2,5-dimethylpyrazolo[1,5-a]pyrimidin-7-amine,
(36) N-(4-methoxybenzyl)-3,5-dimethylpyrazolo[1,5-a]pyrimidin-7-amine,
(37) N-(4-methoxybenzyl)-5-phenylpyrazolo[1,5-a]pyrimidin-7-amine,
(38) N-(4-methoxybenzyl)-2-methyl-5-phenylpyrazolo[1,5-a]pyrimidin-7-amine, and
(39) N-(4-methoxybenzyl)-3-methyl-5-phenylpyrazolo[1,5-a]pyrimidin-7-amine,

8. a pharmaceutical composition which comprises the compound represented by the formula (I) according to the above 1, a salt thereof, a solvate thereof, or a prodrug thereof,
9. the pharmaceutical composition according to the above 8, which is a kinase inhibitor,
10. the pharmaceutical composition according to the above 9, wherein the kinase is c-Jun N-terminal kinase,
11. the pharmaceutical composition according to the above 10, wherein the c-Jun N-terminal kinase is JNK1,
12. the pharmaceutical composition according to the above 8, wherein the compound is represented by the formula (I-1), (I-2) or (I-3) :

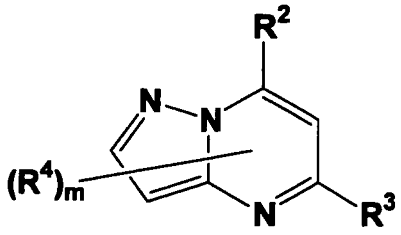

(I-1)

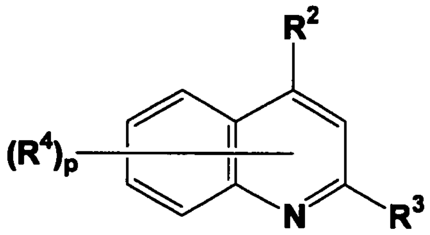

(I-2)

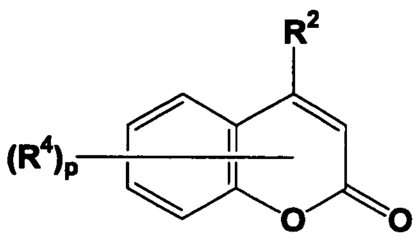

(I-3)

wherein all the symbols represent the same meanings as in the above 3 or 4,
13. the composition according to claim 8, wherein the compound is selected from the group consisting of;

(1) N-(1,3-benzodioxol-5-ylmethyl)-5-chloropyrazolo[1,5-a]pyrimidin-7-amine,
(2) 5-chloro-N-(3-chlorophenyl)pyrazolo[1,5-a]pyrimidin-7-amine,
(3) 5-thien-3-yl-N-(3,4,5-trimethoxybenzyl)pyrazolo[1,5-a]pyrimidin-7-amine,
(4) N-(4-{7-[(4-methoxybenzyl)amino]pyrazolo[1,5-a]pyrimidin-5-yl}phenyl)acetamide,
(5) 2-{4-[(5-chloropyrazolo[1,5-a]pyrimidin-7-yl)amino]phenyl}ethanol,
(6) 5-(2-furyl)-N-(3,4,5-trimethoxybenzyl)pyrazolo[1,5-a]pyrimidin-7-amine,
(7) 5-(4-fluorophenyl)-N-(3,4,5-trimethoxybenzyl)pyrazolo[1,5-a]pyrimidin-7-amine,
(8) 5-(5-methylthien-2-yl)-N-(3,4,5-trimethoxybenzyl)pyrazolo[1,5-a]pyrimidin-7-amine,
(9) 5-(3,4-dimethylphenyl)-N-(pyridin-4-ylmethyl)pyrazolo[1,5-a]pyrimidin-7-amine,
(10) N-(pyridin-4-ylmethyl)-5-quinolin-3-ylpyrazolo[1,5-a]pyrimidin-7-amine,

(11) 5-(3-fluorophenyl)-N-(3,4,5-trimethoxybenzyl)pyrazolo[1,5-a]pyrimidin-7-amine,
(12) N-(pyridin-4-ylmethyl)-5-thien-3-ylpyrazolo[1,5-a]pyrimidin-7-amine,
(13) N-(4-methoxybenzyl)-5-thien-3-ylpyrazolo[1,5-a]pyrimidin-7-amine,
(14) 1-(3-{7-[(4-methoxybenzyl)amino]pyrazolo[1,5-a]pyrimidin-5-yl}phenyl)ethanone,
(15) 5-pyridin-4-yl-N-(3,4,5-trimethoxybenzyl)pyrazolo[1,5-a]pyrimidin-7-amine,
(16) $N^5$-[4-(dimethylamino)phenyl]-$N^7$-propylpyrazolo[1,5-a]pyrimidine-5,7-diamine,
(17) 5-(3-furyl)-N-(3,4,5-trimethoxybenzyl)pyrazolo[1,5-a]pyrimidin-7-amine,
(18) 5-(3-furyl)-N-(thien-2-ylmethyl)pyrazolo[1,5-a]pyrimidin-7-amine,
(19) 5-(3-furyl)-N-(3,4,5-trimethoxyphenyl)pyrazolo[1,5-a]pyrimidin-7-amine,
(20) 5-(4-methylphenyl)-N-(4-pyridinylmethyl)pyrazolo[1,5-a]pyrimidin-7-amine,
(21) 5-(3-methoxyphenyl)-N-(4-pyridinylmethyl)pyrazolo[1,5-a]pyrimidin-7-amine,
(22) 5-(3-furyl)-N-(4-pyridinylmethyl)pyrazolo[1,5-a]pyrimidin-7-amine,
(23) N-(4-methoxybenzyl)-5-(4-methylphenyl)pyrazolo[1,5-a]pyrimidin-7-amine,
(24) N-(4-methoxybenzyl)-5-(3-methoxyphenyl)pyrazolo[1,5-a]pyrimidin-7-amine,
(25) 5-(3-furyl)-N-(4-methoxybenzyl)pyrazolo[1,5-a]pyrimidin-7-amine,
(26) {1-[5-(3-methoxyphenyl)pyrazolo[1,5-a]pyrimidin-7-yl]-2-pyrrolidinyl}methanol,
(27) 5-(4-methyl-2-thienyl)-N-(4-pyridinylmethyl)pyrazolo[1,5-a]pyrimidin-7-amine,
(28) 4-[(3-chloro-4-fluorophenyl)amino]-6-methyl-2H-chromen-2-one,
(29) 4-[(3-chloro-4-fluorophenyl)amino]-8-methyl-2H-chromen-2-one,
(30) 4-[(3-chloro-4-fluorophenyl)amino]-2H-chromen-2-one,
(31) 5-chloro-N-(4-methoxybenzyl)pyrazolo[1,5-a]pyrimidine-7-amine,
(32) 5-chloro-N-(4-methoxybenzyl)-2-methylpyrazolo[1,5-a]pyrimidin-7-amine,
(33) 5-chloro-N-(4-methoxybenzyl)-3-methylpyrazolo[1,5-a]pyrimidin-7-amine,
(34) N-(4-methoxybenzyl)-5-methylpyrazolo[1,5-a]pyrimidin-7-amine,
(35) N-(4-methoxybenzyl)-2,5-dimethylpyrazolo[1,5-a]pyrimidin-7-amine,
(36) N-(4-methoxybenzyl)-3,5-dimethylpyrazolo[1,5-a]pyrimidin-7-amine,
(37) N-(4-methoxybenzyl)-5-phenylpyrazolo[1,5-a]pyrimidin-7-amine,
(38) N-(4-methoxybenzyl)-2-methyl-5-phenylpyrazolo[1,5-a]pyrimidin-7-amine, and
(39) N-(4-methoxybenzyl)-3-methyl-5-phenylpyrazolo[1,5-a]pyrimidin-7-amine,

14. the pharmaceutical composition according to the above 10, which is a preventive and/or therapeutic agent for c-Jun N-terminal kinase-mediated diseases,
15. the pharmaceutical composition according to the above 14, wherein the c-Jun N-terminal kinase-mediated diseases are metabolic diseases or inflammatory diseases,
16. the pharmaceutical composition according to the above 15, wherein the metabolic disease is diabetes mellitus,
17. the pharmaceutical composition according to the above 16, wherein the diabetes mellitus is insulin-resistant diabetes mellitus,
18. the pharmaceutical composition according to the above 15, wherein the inflammatory diseases are osteitis,
19. the pharmaceutical composition according to the above 18, wherein the osteitis is arthritis,
20. a method for inhibiting c-Jun N-terminal kinase, which comprises administering to a mammal an effective amount of the compound according to the above 1, a salt thereof, a solvate thereof or a prodrug thereof,
21. a method for preventing and/or treating c-Jun N-terminal kinase-mediated diseases in a mammal, which comprises administering to a mammal an effective amount of the compound according to the above 1, a salt thereof, a solvate thereof or a prodrug thereof,
22. use of the compound according to the above 1, a salt thereof, a solvate thereof or a prodrug thereof, for the manufacture of a preventive and/or therapeutic agent for c-Jun N-terminal kinase-mediated diseases,
23. a pharmaceutical composition which comprises a combination of the compound according to the above 1, a salt thereof, a solvate thereof or a prodrug thereof and one or two or more medicaments selected from the group consisting of an MTP inhibitor, an HMG-CoA reductase inhibitor, a squalene synthetase inhibitor, a fibrate preparation, an ACAT inhibitor, a 5-lipoxygenase inhibitor, a cholesterol absorption inhibitor, a bile acid absorption inhibitor, a ileum $Na^+$/bile acid cotransporter inhibitor, an LDL receptor activator/expression enhancer, a lipase inhibitor, a probucol preparation, a nicotinic acid preparation, a hypoglycemic sulfonylurea agent, a biguanide preparation, an $\alpha$-glucosidase inhibitor, a rapid-acting insulin secretagogue, an insulin preparation, a DPP4 inhibitor, a PTP1B inhibitor, a $\beta$3 adrenoceptor agonist, a PPAR agonist, and a therapeutic agent for diabetes complications,
24. a pharmaceutical composition which comprises a combination of the compound according to the above 1, a salt thereof, a solvate thereof or a prodrug thereof and one or two or more medicaments selected from the group consisting of a steroid drug, an elastase inhibitor, a cannabinoid-2 receptor stimulator, a prostaglandin, a prostaglandin synthase inhibitor, a phosphodiesterase inhibitor, a metalloproteinase inhibitor, an adhesion molecule inhibitor,

an anti-cytokine protein preparation (anti-TNF-α preparation, anti-IL-1 preparation, anti-IL-6 preparation), an anti-cytokine agent, an immunomodulating agent, a disease modifying anti-rheumatic agent, a steroidal anti-inflammatory agent, and a c-Jun N-terminal kinase inhibitor; and

25. a method of preparing the compound according to the above 1, a salt thereof, a solvate thereof or a prodrug thereof.

In the present specification, an 8- to 10-membered fused heterocyclic ring represented by the formula:

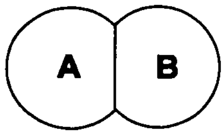

(hereinafter, also abbreviated as fused ring AB) includes, for example, fused heterocyclic rings between 5-memberd ring and 5-memberd ring, fused heterocyclic rings between 5-memberd ring and 6-memberd ring, and fused heterocyclic rings between 6-memberd ring and 6-memberd ring. This fused heterocyclic ring includes 8- to 10-membered, unsaturated fused heterocyclic rings, and 8- to 10-membered, completely saturated fused heterocyclic rings. The "8- to 10-membered, unsaturated or partially unsaturated fused heterocyclic rings" include, for example, indole, isoindole, indolizine, benzofuran, isobenzofuran, benzothiophene, isobenzothiophene, dithianaphthalene, indazole, quinoline, isoquinoline, quinolidine, purine, phthalazine, pteridine, naphthyridine, quinoxaline, quinazoline, cinnoline, benzoxazole, benzothiazole, benzimidazole, chromene, benzofurazan, benzothiadiazole, benzotriazole, pyrazolo[1,5-a]pyrimidine, indoline, isoindoline, dihydrobenzofuran, dihydroisobenzofuran, dihydrobenzothiophene, dihydroisobenzothiophene, dihydroindazole, dihydroquinoline, tetrahydroquinoline, dihydroisoquinoline, tetrahydroisoquinoline, dihydrophthalazine, tetrahydrophthalazine, dihydronaphthyridine, tetrahydronaphthyridine, dihydroquinoxaline, tetrahydroquinoxaline, dihydroquinazoline, tetrahydroquinazoline, dihydrocinnoline, tetrahydrocinnnoline, benzoxathiane, dihydrobenzoxazine, dihydrobenzothiazine, pyrazinomorpholine, dihydrobenzoxazole, dihydrobenzothiazole, dihydrobenzoimidazole, dioxaindane, benzodioxane, chroman, benzodithiolane, benzodithiane, dihydropyrazolo[1,5-a]pyrimidine, tetrahydropyrazolo[1,5-a]pyrimidine, and the like.

The "8- to 10-membered, completely saturated fused heterocyclic rings" include, for example, perhydrobenzofuran, perhydroisobenzofuran, perhydrobenzothiophene, perhydroisobenzothiophene, perhydroindazole, perhydroquinoline, perhydroisoquinoline, perhydrophthalazine, perhydronaphthyridine, perhydroquinoxaline, perhydroquinazoline, perhydrocinnoline, perhydrobenzoxazole, perhydrobenzothiazole, perhydrobenzimidazole, perhydropyrazolo[1,5-a]pyrimidine, and the like.

In the present specification, the "halogen atom" includes, for example, fluorine, chlorine, bromine and iodine, and the like.

In the present specification, the "cyclic group" in the "cyclic group which may have a substituent(s)" includes, for example, a carbocyclic group, a heterocyclic group, and the like.

In the present specification, the carbocyclic group includes, for example, a monovalent group formed by removing an arbitrary hydrogen atom from a "C3-15 monocyclic, bicyclic or tricyclic carbocyclic ring", and the like. The "C3-15 monocyclic, bicyclic or tricyclic carbocyclic ring" includes a C3-15 monocyclic, bicyclic or tricyclic unsaturated carbocyclic ring, a partially or completely saturated carbocyclic ring, a spiro-bound bicyclic carbocyclic ring, and a crosslinked bicyclic carbocyclic ring, and the like.

The "C3-15 monocyclic, bicyclic or tricyclic partially or completely saturated carbocyclic ring"' includes, for example, cyclopropane, cyclobutane, cyclopentane, cyclohexane, cycloheptane, cyclooctane, cyclononane, cyclodecane, cycloundecane, cyclododecane, cyclotridodecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indane, naphthalene, dihydronaphthalene, tetrahydronaphthalene, perhydronaphthalene, heptalene, perhydroheptalene, biphenylene, as-indacene, s-indacene, acenaphthylene, acenaphthene, fluorene, phenalene, phenanthrene, anthracene and the like. The "spiro-bound bicyclic carbocyclic ring" includes, for example, spiro[4.4]nonane, spiro[4.5]decane, spiro[5.5]undecane rings, and the like. The "crosslinked bicyclic carbocyclic ring" includes, for example, bicyclo[2.2.1]heptane, bicyclo[2.2.1]hepta-2-ene, bicyclo[3.1.1]heptane, bicyclo[3.1.1]hepta-2-ene, bicyclo[3.2.1]octane, bicyclo[2.2.2]octane, bicyclo[2.2.2]octa-2-ene, adamantane, noradamantane rings, and the like. Among these, a "C3-15 monocyclic, bicyclic or tricyclic aromatic carbocyclic ring" includes, for example, benzene, azulene, naphthalene, phenanthrene, anthracene rings, and the like.

In the present invention, the heterocyclic group includes, for example, a monovalent group formed by removing an arbitrary hydrogen atom from a "3- to 15-membered monocyclic, bicyclic or tricyclic heterocyclic ring having 1 to 4 nitrogen atom(s), 1 or 2 oxygen atom(s) and/or 1 or 2 sulfur atom(s) as a hetero atom(s) ", and the like. Herein, the "3-

to 15-membered monocyclic, bicyclic or tricyclic heterocyclic ring having 1 to 4 nitrogen atom (s), 1 or 2 oxygen atom (s) and/or 1 or 2 sulfur atom(s) as a hetero atom(s)" includes a 3- to 15-membered monocyclic, bicyclic or tricyclic unsaturated heterocyclic ring having 1 to 4 nitrogen atom(s), 1 or 2 oxygen atom(s) and/or 1 or 2 sulfur atom(s) as a hetero atom(s), or partially or completely saturated one thereof, a spiro-bound bicyclic heterocyclic ring and a crosslinked bicyclic heterocyclic ring.

The "3- to 15-membered monocyclic, bicyclic or tricyclic unsaturated heterocyclic ring having 1 to 4 nitrogen atom(s), 1 or 2 oxygen atom(s) and/or 1 or 2 sulfur atom(s) as a hetero atom(s), or a partially or completely saturated one thereof" includes, for example, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzoxepine, benzoxazepine, benzoxadiazepine, benzothiepine, benzothiazepine, benzothiadiazepine, benzoazepine, benzodiazepine, benzofurazan, benzothiadiazole, benzotriazole, carbazole, β-carboline, acridine, phenazine, dibenzofuran, xanthene, dibenzothiophene, phenothiazine, phenoxazine, phenoxathiin, thianthrene, phenanthridine, phenanthroline, perimidine, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydroxepine, tetrahydroxepine, perhydroxepine, thiirane, thietane, dihydrothiophene, tetrahydrothiophene, dihydrothiopyran, tetrahydrothiopyran, dihydrothiepin, tetrahydrothiepin, perhydrothiepin, dihydroxazole, tetrahydroxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydroxadiazole, tetrahydroxadiazole (oxadiazolidine), dihydroxazine, tetrahydroxazine, dihydroxadiazine, tetrahydroxadiazine, dihydroxazepine, tetrahydroxazepine, perhydroxazepine, dihydroxadiazepine, tetrahydroxadiazepine, perhydroxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, dioxaindane, benzodioxane, chroman, benzodithiolane, benzodithiane, 6,7-dihydro-5H-cyclopenta[b]pyrazine, 5H-cyclopenta[b]pyrazine, imidazo[2,1-b][1,3]thiazole rings, and the like. The "spiro-bound bicyclic heterocyclic ring" includes, for example, azaspiro[4.4]nonane, oxazaspiro[4.4]nonane, dioxaspiro[4.4]nonane, azaspiro[4.5]decane, thiaspiro[4.5]decane, dithiaspiro[4.5]decane, dioxaspiro[4.5]decane, oxazaspiro[4.5]decane, azaspiro[5.5]undecane, oxaspiro[5.5]undecane, dioxaspiro[5.5]undecane rings, and the like. The "crosslinked bicyclic heterocyclic ring" includes, for example, azabicyclo[2.2.1]heptane, oxabicyclo[2.2.1]heptane, azabicyclo[3.1.1]heptane, azabicyclo[3.2.1]octane, oxabicyclo[3.2.1]octane, azabicyclo[2.2.2]octane, diazabicyclo[2.2.2]octane rings, and the like.

Among these, the "3- to 15-membered monocyclic, bicyclic or tricyclic aromatic heterocyclic ring having 1 to 4 nitrogen atom(s), 1 or 2 oxygen atom(s) and/or 1 or 2 sulfur atom(s) as a hetero atom(s) includes, for example, pyrrole, imidazole, triazole, tetrazole, pyrazole, pyridine, pyrazine, pyrimidine, pyridazine, furan, thiophene, oxazole, isoxazole, thiazole, isothiazole, furazan, oxadiazole, thiadiazole, indole, isoindole, benzofuran, isobenzofuran, benzothiophene, isobenzothiophene, indazole, quinoline, isoquinoline, purine, phthalazine, pteridine, naphthyridine, quinoxaline, quinazoline, cinnoline, benzooxazole, benzothiazole, benzimidazole, benzofurazan, benzothiadiazole, benzotriazole, carbazole, β-carboline, acridine, phenazine, dibenzofuran, dibenzothiophene, phenanthridine, phenanthroline, perimidine rings and the like.

In the present specification, the "substituent" in the "cyclic group which may have a substituent(s)" is not particularly limited, so long as it is a substituent. The "substituent" includes, for example, (1) a carboxyl group, (2) a sulfo

group, (3) a sulfino group, (4) a phosphono group, (5) a nitro group, (6) an oxo group, (7) a thioxo group, (8) a cyano group, (9) an amidino group, (10) a dihydroxyboryl(-$B(OH)_2$) group, (11) a halogen atom (which has the same meaning as described above), (12) an alkoxycarbonyl group (for example, a C1-6 alkoxycarbonyl group, such as methoxycarbonyl, ethoxycarbonyl, tert-butoxycarbonyl, etc.), (13) an alkylsulfinyl group (for example, a C1-6 alkylsulfinyl group, such as methylsulfinyl, ethylsulfinyl, propylsulfinyl, butylsulfinyl, pentylsulfinyl, hexylsulfinyl, tert-butylsulfinyl, etc.), (14) an arylsulfinyl group (C6-10 aromatic carbocyclic sulfinyl group, such as phenylsulfinyl, naphthylsulfinyl, etc.), (15) an alkylsulfonyl group (for example, a C1-6 alkylsulfonyl group, such as methylsulfonyl, ethylsulfonyl, propylsulfonyl, butylsulfonyl, pentylsulfonyl, hexylsulfonyl, tert-butylsulfonyl, etc., and the like), (16) an arylsulfonyl group (for example, C6-10 aromatic carbocyclic sulfonyl group, such as phenylsulfonyl, naphthylsulfonyl, etc.), (17) an alkylcarbonylhydrazino group (for example, a C1-6 alkylcarbonylhydrazino group (-NH-NH-CO-(C1-6alkyl), such as methylcarbonylhydrazino, ethylcarbonylhydrazino, tert-butylcarbonylhydrazino, etc.), (18) an arylhydrazone group (for example, a C6-10 aromatic carbocyclic hydrazone group which may have a substituent(s), such as benzaldehyde hydrazone, p-methoxybenzaldehyde hydrazone, etc.) (19) an acyl group (for example, a C1-6 alkanoyl group, such as formyl, acetyl, propanoyl, pivaloyl, etc., a C3-8 cycloalkanoyl group, such as cyclopentylcarbonyl, cyclohexylcarbonyl, etc., a C6-10 aromatic carbocyclic carbonyl group, such as, benzoyl, etc., heterocyclic carbonyl group which may have a substituent(s), such as morpholin-4-ylcarbonyl, piperidin-1-ylcarbonyl, 1-methylpiperazin-4-ylcarbonyl etc., a C6-10 aromatic carbocyclic alkanoyl group which may have a substituent (s), such as phenylmethylcarbonyl, 2-phenylethylcarbonyl, etc., and the like), (20) an optionally protected hydroxy group, (21) an optionally protected thiol group, (22) an optionally protected amino group, (23) a carbamoyl group which may have a substituent(s), (24) a sulfamoyl group which may have a substituent(s), (25) an imino group which may have a substituent(s), (26) an alkyl group which may have a substituent(s), (27) an alkenyl group which may have a substituent(s), (28) an alkynyl group which may have a substituent(s), (29) a carbocyclic group which may have a substituent(s), (30) a heterocyclic group which may have a substituent(s), and the like. These substituents may be substituted in the number of 1 to 5 at a substitutable position.

The "alkyl group" in the "alkyl group which may have a substituent(s)" as the "substituent", includes, for example, a straight or branched C1-8 alkyl group such as methyl, ethyl, propyl, isopropyl, butyl, isobutyl, sec-butyl, tert-butyl, pentyl, hexyl, heptyl, octyl. The "substituent" in the "alkyl group which may have a substituent(s)" is not particularly limited, so long as it is a substituent. The "substituent" includes, for example, (a) a hydroxy group, (b) an amino group, (c) a carboxyl group, (d) a nitro group, (e) a cyano group, (f) a halogen atom (which has the same meaning as described above), (g) a mono- or di-(C1-6 alkyl) amino group (such as, methylamino, ethylamino, propylamino, dimethylamino, diethylamino), (h) a C1-6 alkoxy group (such as, methoxy, ethoxy, propoxy, tert-butoxy, hexyloxy), (i) a C1-6alkylcarbonyloxy group (such as, acetoxy, ethylcarbonyloxy), (j) an acyl group (which has the same meaning as described above), (k) a carbamoyl group which may have a substituent(s), (1) a carbocyclic group which may have a substituent (s), (m) a heterocyclic group which may have a substituent(s), and the like. These substituents may be substituted in the number of 1 to 4 at a substitutable position. Herein, "carbamoyl group which may have a substituent (s)", "carbocyclic group which may have a substituent(s)", and "heterocyclic group which may have a substituent(s)" include the same example of "carbamoyl group which may have a substituent(s)", "carbocyclic group which may have a substituent(s)", and "heterocyclic group which may have a substituent(s)" as the "substituent" hereinafter described.

The "alkenyl group" in the "alkenyl group which may have a substituent(s)" as the "substituent" includes, for example a straight or branched C2-8 alkenyl group such as ethenyl, propenyl, butenyl, butadienyl, pentenyl, pentadienyl, hexenyl, hexadienyl, heptenyl, heptadienyl, octenyl, octadienyl, and the like. The "substituent" in the "alkenyl group which may have a substituent(s)" is not particularly limited, so long as it is a substituent. The "substituent" includes the same example of such as the "substituent" in the "alkyl group which may have a substituent(s)". These substituents may be substituted in the number of 1 to 4 at a substitutable position.

The "alkynyl group" in the "alkynyl group which may have a substituent (s)" as the "substituent" includes, for example, a straight or branched C2-8 alkynyl group such as ethynyl, propynyl, butynyl, butadiynyl, pentynyl, pentadiynyl, hexynyl, hexadiynyl, heptynyl, heptadiynyl, octynyl, octadiynyl, and the like. The "substituent" in the "alkynyl which may have a substituent(s)" is not particularly limited, so long as it is a substituent. The "substituent" includes the same example of such as the "substituent" in the "alkyl group which may have a substituent(s)". These substituents may be substituted in the number of 1 to 4 at a substitutable position.

The "carbocyclic group" in the "carbocyclic group which may have a substituent (s) " includes, for example, a monovalent group formed by removing an arbitrary hydrogen atom from a "C3-15 monocyclic, bicyclic or tricyclic carbocyclic ring" described above, and the like. The "substituent" in the "carbocyclic group which may have a substituent (s)" is not particularly limited, so long as it is a substituent. The "substituent" includes a straight or branched C1-8 alkyl group (which has the same meaning as the "alkyl group" in the "alkyl group which may have a substituent(s)", a straight or branched C2-8 alkenyl group (which has the same meaning as the "alkenyl group" in the "alkenyl group which may have a substituent (s)", a straight or branched C2-8 alkynyl group (which has the same meaning as the "alkynyl group" in the "alkynyl group which may have a substituent (s) ", a hydroxy group, a C1-6 alkoxy group (which has the same meaning as described above), a C6-10 aryloxy group (such as phenoxy), a thiol group, a C1-6 alkylthio group (such as

methylthio, ethylthio, propylthio, isopropylthio, butylthio, isobutylthio, tert-butylthio, pentylthio, hexylthio, etc.), a C6-10 arylthio group (such as phenylthio), an amino group, a mono- or di- (C1-6 alkyl) amino group (which has the same meaning as described above), a mono- or di-(C6-10 aryl)amino group (such as phenyamino, diphenylamino, etc.), a mono- (C1-6 alkyl)-mono-(C6-10 aryl)amino group (such as N-phenyl-N-methylamino, N-phenyl-N-ethylamino, etc.), a carboxyl group, a C1-6 alkylcarbonyloxy group (such as acetoxy, ethylcarbonyloxy, etc.), a halogen atom (which has the same meaning as described above), a cyano group, a nitro group, and the like. These substituents may be substituted in the number of 1 to 5 at a substitutable position.

The "hetrocyclic group" in the "heterocyclic group which may have a substituent (s)" includes, for example, a monovalent group formed by removing an arbitrary hydrogen atom from a "3-to 15-membered monocyclic, bicyclic or tricyclic heterocyclic ring having 1 to 4 nitrogen atom (s), 1 or 2 oxygen atom(s) and/or 1 or 2 sulfur atom(s) as a hetero atom (s)", and the like. The "substituent" in the "heterocyclic group which may have a substituent(s)" is not particularly limited, so long as it is a substituent. The "substituent" includes the same example of such as the "substituent" in the "carbocyclic group which may have a substituent(s)". These substituents may be substituted in the number of 1 to 5 at a substitutable position.

The "imino which may have a substituent(s)" as the "substituent" includes, for example, a non-substituted imino group and an imino group substituted by a C1-6 alkyl group (such as methylimino group, ethylimino group, etc.), an imino group substituted by a C6-10 aromatic carbocyclic group which may have a substituent(s) (such as phenylimino group, p-fluorophenylimino group, p-chlorophenylimino group, etc.), an imino group substituted by a hydroxy group (such as hydroxyimino group, etc.), an imino group substituted by a C1-6 alkoxy group (such as methoxyimino group, ethoxyimino group, etc.), an imino group substituted by a C6-10 aromatic carbocyclic group which may have a substituent (s) (such as phenoxyimino group, p-fluorophenoxyimino group, p-chlorophenoxyimino group, etc.) and the like.

The "optionally protected hydroxyl group" as the "substituent" includes, for example, a non-substituted hydroxyl group, a hydroxy group substituted by an arbitrary "protecting group" and the like. Herein, the protecting group of a hydroxyl group includes, for example, (i) an alkyl group which may have a substituent (s) (which has the same meaning as described above), (ii) a carbocyclic group which may have a substituent (s) (which has the same meaning as described above), (iii) a heterocyclic group which may have a substituent (s) (which has the same meaning as described above), (iv) an acyl group (which has the same meaning as described above), (v) sulfonyl group (such as alkylsulfonyl group (which has the same meaning as described above), (vi) an arylsulfonyl group (which has the same meaning as described above) and the like.) and the like.

The "optionally protected thiol group" as the "substituent" includes, for example, a non-substituted thiol group, a thiol group substituted by an arbitrary "protecting group" and the like. Herein, the protecting group of hydroxy includes, for example, the same example of the "protecting group" in the "optionally protected hydroxy group" and the like.

The "optionally protected amino group" as the "substituent" includes, for example, a non-substituted amino group, an amino group substituted by arbitrary one or two "protecting groups" and the like. Herein, the amino protecting group includes, for example, the same example of the "protecting group" in the "optionally protected hydroxy" and the like.

The "optionally protected carbamoyl group" as the "substituent" includes, for example, a non-substituted carbamoyl, a carbamoyl group substituted by arbitrary one or two "protecting groups" and the like. Herein, the carbamoyl-protecting group includes, for example, an alkyl which may have a substituent (s) (which has the same meaning as described above), a carbocyclic group which may have a substituent (s) (which has the same meaning as described above), a heterocyclic group which may have a substituent(s) (which has the same meaning as described above) and the like. More concretely, the carbamoyl-protecting group includes, for example, an N-mono-C1-6 alkylcarbamoyl group (such as N-methylcarbamoyl, N-ethylcarbamoyl, N-propylcarbamoyl, N-isopropylcarbamoyl, N-butylcarbamoyl, N-isobutylcarbamoyl, N-(tert-butyl)carbamoyl, N-pentylcarbamoyl, N-hexylcarbamoyl and the like), an N-mono-(C1-6alkyl substituted by hydroxy)carbamoyl group (such as N-hydroxymethylcarbamoyl, N-(2-hydroxyethyl)carbamoyl, N-(3-hydroxypropyl)carbamoyl, N-(4-hydroxybutyl)carbamoyl and the like), an N-mono-(C1-6 alkyl substituted by amino)carbamoyl (such as N-aminomethylcarbamoyl, N-(2-aminoethyl)carbamoyl, N-(3-aminopropyl)carbamoyl, N-(4-aminobutyl) carbamoyl and the like), an N-mono-(C1-6 alkyl substituted by dimethylamino)carbamoyl group (such as N-(dimethylamino)methylcarbamoyl, N-(2-dimethylaminoethyl)carbamoyl, N-(3-dimethylaminopropyl)carbamoyl, N-(4-dimethylaminobutyl)carbamoyl and the like), an N-mono(carbocyclic group which may have a substituent(s))carbamoyl group (such as N-cyclopropylcarbamoyl, N-cyclopentylcarbamoyl, N-cyclohexylcarbamoyl, N-phenylcarbamoyl and the like), an N, N-di-C1-6 alkylcarbamoyl group (such as N,N-dimethylcarbamoyl, N,N-diethylcarbamoyl, N,N-dipropylcarbamoyl, N,N-dibutylcarbamoyl, N,N-dipentylcarbamoyl, N,N-dihexylcarbamoyl, N-methyl-N-ethylcarbamoyl and the like) and the like.

The "optionally protected sulfamoyl group" as the "substituent" includes, for example, a non-substituted sulfamoyl group, a sulfamoyl group substituted by arbitrary one or two "protection group(s)" and the like. Herein, the protecting group of a sulfamoyl group includes, for example, an alkyl group which may have a substituent(s) (which has the same meaning as described above) and the like. More concretely, the protecting group of a sulfamoyl group include, for example, an N-mono-C1-6alkylsulfamoyl group (such as N-methylsulfamoyl, N-ethylsulfamoyl, N-propylsulfamoyl, N-isopropylsulfamoyl, N-butylsulfamoyl, N-isobutylsulfamoyl, N-(tert-butyl)sulfamoyl, N-pentylsulfamoyl, N-hexylsulfamoyl

and the like), an N,N-di-C1-6 alkylsulfamoyl group (such as N,N-dimethylsulfamoyl, N,N-diethylsulfamoyl, N,N-dipropylsulfamoyl, N,N-dibutylsulfamoyl, N,N-dipentylsulfamoyl, N,N-dihexylsulfamoyl, N-methyl-N-ethylsulfamoyl and the like) and the like.

In the present specification, the "aliphatic hydrocarbon group" in the "aliphatic hydrocarbon group which may have a substituent (s) " includes, for example, a "straight or branched aliphatic hydrocarbon group", and the like. The "straight or branched aliphatic hydrocarbon group" includes, for example, the "straight or branched alkyl group", the "straight or branched alkenyl group", the "straight or branched alkynyl group", and the like. Herein, the "straight or branched alkyl group" includes, for example, a straight or branched C1-10 alkyl group, such as methyl, ethyl, propyl, isopropyl, butyl, isobutyl, sec-butyl, tert-butyl, pentyl, isopentyl, neopentyl, hexyl, heptyl, octyl, nonyl, decyl and the like. The "straight or branched alkenyl group" includes, for example, a straight or branched C2-10 alkenyl group, such as ethenyl, propenyl, butenyl, butadienyl, pentenyl, pentadienyl, hexenyl, hexadienyl, heptenyl, heptadienyl, octenyl, octadienyl, nonenyl, nonadienyl, decenyl, decadienyl and the like. The "straight or branched alkynyl group " includes, for example, a straight or branched C2-10 alkynyl group, such as ethynyl, propynyl, butynyl, butadiynyl, pentynyl, pentadiynyl, hexynyl, hexadiynyl, heptynyl, heptadiynyl, octynyl, octadiynyl, nonynyl, nonadiynyl, decynyl, decadiynyl and the like.

The "substitutent" in the "aliphatic hydrocarbon group which may have a substituent(s)" is not particularly limited, so long as it is a substituent. The "substituent" includes, for example, (1) a sulfo group, (2) a sulfino group, (3) a phosphono group, (4) a nitoro group, (5) an oxo group, (6) a thioxo group, (7) a cyano group, (8) an amidino group, (9) a dihydroxyboryl group ($-B(OH)_2$), (10) a halogen atom (which has the same meaning as described above), (11) an alkylsulfinyl group (which has the same meaning as described above), (12) an arylsulfinyl group (which has the same meaning as described above), (13) an alkylsulfonyl group (which has the same meaning as described above), (14) an arylsulfonyl group (which has the same meaning as described above), (15) an acyl group (which has the same meaning as described above), (16) an optionally protected hydroxy group (which has the same meaning as described above), (17) an optionally protected thiol group (which has the same meaning as described above), (18) an optionally protected amino group (which has the same meaning as described above), (19) a carbamoyl group which may have a substituent(s) (which has the same meaning as described in the above), (20) a sulfamoyl group which may have a substituent (s) (which has the same meaning as described above), (21) an imino group which may have a substituent (s) (which has the same meaning as described above), (22) an optionally protected carboxyl, (23) a cyclic group which may have a substituent(s) (which has the same meaning as described above) and the like. These substituents may be substituted in the number of 1 to 5 at a substitutable position. Herein, the "optionally protected carboxyl group" includes, for example, a non-substituted carboxyl group and a carboxyl group substituted by an arbitrary "protecting group" and the like. Herein, the protecting group of carboxyl group includes, for example, (i) an alkyl group which may have a substituent(s) (which has the same meaning as described above), (ii) a carbocyclic group which may have a substituent(s) (which has the same meaning as described above), (iii) a heterocyclic group which may have a substituent (s) (which has the same meaning as described above) and the like.

In the present specification, the "optionally protected amino group" includes, for example, a non-substituted amino group, an amino substituted by arbitrary one or two "protecting group (s) " and the like. Herein, the protecting group of amino group includes the same example of the "protecting group" in the "optionally protected hydroxy group" as the substituent described above.

In the present specification, the "optionally protected hydroxy group" includes, for example, a non-substituted hydroxy group, a hydroxy group substituted by an arbitrary "protecting group" and the like. Herein, the protecting group of hydroxy group includes the same example of the "protecting group" in the "optionally protected hydroxy group" as the substituent described above.

In the present specification, the "optionally protected thiol group" includes, for example, a non-substituted thiol group, a thiol group substituted by an arbitrary "protecting group" and the like. Herein, the protecting group of thiol group includes the same example of the "protecting group" in the "optionally protected thiol group" as the substituent described above.

In the present specification, the "optionally protected carboxyl group" includes, for example, a non-substituted carboxyl group, a carboxyl group substituted by an arbitrary "protecting group" and the like. Herein, the protecting group of a carboxyl group includes the same example of the "protecting group" in the "optionally protected carboxyl group" as the substituent described above.

The prodrug for the compound of the formula (I) means a compound which is converted to the compound represented by the formula (I) by the reaction with an enzyme, a gastric acid, or the like, in the living body. Examples of the prodrug for the compound represented by the formula (I) include a compound wherein the amino group of the compound represented by the formula (I) is acylated, alkylated, phosphorylated, or the like (e.g., a compound wherein the amino group of the compound represented by the formula (I) is substituted with eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation, acetoxymethylation, tert-butylatiion, etc.); a compound wherein the hydroxyl group of the compound represented by the formula (I) is acylated, alkylated, phosphorylated, borated, or the like (e.g., a compound

wherein the hydroxyl group of the compound represented by the formula (I) is modified by acetylation, palmitoylation, propanoylation, pivaloylation, succinylation, fumaration, alanylation, dimethylaminomethylcarbonylation, etc.); a compound wherein the carboxyl of the compound represented by the formula (I) is modified by esterification, amidation, or the like (e.g., a compound wherein the carboxyl of the compound represented by the formula (I) is esterified or amidated with ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, methyl amide, etc.), and the like. These compounds may be prepared by per se known method. In addition, the prodrug for the compound represented by the formula (I) may take a hydrate form or a non-hydrate form. In addition, the prodrug for the compound represented by the formula (I) may be a compound which is converted into the compound represented by the formula (I) under the physiological conditions as described in *Pharmaceutical Research and Development,* Vol. 7 "Molecular Design", pages 163-198 published in 1990 by Hirokawa Publishing Co. In addition, compound (I) may be labeled with an isotope (e.g., $^{3}H$, $^{14}C$, $^{35}S$, $^{125}I$, etc.) and the like.

Keto-enol tautomerism is included in the formula (I) of the present invention. For example, in the case where a fused ring AB represents a pyrazolo[1,5-a]pyrimidine ring and $R^1$ represents hydroxy, the following compounds are exemplified.

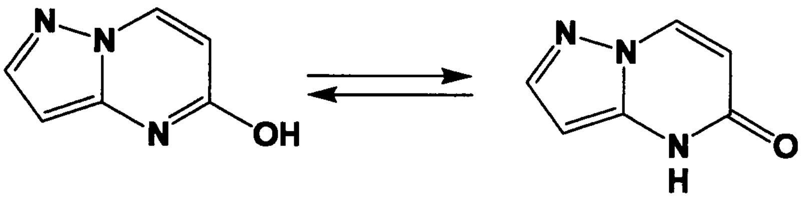

In the present invention, each group represented by the fused ring AB, $R^1$, $R^2$, and $R^3$ is all preferred. In particular, the compounds described in Examples are more preferred. In the following, preferred groups and preferred rings will be listed. All the symbols used herein have the same meanings as described above.

In the present invention, preferable example of the fused ring AB, includes, for example, a 8- to 10-membered fused unsaturated heterocyclic ring, or the like, and more preferably, for example, a pyrazolo[1,5-a]pyrimidine, quinoline, 2H-chromene ring, and the like.

In the present invention, $R^1$ is, for example, preferably a hydrogen atom, an oxo group, a halogen atom, an optionally protected amino group, an optionally protected hydroxy group, a cyclic group which may have a substituent (s), an aliphatic hydrocarbon group which may have a substituent (s) and the like.

In the present invention, n is, for example, preferably an integer of 1 to 4, more preferably, for example, an integer of 2 or 3.

Among the compounds of the present invention, represented by the formula (I), preferred are a compound represented by the formula (I-1) :

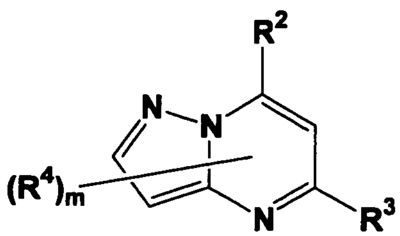

(I-1)

wherein all the symbols have the same meanings as described above, a compound represented by the formula (I-2) :

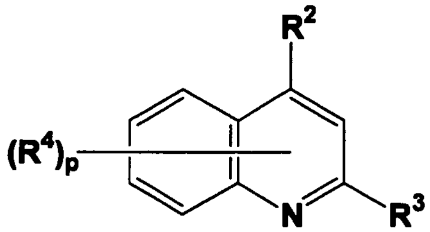

(I-2)

wherein all the symbols have the same meanings as described above, and a compound represented by the formula (I-3):

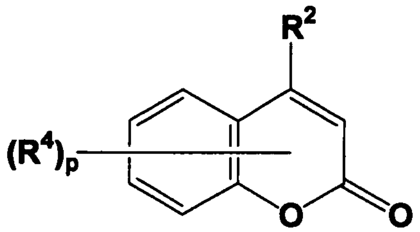

(I-3)

wherein all the symbols have the same meanings as described above, or a salt thereof, a solvate thereof or a prodrug thereof.

In the present specification, $R^2$ is preferably, for example, a monosubstituted amino group, more preferably, for example, an amino group substituted by one cyclic group which may have a substituent(s), an amino group substituted by one aliphatic hydrocarbon group which may have a substituent(s), or the like. Herein, the "cyclic group" in the "cyclic group which may have a substituent(s)" is preferably, for example, a monovalent group formed by removing an arbitrary hydrogen atom from a C5-6 monocyclic carbocyclic ring, more preferably, for example, a monovalent group formed by removing an arbitrary hydrogen atom from benzene (i.e. phenyl) and the like. The "substituent" in the "cyclic group which may have a substituent(s)" is preferably, for example, a C1-4 alkyl group substituted by a hydroxyl group, a halogen atom, and the like, more preferably, for example, 2-hydroxyethyl, chlorine and the like. Herein, the "aliphatic hydrocarbon group" in the "aliphatic hydrocarbon group which may have a substituent (s)" is preferably, for example, a C1-4 alkyl group and the like, more preferably, for example, methyl and the like. The "substituent" in the "aliphatic hydrocarbon group which may have a substituent (s)" is preferably, for example, a cyclic group which may have a substituent(s) and the like. Herein, the "cyclic group" in the "cyclic group which may have a substituent (s) " is preferably, for example, a monovalent group formed by removing an arbitrary hydrogen from a C5-6 monocyclic carbocyclic ring, a monovalent group formed by removing an arbitrary hydrogen from a 5- to 10-membered monocyclic or bicyclic heterocyclic group, more preferably, for example, a monovalent group formed by removing an arbitrary hydrogen from benzene ring, pyridine ring, 1, 3-benzodioxol ring and the like. The "substituent" in the "cyclic group which may have a substituent(s)" is preferably, for example, a C1-4 alkoxy or a halogen atom, such as methoxy, fluorine, chlorine and the like.

In the present specification, $R^3$ is preferably, for example, a halogen atom, a monovalent group formed by removing an arbitrary hydrogen from a C5-6 monocyclic carbocyclic group which may have a substituent (s), a monovalent group formed by removing an arbitrary hydrogen atom from a 5- to 10-membered monocyclic or bicyclic heterocyclic group which may have a substituent(s), and the like. Herein, the "C5-6 monocyclic carbocyclic group" in the "C5-6 monocyclic carbocyclic group which may have a substituent (s) s" is preferably, for example, benzene and the like. The "substituent" in the "C5-6 monocyclic carbocyclic group which may have a substituent (s)" is preferably, for example, a C1-4 acylamino group, a halogen atom, a C1-4 alkyl group, and more preferably, for example, acetylamino, fluorine, methyl and the like. Herein, the "5- to 10-membered monocyclic or bicyclic heterocyclic group" in the "5- to 10-membered monocyclic or bicyclic heterocyclic group which may have a substituent (s) " is preferably, for example, a 5- to 10-membered monocyclic or bicyclic unsaturated heterocyclic ring having 1 to 2 nitrogen atom(s), 1 or 2 oxygen atom(s) and/or 1 sulfur atom as a hetero atom(s) which may have a substituent(s) and the like, and more preferably, for example, furan, thiophene, quinoline and the like. The "substituent" in the "5- to 10-membered monocyclic or bicyclic heterocyclic group which may have a substituent (s) " is preferably, for example, a C1-4 alkyl group, more preferably, for example, methyl and the like. Herein, the "halogen atom" is preferably, for example, fluorine, chlorine and the like.

In the present specification, $R^4$ is preferably, for example, a hydrogen atom, an aliphatic hydrocarbon group which may have a substituent(s) and the like, more preferably a hydrogen atom, a C1-4 alkyl group, especially preferably, a hydrogen atom, methyl and the like. m is preferably, for example, 0 or 1. p is preferably, for example, 0 or 1.

In the present specification, a compound represented by the formula (I) including any combination of the above

preferable substituents is preferred. More concretely, for example, a compound wherein $R^2$ represents 4-(2-hydroxyethyl) phenylamino, 2-chlorophenylamino, 4-methoxybenzylamino, 3,4,5-trimethoxybenzylamino, 1,3-benzodioxol-5-ylmethylamino, pyridin-4-ylmethylamino, 4-fluoro-3-chlorophenylamino or the like, or $R^3$ represents thiophen-3-yl, 4-acetylaminophenyl, furan-2-yl, 4-fluorophenyl, 2-methylthiophene-5-yl, 3,4-dimethylphenyl, quinolin-3-yl, 3-fluorophenyl or the like is preferable.

In the present invention, examples of the preferable compound include, for example,

(1) N-(1,3-benzodioxol-5-ylmethyl)-5-chloropyrazolo[1,5-a]pyrimidin-7-amine,
(2) 5-chloro-N-(3-chlorophenyl)pyrazolo[1,5-a]pyrimidin-7-amine,
(3) 5-thien-3-yl-N-(3,4,5-trimethoxybenzyl)pyrazolo[1,5-a]pyrimidin-7-amine,
(4) N-(4-{7-[(4-methoxybenzyl)amino]pyrazolo[1,5-a]pyrimidin-5-yl}phenyl)acetamide,
(5) 2-{4-[(5-chloropyrazolo[1,5-a]pyrimidin-7-yl)amino]phenyl}ethanol,
(6) 5-(2-furyl)-N-(3,4,5-trimethoxybenzyl)pyrazolo[1,5-a]pyrimidin-7-amine,
(7) 5-(4-fluorophenyl)-N-(3,4,5-trimethoxybenzyl)pyrazolo[1,5-a]pyrimidin-7-amine,
(8) 5-(5-methylthien-2-yl)-N-(3,4,5-trimethoxybenzyl)pyrazolo[1,5-a]pyrimidin-7-amine,
(9) 5-(3,4-dimethylphenyl)-N-(pyridin-4-ylmethyl)pyrazolo[1,5-a]pyrimidin-7-amine,
(10) N-(pyridin-4-ylmethyl)-5-quinolin-3-ylpyrazolo[1,5-a]pyrimidin-7-amine,
(11) 5-(3-fluorophenyl)-N-(3,4,5-trimethoxybenzyl)pyrazolo[1,5-a]pyrimidin-7-amine,
(12) N-(pyridin-4-ylmethyl)-5-thien-3-ylpyrazolo[1,5-a]pyrimidin-7-amine,
(13) N-(4-methoxybenzyl)-5-thien-3-ylpyrazolo[1,5-a]pyrimidin-7-amine,
(14) 1-(3-{7-[(4-methoxybenzyl)amino]pyrazolo[1,5-a]pyrimidin-5 -yl}phenyl)ethanone,
(15) 5-pyridin-4-yl-N-(3,4,5-trimethoxybenzyl)pyrazolo[1,5-a]pyrimidin-7-amine,
(16) $N^5$-[4-(dimethylamino)phenyl]-$N^{7-}$propylpyrazolo[1,5-a]pyrimidine-5,7-diamine,
(17) 5-(3-furyl)-N-(3,4,5-trimethoxybenzyl)pyrazolo[1,5-a]pyrimidin-7-amine,
(18) 5-(3-furyl)-N-(thien-2-ylmethyl)pyrazolo[1,5-a]pyrimidin-7-amine,
(19) 5-(3-furyl)-N-(3,4,5-trimethoxyphenyl)pyrazolo[1,5-a]pyrimidin-7-amine,
(20) 5-(4-methylphenyl)-N-(4-pyridinylmethyl)pyrazolo[1,5-a]pyrimidin-7-amine,
(21) 5-(3-methoxyphenyl)-N-(4-pyridinylmethyl)pyrazolo[1,5-a]pyrimidin-7-amine,
(22) 5-(3-furyl)-N-(4-pyridinylmethyl)pyrazolo[1,5-a]pyrimidin-7-amine,
(23) N-(4-methoxybenzyl)-5-(4-methylphenyl)pyrazolo[1,5-a]pyrimidin-7-amine,
(24) N-(4-methoxybenzyl)-5-(3-methoxyphenyl)pyrazolo[1,5-a]pyrimidin-7-amine,
(25) 5-(3-furyl)-N-(4-methoxybenzyl)pyrazolo[1,5-a]pyrimidin-7-amine,
(26) {1-[5-(3-methoxyphenyl)pyrazolo[1,5-a]pyrimidin-7-yl]-2-pyrrolidinyl}methanol,
(27) 5-(4-methyl-2-thienyl)-N-(4-pyridinylmethyl)pyrazolo[1,5-a]pyrimidin-7-amine,
(28) 4-[(3-chloro-4-fluorophenyl)amino]-6-methyl-2H-chromen-2-one,
(29) 4-[(3-chloro-4-fluorophenyl)amino]-8-methyl-2H-chromen-2-one,
(30) 4-[(3-chloro-4-fluorophenyl)amino]-2H-chromen-2-one,
(31) 5-chloro-N-(4-methoxybenzyl)pyrazolo[1,5-a]pyrimidin-7-amine,
(32) 5-chloro-N-(4-methoxybenzyl)-2-methylpyrazolo[1,5-a]pyrimidin-7-amine,
(33) 5-chloro-N-(4-methoxybenzyl)-3-methylpyrazolo[1,5-a]pyrimidin-7-amine,
(34) N-(4-methoxybenzyl)-5-methylpyrazolo[1,5-a]pyrimidin-7-amine,
(35) N-(4-methoxybenzyl)-2,5-dimethylpyrazolo[1,5-a]pyrimidin-7-amine,
(36) N-(4-methoxybenzyl)-3,5-dimethylpyrazolo[1,5-a]pyrimidin-7-amine,
(37) N-(4-methoxybenzyl)-5-phenylpyrazolo[1,5-a]pyrimidin-7-amine,
(38) N-(4-methoxybenzyl)-2-methyl-5-phenylpyrazolo[1,5-a]pyrimidin-7-amine,
(39) N-(4-methoxybenzyl)-3-methyl-5-phenylpyrazolo[1,5-a]pyrimidin-7-amine, and the like.

Examples of particularly preferable compounds include, for example,

(1) N-(4-{7-[(4-methoxybenzyl)amino]pyrazolo[1,5-a]pyrimidin-5-yl}phenyl)acetamide,
(2) N-(1,3-benzodioxol-5-ylmethyl)-5-chloropyrazolo[1,5-a]pyrimidin-7-amine,
(3) 2-{4-[(5-chloropyrazolo[1,5-a]pyrimidin-7-yl)amino]phenyl}ethanol,
(4) 5-chloro-N-(3-chlorophenyl)pyrazolo[1,5-a]pyrimidin-7-amine,
(5) 5-(2-furyl)-N-(3,4,5-trimethoxybenzyl)pyrazolo[1,5-a]pyrimidin-7-amine,
(6) 5-thien-3-yl-N-(3,4,5-trimethoxybenzyl)pyrazolo[1,5-a]pyrimidin-7-amine,
(7) 5-(3,4-dimethylphenyl)-N-(pyridin-4-ylmethyl)pyrazolo[1,5-a]pyrimidin-7-amine,
(8) N-(pyridin-4-ylmethyl)-5-quinolin-3-ylpyrazolo[1,5-a]pyrimidin-7-amine,
(9) 5-(3-fluorophenyl)-N-(3,4,5-trimethoxybenzyl)pyrazolo[1,5-a]pyrimidin-7-amine,

(10) N-(pyridin-4-ylmethyl)-5-thien-3-ylpyrazolo[1,5-a]pyrimidin-7-amine,
(11) 5-pyridin-4-yl-N-(3,4,5-trimethoxybenzyl)pyrazolo[1,5-a]pyrimidin-7-amine,
(12) 5-(3-furyl)-N-(3,4,5-trimethoxybenzyl)pyrazolo[1,5-a]pyrimidin-7-amine,
(13) 5-(3-furyl)-N-(thien-2-ylmethyl)pyrazolo[1,5-a]pyrimidin-7-amine,
(14) 5-(3-furyl)-N-(3,4,5-trimethoxyphenyl)pyrazolo[1,5-a]pyrimidin-7-amine,
(15) 5-(4-methylphenyl)-N-(4-pyridinylmethyl)pyrazolo[1,5-a]pyrimidin-7-amine,
(16) 5-(3-methoxyphenyl)-N-(4-pyridinylmethyl)pyrazolo[1,5-a]pyrimidin-7-amine,
(17) 5-(3-furyl)-N-(4-methoxybenzyl)pyrazolo[1,5-a]pyrimidin-7-amine,
(18) {1-[5-(3-methoxyphenyl)pyrazolo[1,5-a]pyrimidin-7-yl]-2-pyrrolidinyl}methanol,
(19) 5-(4-methylthien-2-yl)-N-(4-pyridinylmethyl)pyrazolo[1,5-a]pyrimidin-7-amine,
(20) 4-[(3-chloro-4-fluorophenyl)amino]-6-methyl-2H-chromen-2-one, and the like.

All isomers are included in the present invention, unless otherwise specified. For example, an alkyl, an alkoxy, and an alkynylene group includes a straight or branched one. In addition, isomers on double bond, ring, fused ring (E-, Z-, cis-, trans-isomers), isomers generated due to asymmetric carbon atom(s) (R-, S-, α-, β-isomers, enantiomer, diastereomer), optically active isomers with optical rotation (D-, L-, d-, 1-isomers), polar compounds generated by chromatographic separation (highly polar compound, low polar compound), equilibrium compounds, mixtures thereof with arbitrary ratio and racemic mixtures are also included in the present invention. Further, isomers due to the tautomerism are all included in the present invention.

[salt]

In the present invention, the compound represented by the formula (I) may form a salt thereof, and may be N-oxide form thereof or quaternary ammonium salt thereof. Furthermore, these compounds may be a solvate thereof. The compounds of the present invention include all pharmacologically acceptable salts of the compound represented by the formula (I). As pharmacologically acceptable salts, water-soluble salts with little toxicity are preferred. Suitable pharmacologically acceptable salts in the compound of the present invention include, for example, salts of alkali metals (such as potassium, sodium, lithium, and the like); salts of alkaline earth metals (such as calcium, magnesium, and the like); ammonium salts (such as tetramethylammonium salts, tetrabutylammonium salts, and the like); salts of organic amines (such as triethylamine, methylamine, dimethylamine, cyclopentylamine, benzylamine, phenethylamine, piperidine, monoethanolamine, diethanolamine, tris(hydroxylmethyl)methylamine, lysine, arginine, N-methyl-D-glucamine, and the like); and acid addition salts such as salts of inorganic acid (such as hydrochloride, hydrobromide,hydroiodide,sulfate, phosphate,nitrate,and the like), and salts of organic acid (such as, acetate, trifuloroacetate, lactate, tartrate, oxalate, fumarate, maleate, benzoate, citrate, methansulfonate, ethansulfonate, benzenesulfonate, toluenesulfonate, isethionate, gulcuronate, gluconate and the like), and the like. The N-oxide form of the compound represented by the formula (I) means the compound wherein the nitrogen atom was oxidized. The N-oxide form of the compound represented by the formula (I) may additionally form a salt described above. The quaternary ammonium salt of the compound represented by the formula (I) means the compound wherein the nitrogen atom of the compound represented by the formula (I) is quaternized by R° (R° represents an aliphatic hydrocarbon group (which has the same meaning as described above) which may have a substituent (s), and a cyclic group (which has the same meaning as described above) which may have a substituent (s).) The quaternary ammonium salt of the compound represented by the formula (I) may additionally form the salt described above and the N-oxide form described above. The appropriate solvate of the compound represented by the formula (I), a salt thereof, an N-oxide form thereof, and a quaternary ammonium salt thereof, include water, alcohol solvate (such as ethanol) and the like. The solvates are preferably nontoxic and water-soluble. The compounds represented by the formula (I) can be converted into the salt described above, the N-oxide form described above thereof, the solvates described above by conventional means.
The nomenclature and numberling of the compounds used in the present specification is performed using a computer program conducting designation generally according to IUPAC regulations, ACD/Name (registered trademark, version 5.08/17, Advanced Chemistry Development Inc.). For example, the compound:

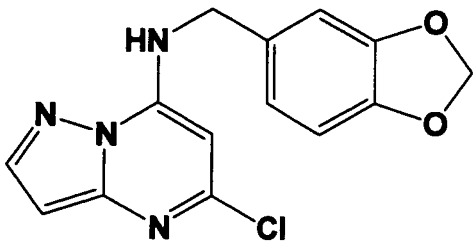

is named as N-(1,3-benzoxol-5-ylmethyl)-5-chloropyrazolo[1,5-a]pyrimidin-7-amine.

Method of producing the compound of the present invention

A compound represented by the formula (I) may be prepared by modifying or combining known methods, for example, methods shown below, methods described in Examples, or methods described in *Comprehensive Organic Transformations: A Guide to Functional Group Preparations,* 2nd Edition (Richard C. Larock, John Wiley & Sons Inc., 1999, or other methods. Furthermore, the starting compound may be used in the form of a salt. An example of the salt used includes a salt of the compound of the formula (I) described above.

Among the compounds represented by the formula (I), a compound in which fused ring AB is pyrazolo[1,5-a] pyrimidine, i.e., a compound represented by the formula (I-A):

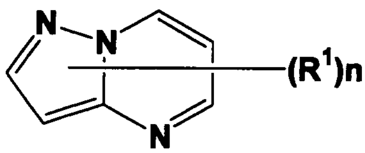

**(I-A)**

wherein all the symbols have the same meanings as described above;
can be prepared by any one of the methods of (a) to (d) shown below.
(a): A compound represented by the formula (I-A) can be prepared by subjecting a compound represented by the formula (II-1):

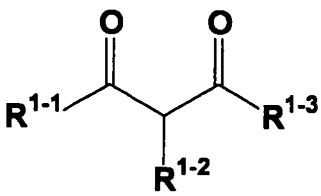

**(II-1)**

wherein $R^{1-1}$, $R^{1-2}$, and $R^{1-3}$ each independently have the same meanings as $R^1$ described above;
a compound represented by the formula (II-2) :

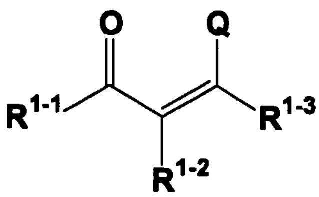

**(II-2)**

wherein $R^{1-1}$, $R^{1-2}$, and $R^{1-3}$ have the same meanings as described above and Q represents an optionally protected amino group; or a compound represented by the formula (II-3) :

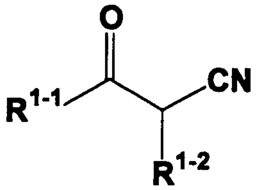

(II-3)

wherein all the symbols have the same meanings as described above;
and a compound represented by the formula (III):

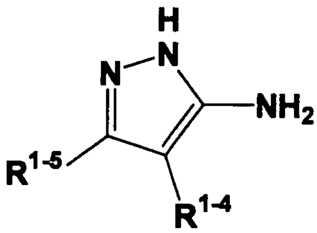

(III)

wherein $R^{1-4}$ and $R^{1-5}$ each independently have the same meanings as $R^1$ described above;
to ring formation reaction, followed by optional deprotection reaction.

This ring formation reaction is known and is carried out by a known method, for example, by heating in the presence of an organic acid (e.g. acetic acid, trifluoroacetic acid, methanesulfonic acid, p-toluenesulfonic acid, etc.) or an organic amine (e.g. piperidine, pyridine, etc.) in the absence or presence of an organic solvent [for example, a benzene solvent (e.g. benzene, toluene, xylene, chrolobenzene, etc.), an ether solvent (e.g. dioxane, etc.), an alcohol solvent (e.g. ethanol, butanol, etc.), hermetically or non-hermetically at about 50°C to reflux temperature for about 10 minutes to 24 hours, preferably about 30 minutes to 5 hours. Herein, amino group represented by Q which has ability to leave include, for example, the amino group of which two hydrogen atoms are substituted by arbitrary substituents and the like. Concretely, Q is, for example, N,N-dialkylamino group such as N,N-dimethlamino, N,N-diethylamino, etc. and the like.

The compound represented by the formula (I-A) wherein at least one of group(s) has a carboxyl group, a hydroxy group, an amino group or a thiol group can be prepared by subjecting the compound in which the respective groups are protected, to deprotection reaction.

A protecting group for a carboxyl group includes, for example, methyl, ethyl, allyl, t-butyl, trichloroethyl, benzyl (Bn), phenacyl, and the like.

A protecting group for a hydroxy group includes, for example, methyl, trityl, methoxymethyl (MOM), 1-ethoxyethyl (EE), methoxyethoxymethyl (MEM), 2-tetrahydropyranyl (THP), trimethylsilyl (TMS), triethylsilyl (TES), t-butyldimethylsilyl (TBDMS), t-butyldiphenylsilyl (TBDPS), acetyl (Ac), pivaloyl, benzoyl, benzyl (Bn), p-methoxybenzyl, allyloxycarbonyl (Alloc), 2,2,2-trichloroethoxycarbonyl (Troc), and the like.

A protecting group for an amino group includes, for example, benzyloxycarbonyl, t-butoxycarbonyl, allyloxycarbonyl (Alloc), 1-methyl-1-(4-biphenyl)ethoxycarbonyl (Bpoc), trifluoroacetyl, 9-fluororenylmethoxycarbonyl, benzyl (Bn), p-methoxybenzyl, benzyloxymethyl (BOM), 2-(trimethylsilyl)ethoxymethyl (SEM), and the like.

A protecting group for a thiol group includes, for example, benzyl, methoxybenzyl, methoxymethyl (MOM), 2-tetrahydropyranyl (THP), diphenylmethyl, acetyl(Ac), and the like.

A protecting group for a carboxyl group, a hydroxy group, an amino group or a thiol group is not particularly limited in addition to the above-described groups as long as it can be deprotected easily and selectively. For example, those described in *Protective Groups in Organic Synthesis* (T. W. Greene, John Wiley & Sons Inc., 1999) may be used.

The deprotection reaction of the protecting group for a carboxyl group, a hydroxy group, an amino group or a thiol group is well known. For example, it is

(1) alkaline hydrolysis,
(2) deprotection of a protecting group in acidic conditions,
(3) deprotection of a protecting group by hydrogen atomolysis,
(4) deprotection of a protecting group containing silyl,
(5) deprotection of a protecting group using a metal,
(6) deprotection of a protecting group using an organometal, and the like.

In the following, these methods are specifically described:

(1) The deprotection of the protecting group by alkaline hydrolysis may be carried out, for example, in an organic solvent (methanol, tetrahydrofuran, 1,4-dioxane, ethylene glycol, etc.) with an alkaline metal hydroxide (sodium hydroxide, potassium hydroxide, lithium hydroxide, etc.), an alkaline earth metal hydroxide (barium hydroxide, calcium hydroxide, etc.), a carbonate (sodium carbonate or potassium carbonate, etc.), an aqueous solution thereof or a mixture thereof at 0 to 200°C.
(2) The deprotection of the protecting group in acidic conditions may be carried out, for example, in an organic solvent (methylene chloride, chloroform, 1,4-dioxane, ethyl acetate, anisole, etc.), an organic acid (acetic acid, trifluoroacetic acid, methanesulfonic acid, p-toluenesulfonic acid, *etc.),* an inorganic acid (hydrochloric acid, sulfuric acid, etc.), or a mixture thereof (hydrogen bromide/acetic acid, etc.) at 0 to 200°C.
(3) The deprotection of the protecting group by hydrogenolysis may be carried out, for example, in a solvent (ethers (tetrahydrofuran, 1,4-dioxane, 1,2-dimethoxyethane, diethyl ether, etc.), alcohols (methanol, ethanol, etc.), benzenes (benzene, toluene, etc.), ketones (acetone, methyl ethyl ketone, *etc.*), nitriles (acetonitrile, etc.), amides (N,N-dimethylformamide, etc.), water, ethyl acetate, acetic acid, a mixture of two or more thereof, etc.) in the presence of a catalyst (palladium on carbon, palladium black, palladium hydroxide, platinum oxide, Raney nickel, etc.) under hydrogen atmosphere at a normal pressure or elevated pressure, or in the presence of ammonium formate at 0 to 200°C.
(4) The deprotection of the protecting group for silyl may be carried out, for example, in an organic solvent which can be mixed uniformly with water (tetrahydrofuran, acetonitrile, etc.), with fluoride (tetrabutylammonium fluoride, an aqueous solution of hydrogen fluoride, hydrogen fluoride-pyridine complex, etc.) at -20 to 40°C.
(5) The deprotection of the protecting group using a metal may be carried out, for example, in an acidic solvent (acetic acid, a buffer of pH 4.2 to 7.2, a mixed solution of the buffer and an organic solvent such as tetrahydrofuran, etc.) in the presence of zinc powder, with or without an ultrasonic wave at a temperature of 0 to 40°C.
(6) The deprotection of the protecting group using a metal complex may be carried out, for example, in an organic solvent (methylene chloride, N,N-dimethylformamide, tetrahydrofuran, ethylacetate, acetonitrile, 1,4-dioxane, ethanol, etc.), water or a mixed solvent thereof in the presence of a trap reagent (tributyltin hydride, triethylsilane, dimedone, morpholine, diethylamine, pyrrolidine, etc.), an organic acid (acetic acid, formic acid, 2-ethylhexanic acid, etc.) and/or an organic acid salt (sodium 2-ethylhexanate, potassium 2-ethylhexanate, etc) in the presence or absence of a phosphine reagent (triphenylphosphine, etc.) using a metal complex (tetrakis(triphenylphosphine)palladium(0), dichlorobis(triphenylphosphine)palladium(II), palladium (II) acetate, chlorotris(triphenylphosphine)rhodium (I), etc.) at 0 to 40°C.

In addition to the above methods, the deprotection reaction may be carried out by the method described in *Protective Groups in Organic Synthesis* (T. W. Greene, John Wiley & Sons Inc., 1999).
By using these deprotection reaction which persons skilled in the art can easily use, the objective compound of the present invention can be prepared.
(b): Among the compounds represented by the formula (I-A), a compound in which at least one of $R^1$(s) represents a halogen atom, i.e., a compound represented by the formula (I-A-b):

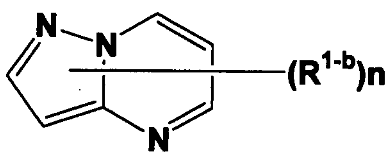

**(I-A-b)**

wherein $R^{1-b}$ represents the same meaning of $R^1$, provided that at least one of $R^{1-b}$ (s) represents a halogen atom and the other symbols have the same meaning as described above) can be prepared by the above method (a). That is, the compound (I-A-b) can be prepared by subjecting a compound in which at least one of $R^1$(s) represents a hydroxy group, i.e., a compound represented by the formula (I-A-a-1):

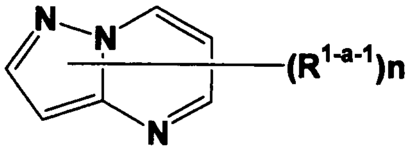 (I-A-a-1)

wherein $R^{1-a-1}$ represents the same meaning of $R^1$, provided that at least one of $R^{1-a-1}$ (s) represents a hydroxy group and the other symbols have the same meaning as described above;
to halogenation, followed by optional deprotection reaction.

The halogenation is carried out by a known method, for example, by the reaction with or without an organic solvent (dichrolomethane, chloroform, benzene, toluene, xylene, chlorobenzene, acetonitrile, etc.), in the presence or absence of a catalyst (dimehtylformamide, dimethylaminopyridine, N,N-dimehtylaniline, etc.), using a halogenating agent (phosphorus oxychloride, phosphorus trichloride, phosphorus tribromide, phosphorus pentachloride, phosphorus pentabromide, thionyl chloride, etc.) at about -10 to 150°C.

The deprotection reaction can be carried out by the above described method.
(c) : Among the compounds represented by the formula (I-A), a compound in which any of $R^1$s is not a halogen atom, i.e., a compound represented by the formula (I-A-c):

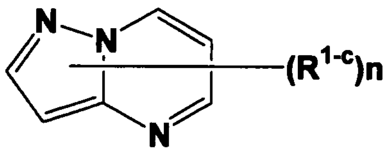 (I-A-c)

wherein $R^{1-c}$ has the same meaning of $R^1$, provided that any of $R^1$s is not a halogen atom and the other symbols have the same meanings as described above) can be prepared by the methods of (c-1) to (c-3) shown below.
(c-1) A compound in which at least one of $R^{1-c}$(s) represents a cyano group, an optionally protected hydroxy group, an optionally protected amino group, a cyclic group which may have a substituent(s), or an aliphatic hydrocarbon which may have a substituent(s), i.e., a compound represented by the formula (I-A-c-1):

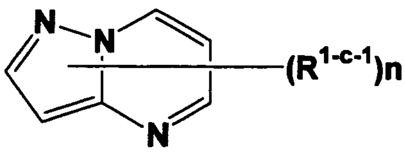 (I-A-c-1)

wherein $R^{1-c-1}$ has the same meaning as in $R^{1-c}$, provided that at least one of $R^{1-c}$(s) represents a cyano gtoup, an optionally protected hydroxy group, an optionally protected amino group, a cyclic group which may have a substituent (s) or an aliphatic hydrocarbon which may have a substituent (s) and the other symbols have the same meanings as described above) can be prepared by subjecting a compound represented by the formula (I-A-b) and a compound represented by the formula (IV):

$R^{1-C-1}$—y (IV)

wherein Y represents hydrogen atom, alkali metal (such as lithium, sodium, potassium, etc.), magnesium halide (such as magnesium chloride (MgCl), magnesium bromide (MgBr), magnesium iodide (MgI), etc.), zinc halide (such as zinc bromide (ZnBr), zinc iodide (ZnI), etc.), boronic acid, boronic ester (such as methyl borate, isopropyl borate, etc.), copper, alkylsilyl (such as trimethylsilyl, etc.), and other symbols have the same meanings as described above) to amination, etherification, alkylation, cyanation or coupling reaction and if necessary, to deprotection reaction.

The amination reaction in the case where $R^{1-c-1}$ represents an optionally protected amino group and Y represents a hydrogen atom, is carried out by a known method, for example, by the reaction with or without an inert organic solvent (dimethylformamide, dimethyl sulfoxide, chloroform, methylene chloride, diethyl ether, tetrahydrofuran, methanol, ethanol, benzene, toluene, xylene, etc.), in the presence or absence of a base [alkali metal hydroxide (such as sodium hydroxide, potassium hydroxide, lithium hydroxide, etc.), alkali earth metal hydroxide (such as barium hydroxide, calcium hydroxide, etc.) or carbonate (such as sodium carbonate, potassium carbonate, cesium carbonate, etc.) or aqueous solution thereof, or these mixture, alkali metal hydride (such as sodium hydride, potassium hydride, etc.), alkali metal alkoxide (such as sodium methoxide, sodium ethoxide, potassium t-butoxide, etc.), alkali metal amide (such as sodium amide, potassium amide, etc.), amine (such as triethylamine, tributhylamine, diisopropylethylamine, N,N-dimethylaniline, pyridine, lutidine, collidine, 4-(dimethylamino)pyridine, etc.) etc.] at 0 to 150°C.

The etherification in the case where $R^{1-c-1}$ represents an optionally protected hydroxy group and Y represents a hydrogen atom, is carried out by a known method, for example, by the reaction in an inert organic solvent (dimethylformamide, dimethyl sulfoxide, chloroform, methylene chloride, diethyl ether, tetrahydrofuran, methanol, ethanol, benzene, toluene, xylene, etc.), in the presence of a base [an alkali metal hydroxide (such as sodium hydroxide, potassium hydroxide, lithium hydroxide, etc.), an alkaline earth metal hydroxide (such as barium hydroxide, calcium hydroxide, etc.) or a carbonate (such as sodium carbonate, potassium carbonate, cesium carbonate, etc.), or an aqueous solution thereof, or these mixture, an alkali metal hydride (such as sodium hydride, potassium hydride, etc.), an alkali metal alkoxide (such as sodiummethoxide, sodiumu ethoxide, potassium t-butoxide, etc.), an alkali metal amide (such as sodium amide, potassium amide, etc.), an amine (such as triethylamine, tributhylamine, diisopropylethylamine, N,N-dimethylaniline, pyridine, lutidine, collidine, 4- (dimethylamino) pyridine at 0 to 150°C.

The alkylation reaction in the case where $R^{1-c-1}$ represents aliphatic hydrocarbon group which may have a substituent (s) and Y represents an alkali metal, magnesium halide, and zinc halide is carried out by a known method, for example, by the reaction in an organic solvent (such as tetrahydrofuran, diethyl ether, etc.), in the presence or absence of a catalyst (1,3-bis(diphenylphosphino)propane]dichloronickel(II) ($NiCl_2$ (dppp), *etc.*) at about -78 to 40°C. This reaction is preferably carried out under the atmosphere of an inert gas (such as nitrogen gas, argon gas, etc.).

The cyanation reaction in the case where $R^{1-c-1}$ represents cyano and Y represents an alkali metal (such as lithium, sodium, potassium, etc.), copper, an alkylsilyl group (such as trimethylsilyl, etc.) is carried out by a known method, for example, by the reaction with or without an organic solvent (such as dimethylformamide, dioxane, acetonitrile, N-methylpyrrolidone, hexamethylphosphoramide, etc.), in the presence or absence of a catalyst (such as tetrakis(triphenylphosphine)palladium ($Pd(PPh_3)_4$), tetrakis (triphenylphosphine) nickel (Ni $(PPh_3)_4$) and a tertiary amine at room temperature to 120°C.

The coupling reaction in the case where $R^{1-c-1}$ represents a cyclic group which may have a substituent (s) and Y represents boronic acid or boronic ester is carried out by a known method, for example, in an organic solvent (such as benzene, toluene, dimethylformamide, dioxane, tetrahydrofuran, methanol, acetonitrile, dimethoxyethane, aceton, etc.) in the presence of a base (sodium ethylate, sodium hydroxide, potassium hydroxide, triethylamine, sodium carbonate, sodium bicarbonate, potassium carbonate, cesium carbonate, thallium carbonate, tripotassium phosphate, cesium fluoride, barium hydroxide, tetrabutylammonium fluoride, etc.) or an aqueous solution thereof, or a mixture thereof, and a catalyst (such as tetrakis(triphenylphosphine)palladium ($Pd(PPh_3)_4$), dichlorobis(triphenylphosphine)palladium ($PdCl_2$ $(PPh_3)_2$), palladium acetate ($Pd(OAc)_2$), palladium black, 1,1'-bis(diphenylphosphinoferrocene)dichloropalladium ($PdCl_2$ $(dppf)_2$), dichlorodiallyl palladium ($PdCl_2(allyl)_2$), iodophenylbis(triphenylphosphine)palladium (PhPdI $(PPh_3)_2$), etc.) at room temperature to 120°C.

The deprotection reaction can be carried out by the method mentioned above.

(C-2): A compound in which at least one of $R^{1-c}$ (s) represents an optionally protected carboxyl group, i.e., a compound represented by the formula (I-A-c-2):

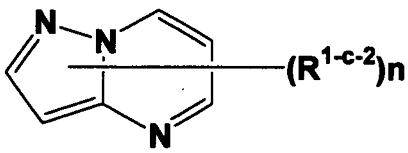

(I-A-c-2)

wherein $R^{1-c-2}$ has the same meaning as $R^{1-c}$, provided that at least one of $R^{1-c-2}$ (s) represents an optionally protected carboxyl group, and the other symbols have the same meanings as described above; can be prepared by subj ecting the compound represented by formula (I-b) to carboxyl insertion reaction, and if necessary, deprotection reaction.

The carboxyl insertion reaction is carried out by a known method, for example, (1) the reaction in an organic solvent (such as toluene, etc.) in the presence or absence of an alcohol (such as methanol, ethanol, etc.) or water and in presence of a catalyst (such as tetrakis(triphenylphosphine)palladium ($Pd(PPh_3)_4$), palladium acetate ($Pd(OAc)_2$), palladium-carbon, etc.) under carbon dioxide atmosphere;

(2) the reaction in an organic solvent (such as dimethylformamide, etc.) in the presence of a quaternary ammonium salt

(such as tetrabuthylammonium bromide, tetrabuthyl ammonium fluoroborate under carbon dioxide atmosphere.

The deprotection reaction can be carried by the method described above.

(C-3) : A compound in which at least one of $R^{1-c}$ (s) represents carboxyl group, i.e., a compound represented by the formula (I-A-c-3):

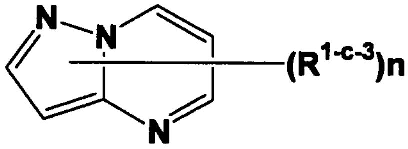

(I-A-c-3)

wherein $R^{1-c-3}$ has the same meaning as $R^{1-c}$, provided that at least one of $R^{1-c-3}$ (s) represents an optionally protected carboxyl group, and the other symbols have the same meanings as described above) can be prepared by subjecting a compound having at least one cyano group prepared by the above described method, i.e., a compound represented by the formula (I-A-c-1-1):

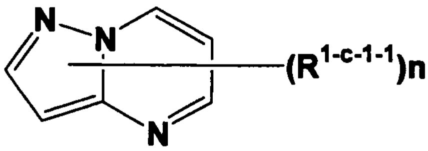

(I-A-c-1-1)

wherein $R^{1-c-1-1}$ has the same meaning as $R^{1-c-1}$, provided that at least one of $R^{1-c-1-1}$ (s) represents cyano group, and the other symbols have the same meanings as described above) to hydrolysis reaction, followed by optional deprotection reaction.

The hydrolysis reaction includes alkali hydrolysis reaction and acid hydrolysis reaction. Each reaction can be carried out by a known method.

The alkali hydrolysis reaction is carried out by the same method as the deprotection reaction by the alkali hydrolysis reaction. The acid hydrolysis reaction is carried out in a similar manner to the deprotection reaction under acidic conditions.

The deprotection reaction can be carried by the method described above.

(d): Among the compounds represented by the formula (I-A), a compound in which one substituent represents an optionally protected amino group, i.e., a compound represented by the formula (I-A-d):

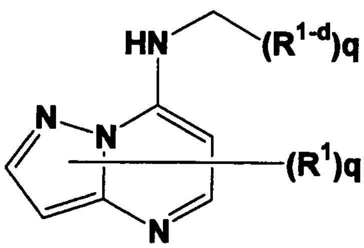

(I-A-d)

wherein $R^{1-d}$ represents an optionally protected amino group, and q represents 0 or an integer of 1 to 7) can be prepared by subjecting the compound prepared by the method described above (a), i.e., a compound represented by the formula (I-A-a-2) :

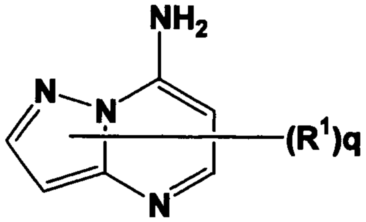

(I-A-a-2)

wherein all the symbols have the same meanings as described above) to reductive amination reaction, followed by optional deprotection reaction.

The reductive amination reaction is carried out by a known method, for example, by the reaction in an organic solvent (such as dichloroethane, dichloromethane, dimethylformamide, acetic acid and a mixture thereof, etc.), in the presence of reductant (such as sodium triacetoxyborohydride, sodium cyanotriacetoxyborohydride, sodium cyanoborohydride, etc.) at 0 to 40°C.

Among the compounds represented by the formula (I), a compound in which the fused ring AB represents a heterocyclic group other than pyrazolo [1, 5-a] pyrimidine ring can be prepared in a similar manner to the above method (b), (c) or (d), using a starting material such as a compound wherein at least one of $R^1$(s) represents a hydroxy group, a halogen atom, or an amino group.

The compounds represented by the formulae (II-l), (II-2), or (III) to be used as the starting materials or the reagents are known compounds, and they can be prepared easily by the combination of known methods (such as the method described in Comprehensive Organic Transformations : A Guide to Functional Group Preparations, 2nd Edition (Richard C. Larock, John Wiley & Sons Inc, 1999) and the like) . For example, among the compounds represented by the formula (II-1), dimethyl malonate (CAS registry number: 108-59-8), diethyl malonate (CAS registry number: 105-53-3), diethyl methyl malonate (CAS registry number: 609-08-5), diethyl phenyl malonate (CAS registry number: 83-13-6) are known compounds. In addition, among the compounds represented by the formula (III), for example, 1H- pyrazol-5-amine (CAS registry number: 1820-80-0), 3-methyl-1H-pyrazol-5-amine (CAS registry number: 31230-17-8) and the like are known compounds.

Among the compounds represented by the formula (I), compounds in which the fused ring AB represents a pyrazolo[1,5-a]pyrimidine ring and at least one of $R^1$(s) represents a hydroxy group, a halogen atom or an amino group, such as 5-chloropyrazolo[1,5-a]pyrimidin-7-amine (CAS registry number: 245095-96-9), 7-amino-2-methylpyrazolo[1,5-a]pyrimidin-5 (4H) -one (CAS registry number: 96335-50-1), 7-aminopyrazolo[1,5-a]pyrimidin-5(4H)-one (CAS registry number: 89418-10-0), 7-hydroxypyrazolo[1,5-a]pyrimidin-5(4H)-one (CAS registry number: 57489-70-0), 5,7-dichloropyrazolo[1,5-a]pyrimidine (CAS registry number: 57489-77-7) and the like, are known compounds.

Furthermore, compounds in which the fused ring AB represents a pyrazolo[1,5-a] pyrimidine ring, one of $R^1$s represents a cyclic group and the other $R^1$ represents a halogen atom or an amino group, such as 7-chloro-5-cyclohexylpyrazolo[1,5-a]pyrimidine (CAS registry number: 189018-71-1), 7-chloro-5-(3-thienyl)pyrazolo[1,5-a]pyrimidine (CAS registry number: 183958-57-8), 7-chloro-5-(2-thienyl)pyrazolo[1,5-a]pyrimidine (CAS registry number: 183958-56-7), 7-chloro-5-(3-furanyl)pyrazolo[1,5-a]pyrimidine (CAS registry number: 183958-55-6), 7-chloro-5-(2-furanyl)pyrazolo[1,5-a]pyrimidine (CAS registry number: 183958-54-5), 5-(3-furanyl)pyrazolo[1,5-a]pyrimidin-7-amine (CAS registry number: 174859-76-8), 5-(2-furanyl)pyrazolo[1,5-a]pyrimidin-7-amine (CAS registry number: 174859-75-7), 5-(3-thienyl)pyrazolo [1,5-a]pyrimidin-7-amine (CAS registry number: 174859-67-7), 5-(2-thienyl)pyrazolo[1,5-a]pyrimidin-7-amine (CAS registry number: 174859-66-6), 5-(2-methoxyphenyl)pyrazolo[1,5-a]pyrimidin-7-amine (CAS registry number: 174859-71-3), 5-(3-methoxyphenyl)pyrazolo[1,5-a]pyrimidin-7-amine (CAS registry number: 174859-72-4), 5-(4-methoxyphenyl)pyrazolo[1,5-a]pyrimidin-7-amine (CAS registry number: 174859-65-5), 5-phenylpyrazolo[1,5-a]pyrimidin-7-amine (CAS registry number: 93669-80-8), 7-chloro-5-phenylpyrazolo[1,5-a]pyrimidine (CAS registry number: 33149-25-6) and the like are known compounds.

Furthermore, among the compounds in which the fused ring AB represents a heterocyclic group other than pyrazolo[1,5-a]pyrimidine, compounds in which at least one of $R^1$(s) represents a hydroxy group, a halogen atom, or an amino group, such as 4-amino-2H-chromen-2-one (CAS registry number: 53348-92-8), 4-chloro-6-fluoro-2H-chromen-2-one (CAS registry number: 138709-42-9), 4-chloro-6-(trifluoromethoxy)-2H-chromen-2-one (CAS registry number: 174013-28-6), 4-bromo-2H-chromen-2-one (CAS registry number: 938-40-9), 4-chloro-2H-chromen-2-one (CAS registry number: 17831-88-8), 4-chloro-6-methyl-2H-chromen-2-one (CAS registry number: 51069-75-1), 4-chloro-6-methoxy-2H-chromen-2-one (CAS registry number: 71797-98-3), 2,4-dichloroquinoline (CAS registry number: 703-61-7), 4-amino-6-methylquinolin- 2(1H)-one (CAS registry number: 159680-41-8), 2,4- dichloroquinolin-6-carbonitrile (CAS registry number: 150453-93-3), quinolin-2,4-diamine (CAS registry number: 146136-78-9), 2,4-dibromo-6-methylquinoline (CAS registry number: 139719-21-4), 2,4-dichloro-6-methylquinoline (CAS registry number: 102878-18-2), 2-chloroquinolin-4-amine (CAS registry number: 80947-25-7), 2,4-dichloro-6- methoxyquinoline (CAS registry number: 70049-46-6), 2-bromoquinolin-4-amine (CAS registry number: 36825-35-1), 6-methylquinolin-2,4-diamine (CAS registry number: 1955-64-2) and the like are known compounds.

In each reaction described in the specification, it may be possible to use a solid phase reagent which is appropriately supported on a polymer (for example, polystyrene, polyacrylamide, polypropylene, polyethyleneglycol, etc.).

In the present specification, the end products obtained in the final reaction may be purified by conventional techniques. For example, the purification may be carried out by distillation at atmospheric or reduced pressure, high performance liquid chromatography with silica gel or magnesium silicate, thin layer chromatography, column chromatography, washing or recrystallization. The purification may be done in each reaction or after several reactions.

In the present specification, the reactions with heating, as will be apparent to those skilled in the art, may be carried with water bath, oil bath, sand bath or microwave.

Pharmacological Activity

A pharmacological test except for the Biological Examples hereinafter described includes, for example, the following method. By the method shown below, the in vivo effect of the compound represented by the formula (I) can be proved. A vehicle used for the administration of the compound represented by the formula (I) to an animal may be any material so long as it can suspend or dissolve the compound into safe and administrable state. For example, it is possible to appropriately select and use vehicles which those skilled in the art use for the administration to an animal, and examples of such vehicles are methylcellulose, carboxymethyl cellulose, hydroxypropyl cellulose, hydroxypropylmethyl cellulose, propylene glycol, polyethylene glycol, sugar, sugar alcohol, edible oil, distilled water, physiological saline solution, and a mixture thereof and the like.

(1) Consideration of the improvement in insulin-resistance on diabetes model of KKAy mice
Male, 8-weeks old KKAy/Ta Jcl mice are pre-breaded individually in single cages for approximately one week. During pre-breaded and test term, mice are provided pellet diet and tap water from bottle of feed water *ad libitum.* On the first day of the experiment (Day 0), the body weight of mice are measured. Blood samples are collected from coccygeal vein using a microcapillary to measure plasma glucose concentration. Based on plasma glucose concentration, mice are divided into some groups (five mice per group) using a stratified randomization method and started dosing. The dosing can be carried out by oral gavage administration compulsorily or parenteral administration by subcutaneous injection, after suspending or dissolving the test compound in the above described vehicle. The control group receives preferably only the vehicle. The doses and the administration frequency can be increased or decreased appropriately by the effect of the test compound, preferably, for example, about 0.1 mg/kg body weight to 100 mg/kg body weight, about 1 to 3 times par day, every day. The endpoints of the efficacy on the present model include body weight, food intakes, blood glucose level, triglycereide of plasma, insulin, weight of liver. These endpoints can be measured after arbitrary days from starting the dosing. For example, on the second day (Day 2) to seventh day (Day 7) from starting dosing, the efficacy on the model can be confirmed by measuring these endpoints. On the model, pioglitazone can show the efficacy such as body weight gain, decline of blood glucose level and insulin level, and the like, by oral administration one time per day and from 50 mg/kg body weight.

(2) Consideration of the inhibition of the TNF-α production on cytokine-producing mouse model
Male balb/c mice are administrated (preferably orally administrated) the test compound suspended or dissolved in the above described vehicle, and after 30 minutes, LPS (055:B5, Difco) is administrated intraperitoneally at the dose of 1 mg/kg body weight (five mice per group). The control group (five mice) receives only the vehicle. After 30 minutes from LPS treatment, under ether anesthesia, blood samples are collected from vein abdominalis with heparin and centrifuged (12000 r.p.m., 3 minutes, 4°C) to obtain plasma samples. Obtained samples are conserved at -80°C before use. The amount of TNF-α can be quantitatively analyzed by using a commercial ELISA kit (such as, R&D: #MTA00) and the like.

(3) Consideration of the inhibition of the TNF-α production on cytokine-producing rat model

Female Lew rats are administrated (preferably orally administrated) the test compound suspended or dissolved in the above described vehicle, and after 2 hours, LPS (055:B5, Difco) is administrated intravenously at the dose of 10 μg/kg body weight (five mice per group). The control group (five mice) receives only the vehicle. After 90 minutes from LPS treatment, under ether anesthesia, blood samples are collected from vein abdominalis with heparin and centrifuged (12000 r.p.m., 3 minutes, 4 °C) to obtain plasma samples. Obtained samples are conserved at -80°C before use. The amount of TNF-α can be quantitatively analyzed by using a commercial ELISA kit (such as, Genzyme/Techne: #10516) and the like.

Toxicity

The toxicity of the compound represented by the formula (I) of the present invention is very low, and thus it is considered that the compound is sufficiently safe to be used as a pharmaceutical.

Application to pharmaceutical

The compound represented by the formula (I), a salt thereof, a solvate thereof, or a prodrug thereof (hereinafter, which may be abbreviated to the compound of the present invention) can be used as an agent for prevention and/or treatment of kinase-related diseases, JNK related diseases, or c-Jun related diseases, for example, metabolic disease (e.g. diabetes mellitus such as insulin-resistant diabetes mellitus or non-insulin-resistant diabetes mellitus, hyperlipemia, other insulin-resistant diseases, and the like), inflammatory diseases (e.g. rhinitis, pharyngitis, bronchitis, pneumonia, pleurisy, bronchial asthma, chronic pulmonary emphysema, pulmonary fibrosis, inflammatory bowel disease, acute pancreatitis, chronic pancreatitis, adult respiratory distress syndrome, chronic thyroiditis, autoimmune gastritis and the like), scleroderma, deep lupus erythematosus, Graves' disease, autoimmune neutropenia, thrombocytopenia, myasthenia gravis, multiple myeloma, acute myeloblasticleukemia, chronicsarcoma, chronic myelocytic leukemia, metastatic melanoma, Kaposi's sarcoma, debilitating disease, Huntington's disease, ischemic/reperfusion disorders of stroke, myocardial ischemic symptom, ischemic heart disease, renal ischemia, neovascular glaucoma, infantile hemangioma, vascular proliferation, cardiac hypertrophy, abnormal immune response, pyrexia, cellular senescence, apoptosis-related diseases and the like, because the compound has outstanding kinase inhibitory activity in mammal (for example, human, non-human animal such as simian, sheep, bos, horse, dog, cat, rabbit, rat, mouse, etc.).

The compound of the present invention can be used as an agent for prevention and/or treatment of TNF-$\alpha$-mediated diseases, for example, inflammatory disease (for example, diabetic complication (e.g. retinopathy, nephropathy, nervous disorder, macrovascular disease, etc.), inflammation, dermatitis, atopic dermatitis, hepatic inflammation, inflammation of the kidneys, glomerulonephritis, pancreatitis, psoriasis, gout, Addison's disease, osteitis syndrome (e.g. osteitis such as osteomyelitis, osteomalacia, periostitis, etc.), arthritis (e.g. rheumatoid arthritis, osteoarthritis, rheumatoid spondylitis, gouty arthritis, synovitis, etc.), inflammatory eye disease, inflammatory pulmonary disease (e.g. chronic pneumonia, pulmonarysilicosis, pulmonarysarcoidosis, lung tuberculosis , adult respiratory distress syndrome (ARDS), severe acute respiratory syndrome (SARS), etc.), inflammatory enteropathy (e.g. Crohn's disease, chronic ulcerative colitis, etc.), allergy disease (e.g. allergic dermatitis, allergic rhinitis, etc.), autoimmune diseases, autoimmune hemolytic anemia, systemic erythematosus, rheumatism, Castleman's disease, immune rejection accompanied with implanting (e.g. graft-versus-host reaction, etc.) and the like); nervous disorder (for example, central nervous system damage (e.g. cerebrovascular disorder such as cerebral hemorrhage and cerebral infarction, etc., head injury, spinal cord injury, brain edema, multiple sclerosis, etc.), neurodegenerative disease (e.g. Alzheimer disease, Parkinson's disease, amyotrophic lateral sclerosis (ALS), AIDS encephalosis, etc.), cerebral meningitis, Creutzfeldt-Jakob disease etc.); pulmonary problems (e.g. asthma, chronic obstructive pulmonary disease (COPD), etc.); circulatory system disease (e.g. angina pectoris, cardiac failure, congestive cardiac failure, acute cardiac failure, chronic cardiac failure, cardiac infarction, acute cardiac infarction, cardiac infarction prognosis, intraatrialmyxoma, arterial sclerosis, hypertension, dialysis hypotension, thrombosis, diffuse intravascular coagulation syndrome (DIC), reperfusion injury, post-PTCA restenosis, etc.); urinary system disease (e.g. renal failure, etc.); metabolic disorder and endocrine disease (e.g. diabetes, hyperlipemia, etc.); bone disease (e.g. osteoporosis, etc.); cancer disease (e.g. malignancy such as growth and metastasis of malignant tumor, etc.), multiple myeloma, plasma cell leukemia, cancerous cachexia, etc.); infectious disease (e.g. virus infection caused by cytomegalovirus, influenza virus, herpes virus, corona virus, etc., cachexia accompanied with infection, cachexia related to acquired immunodeficiency syndrome (AIDS), blood poisoning such as sepsis, septic shock, endotoxic shock, gram-negative sepsis, toxic shock syndrom, severe acute respiratory syndrome (SARS) accompanied with virus infection, etc.); and the like, because the compound has TNF-$\alpha$ production-inhibitoryactivityinmammal (e.g. human, non-human animal (such as, simian, sheep, bos, horse, dog, cat, rabbit, rat, mouse, etc.).

Kinase-related diseases, JNK related disease, c-Jun related disease, and TNF-$\alpha$-mediated diseases are not limited the above described diseases, and include all diseases in which the involvement of those diseases has so far been suggested or will be found afterward.

Furthermore, the compound of the present invention can have the inhibitory activity on eosinophil infiltration according to the kinase inhibitory activity, JNK inhibitory activity, and the TNF-$\alpha$ production-inhibitory activity. By this activity, the compound of the present invention can be used as a remedial agent for nasal obstruction. Therefore, the compound of the present invention can be used as an agent for prevention and/or treatment of eosinophil infiltration-related diseases, for example, chronic urticaria, atopic dermatitis, allergic rhinitis, allergic conjunctivitis, hypersensitivity pneumonitis, eczema, herpetic dermatitis, psoriasis, eosinophilic pneumonia (PIE syndrom), chronic obstructive pulmonary disease (COPD), asthma, contact dermatitis, pruritus, dry dermatitis, acute urticaria, prurigo, etc. and also can be used as a remedial agent for nasal obstruction.

There is no particular limitation on the compound of the present invention, so far as it plays a role to inhibit the function of JNK as protein kinase. Therefore, there is no particular limitation on the specificity to JNK subtype (JNK1, JNK2, JNK3). It is, for example, inhibitory substance for nonspecific JNK including JNK1 (for example, those among the enzyme systems described in the Examples mentioned below, in which deviation of IC50 value between JNK1 and JNK2, JNK1 and JNK3, JNK1 and JNK2 and JNK3 is 10 times or less, etc.), or inhibitory substance specifically for JNK1 (for example, those among the enzyme systems described in the Examples mentioned below, in which deviation of IC50 value between JNK1 and other enzymatic activity is at least 10 times or more, etc.) . Those specifically inhibit JNK1 are especially preferred.

When the compound of the present invention is used for the aforementioned purposes, normally it is administered systemically or locally by oral route or parenteral route.

The compound of the present invention is safe and low in toxicity so that it may be administrated to a mammal including human or a non-human animal (e.g. simian, sheep, bos, horse, dog, cat, rabbit, rat, mouse, etc.).

The doses to be administered are determined depending upon, for example, age, body weight, symptom, the desired therapeutic effect, the route of administration, the duration of the treatment, and the like. For a human adult, generally 1 mg to 1000 mg per dose is orally administered once to several times a day, or 1 mg to 100 mg per dose is parenterally (preferably intravenously) administered once to several times a day, or intravenously administered continuously for 1 to 24 hours a day.

As mentioned above, the doses to be administered depend upon various conditions. Therefore, there may be cases where doses lower than or greater than the ranges specified above are applied.

The compound of the present invention may be safely administered orally or parenterally (e.g. local, rectal, intravenous administration) alone or by mixing with a pharmaceutically acceptable carrier to be made into a medicinal preparation, for example, solid agents for oral administration (for example, tablets including those coated with sugar or film, powder, pills, granules, capsules, etc.), liquid agents for oral administration, injections, suppositories, sustained release drug, etc., in accordance with a known method generally used as a manufacturing method of a medicinal preparation. The amount of the compound of the present invention in such preparations is about 0.01 % of part weight to about 100 % of part weight, preferablely about 0.1 % of part weight to about 50 % of part weight, and more preferably, about 0.5 % of part weight to about 20 % of part weight, relative to the whole of the preparation.

The compound of the present invention used in the production of those medicinal preparations is not limited to substantially pure and single substance, and may include impurities (e.g. by-product, solvent, raw material, etc. which is derived from the production steps) as far as they are pharmaceutically acceptable as pharmaceutical bulk.

The carrier which is used in the production of the medicinal preparation includes various conventional organic or inorganic carrier materials, such as vehicles, lubricants, binders and disintegrants of solid preparation, or solvents, solution adjuvants, suspending or emulsifying agents, tonicity agent, buffering agents and soothing agents, etc. of liquid preparation. If necessary, conventional preservatives, antioxidants, coloring agents, sweetening agents, absorbents, humectants can be used appropriately on adequate dose.

Solid agents for oral administration include tablets, pills, capsules, dispersible powders and granules. Capsules include hard capsules and soft capsules. In such solid agents, one or more of the active compound (s) may be alone, or admixed with vehicles (such as lactose, mannitol, glucose, microcrystalline cellulose, starch, corn starch, light anhydrous silicic acid, etc.), binders (such as hydroxypropyl cellulose, polyvinylpyrrolidone, magnesium metasilicate aluminate, crystalline cellulose, white sugar, D-mannitol, dextrin, hydroxypropylmethyl cellulose, starch, sucrose, gelatin, methylcellulose, sodium carboxymethyl cellulose, etc.), disintegrants (such as cellulose calcium glycolate, starch, carboxymethyl cellulose, carboxymethyl cellulose calcium, sodium carboxymethyl starch, L-hydroxypropyl cellulose, etc.), lubricants (such as magnesium stearate, calcium stearate, tarc, colloidal silica, etc.), and formulated according to common methods. The solid agents may, if desired, be coated with coating agents (such as white sugar, gelatin, hydroxypropyl cellulose or hydroxypropylmethyl cellulose phthalate), or be coated with two or more films. And further, coating may include containment within capsules of absorbable materials such as gelatin.

Liquid agents for oral administration include pharmaceutically acceptable solutions, suspensions, emulsions, syrups, elixirs, etc. In such liquid agents, one or more of the active compound (s) may be dissolved, suspended or emulsified into diluent (s) commonly used in the art (such as purified water, ethanol or a mixture thereof). The liquid agents may further comprise some additives, such as wetting agents, suspending agents, emulsifying agents, sweetening agents, flavoring agents, aroma, preservatives or buffering agents.

Injections for parenteral administration include any types of injections including drops. Examples of injections include intramuscular injections, subcutaneous injections, intradermal injections, intraarterial injections, intravenous injections, intraabdominal injections, intraspinal injections, intravenous drips, etc. Injections for parenteral administration also include sterile aqueous, suspensions, emulsions and solid forms which are dissolved or suspended into solvent (s) for injection immediately before use. In injections, one or more of the active compound (s) may be dissolved, suspended or emulsified into solvent (s) . Examples of the solvents include distilled water for injection, physiological saline, macrogol, vegetable oil (e. g. sesame-seed oil, corn oil, olive oil, etc.), propylene glycol, polyethylene glycol, alcohol such as

ethanol, or a mixture thereof.

Injections may comprise some additives, such as stabilizing agents (e.g. D-sorbitol, D-mannitol, L-alanine, ascorbic acid, albumin, inositol, sodium gluconic acid, sodium thioglycolate, polyoxyethylene hardened castor oil, etc.), solution adjuvants (e.g. glutamic acid, aspartic acid, POLYSORBATE 80 (registered trade mark), polyethylene glycol, propylene glycol, D-mannitol, benzyl benzoate, ethanol, trisaminomethane, cholesterol, triethanolamine, sodium carbonate, sodium citrate, etc.), emulsifying agents or emulsifying agents (e.g. surface-active agents such as stearyltriethanolamine, sodium lauryl sulfate, laurylaminopropionic acid, lecithin, benzalkonium chloride, benzetonium chloride, glycerin monostearate, etc.; hydrophilic polymer such as polyvinyl alcohol, polyvinylpyrrolidone, sodium carboxymethylcellulose, methylcellulose, hydroxymethylcellulose, hydroxyethylcellulose, hydroxypropylcellulose, etc.; and the like), soothing agents (e.g. benzyl alcohol, etc.), tonicity agents (e.g. glucose, D-sorbitol, sodium chloride, glycerin, D-mannitol, etc.), buffering agents (e.g. phosphate buffer, acetate buffer, carbonate buffer, citrate buffer, etc.), preservatives (e.g. parahydroxybenzoate esters, chlorobutanol, benzylalcohol, phenethyl alcohol, dehydroacetate, sorbic acid, etc.), antioxidants (e.g. sulfite salt, ascorbic acid, $\alpha$-tocopherol, etc.), and the like. They may be sterilized at a final step, or may be prepared and compensated according to aseptic manipulations. They may also be manufactured into sterile solid agents, for example, freeze-dried products, which may be dissolved in sterile water or some other sterile diluent (s) for injection immediately before use.

Freeze drying can be carried out by the known method. Generally, a preferable method is to dry by freezing at -25°C or below, and then raising the temperature of a drying rack to 25°C to 40°C, while holding the vacuum pressure of a dry warehouse at about 13.3 Pa or below.

The other preparations for parenteral administration include liquids for external use, ointments, liniments, insufflations, spray preparations, suppositories and pessaries for vaginal administration which comprise one or more of the active substance(s) and may be prepared by methods known per se. Spray preparations may comprise, in addition to a diluent used in general, a stabilizer such as sodium bisulfite and an isotonization buffer, for example, tonicity agents such as sodium chloride, sodium citrate or citric acid. The preparation process of spray preparation is described in detail in, for example, U.S. Patent Nos. 2,868,691 and 3,095,355.

The compound of the present invention may be administered as a combination preparation by combining with other pharmaceuticals for the purpose of

1) supplementating and/or enhancing the preventive and/or treatment effect of the compound,
2) improving pharmacokinetics and absorption of the compound, and reducing the dose of the compound, and/or
3) reducing side effect of the compound.

In addition, the compound of the present invention may be combined and administered as a combination preparation for the purose of

1) supplementing and/or enhancing the preventive and/or treatment effect of the other pharmaceuticals to be combined (hereinafter, which may be abbreviated to a concomintant drug(s)),
2) improving pharmacokinetics and absorption of the concomitant drug(s) and reducing the dose of the concomitant drug(s), and/or
3) reducing side effect of the concomitant drug(s).

The combination preparations of the compound of the present invention and a concomitant drug (s) may be administered as one combination preparation comprising these components, or may be administered separately. When they are administered separately as independent preparations, they may be administered simultaneously or with time lag. Administration with time lag includes the method of administering the compound of the present invention before other drugs and vice versa, and each administration route may be the same or different. There is no limitation on a disease on which the combination preparations of the compound of the present invention and a concomitant drug(s) have preventive and/or treatment effects, so long as the preventive and/or treatment effect of the combination preparation is supplemented and/or enhanced in the disease. There is no limitation on the weight ratio between the compound of the present invention and the concomitant drug (s) in a combined preparation by combining the compound of the present invention with the concomitant drug(s).

Furthermore, the concomitant drug(s) is not limited to a low molecular weight compound, and may be a macromolecule protein, polypeptide, polynucleotide (DNA, RNA, gene), antisense, decoy, antibody, vaccine and the like. The dosage of the concomitant drug (s) can be properly selected according to the clinical dosage. The compounding ratio of the compound of the present invention and the concomitant drug(s) can be properly selected by the age and body weight of the object, administration route, administration term, target disease, symptom, combination and the like. For example, the amount of the concomitant drug (s) may be used 0.01 parts by weight to 100 parts by weight relative to 1 part by weight of the compound of the present invention.

The concomitant drug (s) may be administrated in the proper combination of arbitrary one or two or more member (s) selected from the same or different groups in arbitrary proportion.

The concomitant drug(s) for supplementation and/or enhancement of the prophylactic and/or therapeutic effect of the compound of the present invention includes not only those which have so far been found but also those which will be found on the basis of the aforementioned mechanism. The concomitant drug (s) which can be used in combination with the compounds of the present invention include, for example, those given below.

Examples of the concomitant drug(s) for supplementing and/or enhancing the preventive and/or therapeutic effect of the compound of the present invention on rheumatoid arthritis, osteoarthritis, arthritis includes a steroid, an elastase inhibitor, a cannabinoid-2 receptor stimulator, a prostaglandin, a prostaglandin synthase inhibitor, a phosphodiesterase inhibitor, a metalloproteinase inhibitor, an adhesion molecule inhibitor, an anti-cytokine protein preparation such as an anti-TNF-α preparation, an anti-IL-1 preparation, an anti-IL-6 preparation ; an anti-cytokine agent, an immunomodulatory agent, a disease modifying anti-rheumatic drug, a non-steroidal antiinflammatory drug, a c-Jun N-terminal kinase inhibitor , and the like.

Examples of the concomitant drug(s) for supplementing and/or enhancing the preventive and/or therapeutic effect of the compound of the present invention on inflammatory bowel disorder, Crohn's disease, ulcerative colitis, etc. include a steroid, an elastase inhibitor, a cannabinoid-2 receptor stimulator, a prostaglandin, a prostaglandin synthase inhibitor, a phosphodiesterase inhibitor, a metalloproteinase inhibitor, an adhesion molecule inhibitor, an anti-cytokine protein preparation, an anti-cytokine agent, an immunomodulatory agent, a leukotriene receptor inhibitor, an anticholinergic agent, a 5-lipoxygenase inhibitor, a nitric oxide synthase inhibitor, a interleukin-8 antagonist, a poly(ADP)-ribose polymerase inhibitor, a mitochondrial benzodiazepine receptor agonist, an antioxidant drug, a local anesthetic agent, a gastrointestinal ulcer agent, an enhancing agent of defensive factor, mesalazine, salazosulfapyridine, and the like.

Examples of the concomitant drug(s) for supplementing and/or enhancing the preventive and/or therapeutic effect of the compound of the present invention on asthma, chronic obstructive pulmonary disease, adult respiratory distress syndrome include a steroid, an elastase inhibitor, a cannabinoid-2 receptor stimulator, a prostaglandin, a prostaglandin synthase inhibitor, a phosphodiesterase inhibitor, a metalloproteinase inhibitor, an adhesion molecule inhibitor, a leukotriene receptor inhibitor, an anticholinergic agent, a thromboxane A2 receptor antagonist, a thromboxane synthetase inhibitor, β2-adrenaline receptor stimulator, a xanthine derivative, an expectorant, an antibacterial agent, an anti-histaminic agent, an anti-cytokine protein preparation, an anti-cytokine agent, a forskolin preparation, a mediator release inhibitor, and the like.

Examples of the concomitant drug(s) of the compound of the present invention as a preventive and/or therapeutic agent for hyperlipemia includes, for example, a MTP (Microsomal Triglyceride Transfer Protein) inhibitor, an HMG-CoA reductase inhibitor, a squalene synthetase inhibitor, a fibrate preparation, anACAT (Acyl-CoA: cholesterol O-acyltransferase) inhibitor, a5-lipoxygenase inhibitor, a cholesterol absorption inhibitor, a bile acid absorption inhibitor, a ileum $Na^+$/bile acid cotransporter (IBAT) inhibitor, an LDL receptor activator/enhanced expression, a lipase inhibitor, a probucol preparation, a niacin preparation.

Examples of the concomitant drug(s) of the compound of the present invention as a preventive and/or therapeutic agent for diabetes mellitus (insulin-resistant diabetes mellitus or non-insulin-resistant diabetes mellitus), diabetes complication and the like, include a sulfonylurea hypoglycemic drug, a biguanide preparation, an α-glucosidase inhibitor, a rapid-acting insulin secretagogue, an insulin preparation, a DPP4 (dipeptidyl peptidase) inhibitor, a PTP1B inhibitor, a β3 adrenoceptor agonist, a PPAR (for example, PPARα, PPARγ, PPARδ) agonist, and diabetes complication therapeutic agent and the like.

Examples of the MTP inhibitor include BMS-201038, BMS-212122, BMS-200150, GW-328713, R-103757, and the like.

Examples of the HMG-CoA reductase inhibitor include atorvastatin, fluvastatin, lovastatin, pitavastatin, pravastatin, rosuvastatin, simvastatin, and the like.

Examples of the ACAT inhibitor include F-12511, F-1394, CI-1011, melinamide, FCE27677, RP73163, and the like.

Examples of the squalene synthetase inhibitor include TAK-475 and the like.

Examples of the fibrate preparation include gemfibrozil, clofibrate, bezafibrate, fenofibrate, and the like.

Examples of the cholesterol absorption inhibitor include SCH48461 and the like.

Examples of the bile acid absorption inhibitor include cholestyramine, cholestagel, and the like.

Examples of the LDL receptor activator/enhanced expression agent include MD-700, LY295427, and the like.

Examples of the lipase inhibitor include orlistat and the like.

Examples of the sulfonylurea hypoglycemic agent include acetohexamide, glibenclamide, gliclazide, glyclopyramide, chlorpropamide, tolazamide, tolbutamide, glimepiride, and the like.

Examples of the biguanide preparation include buformin hydrochloride, metformin hydrochloride, and the like.

Examples of a α-glucosidase inhibitor include acarbose, voglibose, and the like.

Examples of the rapid-acting insulin secretagogue include nateglinide, repaglinide, and the like.

Examples of a DPP4 inhibitor include NVP-DPP728A and the like.

Examples of the β3 adrenoceptor agonist include AJ9677, L750355, CP331648, and the like.

Examples of the PPAR agonist include pioglitazone, troglitazone, rosiglitazone, JTT-501, and the like.

Examples of a diabetes complication therapeutic agent include epalrestat and the like.

Examples of the steroidal agent include clobetasol propionate, diflorasone diacetate, fluocinonide, mometasone furoate, betamethasone dipropionate, betamethasone butyrate propionate, betamethasone valerate, difluprednate, diflucortolone valerate, amcinonide, halcinonide, dexamethasone, dexamethasone propionate, dexamethasone valerate, dexamethasone acetate, hydrocortisone acetate, hydrocortisone butyrate, hydrocortisone butyrate propionate, deprodone propionate, prednisolone valerate-acetate, fluocinolone acetonide, beclometasone dipropionate, triamcinolone acetonide, flumetasone pivalate, alclometasone dipropionate, clobetasone butyrate, prednisolone, fludroxycortide, cortisone acetate, hydrocortisone, hydrocortisone sodium phosphate, hydrocortisone sodium succinate, fludrocortisone acetate, prednisolone acetate, prednisolone sodium succinate, prednisolone butylacetate, prednisolone sodium phosphate, halopredone acetate, methylprednisolone, methylprednisolone acetate, methylprednisolone sodium succinate, triamcinolone, triamcinolone acetate, dexamethasone sodium phosphate, dexamethasone palmitate, paramethasone acetate, betamethasone, fluticasone propionate, budesonide, flunisolide, ST-126P, ciclesonide, dexamethasone palomithionate, mometasone furoate, prasterone sulfonate, deflazacort, methylprednisolone suleptanate, methylprednisolone sodium succinate, and the like.

Examples of the elastase inhibitor include ONO-5046, ONO-6818,MR-889, PBI-1101, EPI-HNE-4, R-665, ZD-0892, ZD-8321, GW-311616, DMP-777, L-659286, L-658758, L-680833, L-683845, AE-3763, and the like.

Examples of the prostaglandins (hereinafter, abbreviated as PG) include PG receptor agonists, PG receptor antagonists, and the like.

Examples of the PG receptor include PGE receptors (EP1, EP2, EP3 and EP4), PGD receptors (DP, CRTH2), PGF receptors (FP), PGI receptors (IP), TX receptors (TP), and the like.

Examples of the prostaglandin synthase inhibitor include, salazosulfapyridine, mesalazine, olsalazine, 4-aminosalicylic acid, JTE-522, auranofin, carprofen, diphenpyramide, flunoxaprofen, flurbiprofen, indometacin, ketoprofen, lornoxicam, loxoprofen, meloxicam, oxaprozin, parsalmide, piproxen, piroxicam, piroxicam betadex, piroxicam cinnamate, tropineindometacinate, zaltoprofen, pranoprofen, and the like.

Examples of the phosphodiesterase inhibitor include PDE4 inhibitors such as rolipram, cilomilast (trade name: Ariflo), Bay19-8004, NIK-616, roflumilast (BY-217), cipamfylline (BRL-61063), atizoram (CP-80633), SCH-351591, YM-976, V-11294A, PD-168787, D-4396, IC-485, PDE5 inhibitors such as sildenafil, and the like.

Examples of the adhesion molecule inhibitor include an antagonist such as α4 integrin, and the like.

Examples of the anti-TNF-α preparation include antibody against TNF-α, soluble TNF-α receptor, antibody against TNF-α receptor, soluble TNF-α receptor binding protein, and specifically, infliximab, etanercept, and the like.

Examples of the anti-IL-1 preparation include antibody against IL-1, soluble IL-1 receptor, antibody against IL-1Ra and/or IL-1 receptors, and specifically, for example, anakinra and the like.

Examples of the anti-IL-6 preparation include antibody against IL-6, soluble IL-6 receptor, antibody against IL-6 receptor, and for example, MRA and the like.

Examples of the immunosuppressing agent include methotrexate, cyclosporin, ascomycin, leflunomide, bucillamine, salazosulfapyridine, azathioprine, tacrolimus, cyclophosphamide and the like.

Examples of the disease modifying anti-rheumatic agent include gold thioglucose, sodium aurothiomalate, auranofin, chloroquine, actarit, D-penicillamine preparation, lobenzarit disodium, bucillamine, hydroxychloroquine, salazosulfapyridine, and the like.

Examples of the non-steroidal anti-inflammatory agent include sasapyrine, sodium salicylate, aspirin, aspirin-dialminate, diflunisal, indometacin, suprofen, ufenamate, dimethyl isopropylazulene, bufexamac, felbinac, diclofenac, tolmetin sodium, clinoril, fenbufen, napumetone, proglumetacin, indometacin farnesil, acemetacin, proglumetacin maleate, amfenac sodium, mofezolac, etodolac, ibuprofen, ibuprofen piconol, naproxen, flurbiprofen, flurbiprofenaxetil, ketoprofen, fenoprofen calcium, tiaprofen, oxaprozin, pranoprofen, loxoprofen sodium, alminoprofen, zaltoprofen, mefenamic acid, mefenamic acid aluminium, tolfenamic acid, floctafenine, ketophenylbutazone, oxyphenbutasone, piroxicam, tenoxicam, ampiroxicam, napageln ointment, epirizole, tiaramide hydrochloride, tinoridine hydrochloride, emorfazone, sulpyrine, migrenin, Saridon, Sedes G, Amipylo N, sorbone, pyrin derivatives for cough and cold preparations, acetaminophen, phenacetin, dimetotiazine mesilate, simetride, non-pilin derivatives for cough and cold preparations, and the like.

Examples of the leukotriene receptor antagonist include pranlukast hydrate, montelukast, zafirlukast, seratrodast, MCC-847, KCA-757, CS-615, YM-158, L-740515, CP-195494, LM-1484, RS-635, A-93178, S-36496, BIIL-284, ONO-4057, and the like.

Examples of the anti-choline agent include ipratropium bromide, oxitropium bromide, flutropium bromide, cimetropium bromide, temiverine, tiotropium bromide, revatropate (UK-112166), and the like.

Examples of the topical anesthetics include cocaine hydrochloride, procaine hydrochloride, lidocaine, dibucaine

hydrochloride, tetracaine hydrochloride, and the like.

Examples of the defense factor enhancing agent include sucralfate, aldioxa, teprenone, cetraxate hydrochloride, and the like.

Examples of the thromboxane A2 receptor antagonist include seratrodast, ramatroban, domitroban calcium hydrate, KT-2-962, and the like.

Examples of the thromboxane synthase inhibitor include ozagrel hydrochloride, ozagrel sodium, imitrodast sodium, and the like.

Examples of the β2 adrenaline receptor stimulating agent include fenoterol hydrobromide, salbutamol sulfate, terbutalinesulfate, formoterolfumarate, salmeterolxinafoate, isoproterenol sulfate, orciprenaline sulfate, clorprenaline sulfate, epinephrine, trimetoquinol hydrochloride, hexoprenalinemesyl sulfate, procaterol hydrochloride, tulobuterol hydrochloride, tulobuterol, pirbuterol hydrochloride, clenbuterol hydrochloride, mabuterol hydrochloride, ritodrine hydrochloride, bambuterol, dopexamine hydrochloride, meluadrine tartrate, AR-C68397, levosalbutamol, formoterol, KUR-1246, KUL-7211, AR-C89855, S-1319, and the like.

Examples of the xanthine derivative include aminophylline, theophylline, doxofylline, sipamphylline, diprophylline, and the like.

Examples of the expectorant agent include foeniculated ammonia spirit, sodium hydrogen carbonate, bromhexine hydrochloride, carbocysteine, ambroxol hydrochloride, ambroxol hydrochloride sustained preparation, methylcysteine hydrochloride, acetylcysteine, ethyl L-cysteine hydrochloride, tyloxapol, and the like.

Examples of the antibiotic include sodium cefuroxime, meropenem trihydrate, netilmicin sulfate, sisomicin sulfate, ceftibuten, PA-1806, IB-367, tobramycin, PA-1420, doxorubicin, astromicin sulfate, cefetamet pivoxil hydrochloride, and the like.

Examples of the antibiotic for an inhalant include PA-1806, IB-367, tobramycin, PA-1420, doxorubicin, astromicin sulfate, cefetamet pivoxil hydrochloride, and the like.

Examples of the antihistamine agent include ketotifen fumarate, mequitazine, azelastine hydrochloride, oxatomide, terfenadine, emedastine fumarate, epinastine hydrochloride, astemizole, ebastine, cetirizine hydrochloride, bepotastine, fexofenadine, loratadine, desloratadine, olopatadine hydrochloride, TAK-427, ZCR-2060, NIP-530, mometasone furoate, mizolastine, BP-294, andolast, auranofin, acrivastine, and the like.

Examples of the anti-histamine agent includes ketotifen fumarate, mequitazine, azelastine hydrochloride, oxatomide, terfenadine, emedastine difumarate, epinastine hydrochloride, astemizole, ebastine, cetirizine hydrochloride, bepotastine, fexofenadine, loratadine, desloratadine, olopatadine hydrochloride, TAK-427, ZCR-2060,NIP-530, mometasone furoate, mizolastine, BP-294, andolast, auranofin, acrivastine, and the like.

Examples of the anti-cytokine agent include any non-protein preparation which inhibits the activity of cytokine, for example, MAP kinase inhibitor, gene modulator, cytokine production inhibitor, TNF-α conversion enzyme inhibitor, IL-1β conversion enzyme inhibitor, IL-6 antagonist, IL-8 antagonist, chemokine antagonist, gene therapy agents, antisense compound, and the like.

Examples of the MAP kinase inhibitor include PD-98059 and the like.

Examples of the gene modulator include inhibitors of a molecule which relates to signal transduction, for example, NF-κB, IKK-1, IKK-2, AP-1, and the like.

Examples of the cytokine inhibitor include suplatast tosylate (trade name: IPD), T-614, SR-31747, sonatimod, and the like.

Examples of the chemokine antagonist include ONO-4128 and the like.

Examples of the gene therapy agent include those which is aimed to increase the expression of gene having anti-inflammatory effect, such as interleukin 4, interleukin 10, soluble IL-1 receptor, soluble TNF-α receptor, and the like.

Examples of the mediator release inhibitor include tranilast, sodium cromoglicate, amlexanox, repirinast, ibudilast, dazanolast, pemirolast potassium, and the like.

Examples of the c-Jun N-terminal kinase inhibitor include the compounds described in WO00/35906, WO00/35909, WO00/35921, WO00/64872, WO00/75118, and the like.

The following excellent effects can be obtained by combining the compound of the present invention with the concomitant drug(s).

(1) The concomitant use can decrease the dose compared to administration of the compound alone of the present invention or the concomitant drug(s) alone;

(2) The compound of the present invention and the concomitant drug can be selected according to a patient' s symptom (mild case, severe case etc.);

(3) The selection of the concomitant drug(s) of which mechanism of the action is different from that of the compound of the present invention can decrease the dose in patients and extend the therapeutic period;

(4) The selection of the concomitant drug(s) of which mechanism of the action is different from that of the compound of the present invention can maintain the therapeutic effect;

(5) The combination of the compound of the present invention with the concomitant drug(s) can obtain the synergistic effect.

Especially, in the case that the concomitant drug(s) is a steroid drug, it is possible to take a steroid drug of weak action as compared with administration of the steroid drug alone.

Generally, in the case of the combination of fibrate preparations with a HNG-CoA reductase inhibitor, it is known that rhabdmyolysis may occur as a side effect. However, the incidence and the degree of rhabdmyolysis can be decreased by using the above described concomitant drugs.

Hereinafter, to use the compound of the present invention in conjunction with a concomitant drug(s) is termed "the combination preparation of the present invention". In the case of using the combination preparation of the present invention, there is no particular limitation for administration time of the compound of the present invention or and a concomitant drug(s). The administration of the compound of the present invention or pharmaceutical composition thereof and a concomitant drug(s) or pharmaceutical composition thereof to the administration object includes a simultaneous administration and administrations with time difference. The dose of a concomitant drug can be properly selected according to object of the administration, route of the administration, disease, combination, etc., as far as it conforms to the clinical dose. There is no particular limitation on the way of administration, as far as the compound of the present invention and a concomitant drug(s) are combined in vivo. The way of administration includes, for example, (1) administration of a single preparation obtained by preparing the compound of the present invention and a concomitant drug (s) simultaneously, (2) simultaneous administration of two kind of preparation obtained by preparing the compound of the present invention and a concomitant drug(s) separately by the same route of administration, (3) administrations with time difference of two kind of preparation obtained by preparing the compound of the present invention and a concomitant drug(s) separately by the same route of administration, (4) simultaneous administration of two kind of preparation obtained by preparing the compound of the present invention and a concomitant drug(s) separately by different route of administration, (5) administrations with time difference of two kind of preparation obtained by preparing the compound of the present invention and a concomitant drug (s) separately by different route of administration (such as, administration in the order of the compound of the present invention and a concomitant drug(s), or vice-versa, etc.

In the administration of the combination preparation of the present invention, the concomitant drug(s) of the present invention and/or the concomitant drug(s) can be safely administered as they are or after being mixed with a pharmaceutically acceptable carrier according to a per se known method usually employed in the production of pharmaceutical preparations, orally or parenterally (for example, topical administration, rectal administration, intravenous administration, etc.) in the form of solid preparations for internal use (e.g. tablets including sugar coated tablets and film-coating tablets), powders, pills, granules, capsules, etc.), liquid preparations for internal use, liquid preparations for external use, injections, suppositories, delayed-release preparations or the like.

The carrier which is used in the production of the pharmaceutical preparation includes various conventional organic or inorganic carrier materials, such as excipients, lubricants, binders and disintegrators for solid preparations, solvents, solubilizers, suspending or emulsifying agents, isotonic agents, buffers and soothing agents, etc. If necessary, conventional preservatives, antioxidants, coloring agents, sweetening agents, adsorbents, wetting agents, and the like can be used appropriately in a suitable amount.

The excipient includes, for example, lactose, mannitol, glucose, microcrystalline cellulose, starch, corn starch, light anhydrous silicic acid, and the like. The binder includes, for example, hydroxypropyl cellulose, polyvinylpyrrolidone, magnesium metasilicate aluminate, crystalline cellulose, white soft sugar, D-mannitol, dextrin, hydroxypropylmethyl cellulose starch, sucrose, gelatin, methylcellulose, sodium carboxymethylcellulose, and the like. The disintegrator includes, for example, cellulose calcium glycolate, starch, carboxymethylcellulose, carboxymethylcellulose calcium, sodium carboxymethylstarch, L-hydroxypropylcellulose, and the like. The lubricant includes, for example, magnesium stearate, calcium stearate, talk, colloid silica and the like. The solvent medium includes, for example, distilled water for injection, physiological saline solution, macrogol, vegetable oil (such as sesame oil, corn oil, olive oil), alcohols (e.g. propylene glycol, polyethylene glycol, ethanol, etc.) or a mixture thereof.

The stabilizer includes, for example, D-sorbitol, D-mannitol,L-alanine,ascorbic acid,albumin, inositol, sodium gluconate, sodium thioglycolate, polyoxyethylene hardened caster oil, etc. The solubilizer includes, for example, glutamic acid, aspartic acid , Polysolbate 80 (trade name), polyethylene glycol, propylene glycol, D-mannitol, benzyl benzoate, ethanol, trisaminomethane, cholesterol, triethanolamine, sodium carbonate, citric sodium, etc. The emulsifying or suspending agent includes, for example, surfactants (for example, stearyl triethanolamine, sodium lauryl sulfate, laurylaminopropionate, lecithin, benzalkonium chloride, benzethonium chloride, glyceryl monostearate, etc.), hydrophilic polymers (for example, polyvinyl alcohol, polyvinylpyrrolidone, sodium carboxymethylcellulose, methylcellulose, hydroxymethylcellulose, hydroxyethylcellulose, hydroxypropylcellulose etc.) and the like. The soothing agents include, for example, benzyl alcohol, and the like. The isotonic agents include, for example, glucose, D-sorbitol, sodium chloride, glycerin, D-mannitol, and the like. The buffers include, for example, a buffer solution of phosphates, acetates, carbonates, citrates, or the like. The preservative includes, for example, p-hydroxybenzoic acid ester, chlorobutanol, benzyl alcohol, phenethyl

alcohol, dehydroacetic acid, sorbic acid, and the like. The antioxidant includes, for example, sulfites, ascorbic acid, α-tocopherol, and the like.

The compounding ratio of the compound of the present invention in a combination preparation varies depending on the dosage form. It is usually about 0.01% by weight to 100% by weight relative to the whole preparation, preferably about 0.1% by weight to 50% by weight relative to the whole preparation, more preferably about 0.5% by weight to 20% by weight relative to the whole preparation.

The compounding ratio of the concomitant drug(s) in the combination preparation of the present invention varies depending on the dosage form. It is usually about 0.01% by weight to 100% by weight, preferably about 0.1% by weight to 50% by weight relative to the whole preparation, more preferably about 0.5% by weight to 20% by weight relative to the whole preparation.

The content of the additive such as carrier, etc. in the combination preparation of the present invention varies depending on the dosage form. It is usually about 1% by weight to 99.99% by weight, preferably about 10% by weight to 90% by weight relative to the whole preparation. In addition, it may be the same in the formulation of the compound of the present invention and the concomitant drug(s) independently.

These drug preparations can be prepared by the usual method (such as the method described in Japanese Pharmacopoeia, etc.). The tablet can be prepared by mixing uniformly the compound of the present invention and/or the concomitant drug(s) in the presence or absence of excipients, disintegrators, or other appropriate additives to prepare granulated powder in an appropriate manner, and then compacting with a lubricant, etc. ; by mixing uniformly the compound of the present invention and/or the concomitant drug (s), in the presence or absence of excipients, disintegrators, or other appropriate additives in an appropriate manner, and then compacting the mixture directly; or by optionally adding an appropriate additive to previously granulated powder, mixing the mixture uniformly and then compacting into tablets. If necessary, the tablet may be prepared with coloring agents, flavoring substance, etc. Furthermore, it can be coated by using appropriate coating agents.

The injection preparation can be prepared by the following method. A certain amount of the compounds of the present invention and/or a concomitant drug (s) is dissolved, suspended or emulsified usually in an aqueous medium such as distilled water for injection, physiological saline solution, and Ringer solution, or in a non-aqueous medium such as vegetable oil, etc. ; or a certain amount of the compound of the present invention and/or a concomitant drug(s) is sealed in a container for injection. The carrier for the preparation for oral administration includes a conventional material used in the field of pharmaceutical formulation, such as starch, mannitol, crystalline cellulose, sodium carboxymethylcellulose, etc. The carrier for injections includes, for example, distilled water, physiological saline solution, glucose solution, infusion, and the like.

Although the dose of the combination preparation of the present invention depends on the age, weight, disease symptom, therapeutic effect, administration route, therapy period, and the like, the compound of the present invention and the concomitant drug(s) are usually administered orally once or several times per day at a dose per administration of from 0.1 mg to 1000 mg per human adult, or parenterally (preferably intravenous administration once or several times per day at a dose per administration of from 0.1 mg to 100 mg per human adult, or continuously administered intravenously for 1 hour to 24 hours per day.

It goes without saying that the dose of these compounds may be less than the aforementioned value or may need to exceed the aforementioned range because the dose varies under various conditions as mentioned above. The concomitant drug (s) can be administrated at arbitrary dose as far as the side effect is not a serious problem and the purpose of the present invention can be achieved. The daily dose as a concomitant drug (s) differs depending on age, sex, body weight, different sensitivity, time and interval of administration object, characteristics of pharmaceutical preparation, dispensing, kind, and type of active ingredient of medicinal preparation; and the like, so that it is not particularly limited.

In the mode of administration of the combination preparation of the present invention, the compound of the present invention may be administered simultaneously, or the combination preparation may be administered firstly followed by administering the compound of the present invention, or the compound of the present invention may be administered firstly, followed by administering the concomitant drug(s). In the case of time difference administration, time difference differs depending on active ingredient to be administered, dosage form, and administration route. For example, in the case where the concomitant drug (s) may be administered firstly, the compound of the present invention can be administered within 1 minute to 3 days, preferably 10 minutes to 1 day, more preferably 15 minutes to 1 hour after the administration of the concomitant drug(s) of the present invention.

In the case where the compound of the present invention is administered firstly, the concomitant drug (s) of the present invention can be administered within 1 minute to 1 day, preferably 10 minutes to 6 hours, more preferably 15 minutes to 1 hour after the administration of the compound of the present invention, and the like.

## EFFECT OF THE INVENTION

The c-Jun N terminal kinase inhibitor of the present invention comprising the compound represented by the formula (I), a salt thereof, or a solvate thereof, or a prodrug thereof can be very useful as a preventing and/or treatment for various diseases including, for example, metabolic diseases such as diabetes, inflammatory diseases such as rheumatoid arthritis, and the like, because of inhibitory activity against not only JNK but also TNF-α production, and low toxicity.

## BEST MODE FOR CARRYING OUT THE INVENTION

The following Examples are provided to illustrate the present invention in detail, but are not to be construed as limiting the invention.

## EXAMPLES

Manufacturing Example

Measurement condition for HPLC was conducted as follows unless otherwise stated.
Column used: Xterra (registered trademark) MS $C_{18}$ 5 μm, 4.6 × 50 mm I.D.
Flow rate used: 3 ml/min
Solvents used:

Liquid A: an aqueous solution of 0. 1% trifluoroacetic acid
Liquid B: a solution of 0.1% trifluoroacetic acid-acetonitrile
The mixing ratio of Liquid A and Liquid B was fixed at 95/5 for 0.5 minutes after the initiation of measurement. Thereafter, the mixing ratio of Liquid A and Liquid B was changed linearly to 0/100 over a period of 2.5 minutes. Then, for 3 minutes, the mixing ratio of Liquid A and Liquid B was fixed at 0/100. Thereafter, the mixing ratio of Liquid A and Liquid B was changed linearly to 95/5 over a period of 0.1 minutes. Then, for 3 minutes, the mixing ratio of Liquid A and Liquid B was fixed at 95/5.

Measurement of mass spectrum was performed by ESI under the condition of Pos. 20V, unless otherwise stated.
The solvents in parenthesis show the developing or eluting solvents and the ratios of the solvents used are by volume in chromatographic separations and TLC.
Unless otherwise indicated, the NMR data are $^{1}$H-NMR data (300 MHz). The parentheses in the NMR data mean the solvents used in the measurements.

Example 1:

5-chloro-N-(3-chlorophenyl)pyrazolo[1,5-a]pyrimidin-7-amine

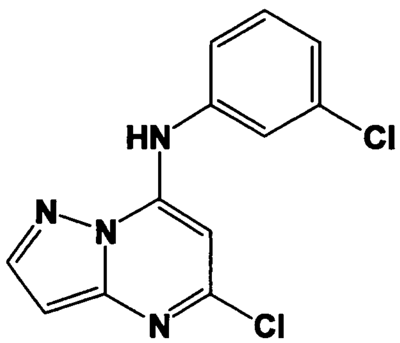

Using 5,7-dichloropyrazolo[1,5-a]pyrimidine (CAS registry: 57489-77-7) and 3-chloroaniline by the same procedure described in Chem. Pharm. Bull., 1999, 47, 928, the compound of the present invention having the following physical data was obtained.
HPLC retention time (minutes): 3.90; Mass data: 279 $(M+H)^+$. Examples 1(1) and 1(2)
The following compounds were obtained, using a corresponding amine compound instead of 3-chloroaniline

by the same procedure as Example 1.

Example 1 (1) :

N-(1,3-benzodioxol-5-ylmethyl)-5-chloropyrazolo[1,5-a]pyrimidin-7-amine
HPLC retention time (minutes): 3.67; Mass data: 303 $(M+H)^+$.

Example 1(2):

2-{4-[(5-chloropyrazolo[1,5-a]pyrimidin-7-yl)amino]phenyl}ethanol
HPLC retention time (minutes): 3.42; Mass data: 289 $(M+H)^+$.

Example 2:

4-[(3-chloro-4-fluorophenyl)amino]-6-methyl-2H-chromen-2-one

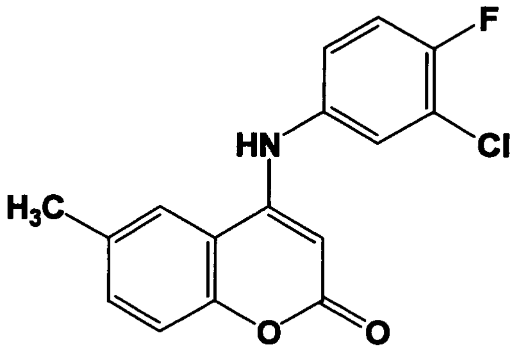

The compound of the present invention having the following physical data was obtained, using 4-chloro-6-methyl-2H-chromen-2-one (CASregistry:51069-75-1) in stead of 5,7-dichloropyrazolo[1,5- a]pyrimidine and 3-chloro-4-fluoroaniline in stead of 3-chloroaniline by the same procedure as Example 1.
NMR ($d_6$-DMSO): $\delta$ 2.41 (s, 3H), 5.28 (s, 1H), 7.27 (d, J=8.2Hz, 1H), 7,39 (ddd, J=9.1, 4.5, 2.5Hz, 1H), 7.48 (m, 1H), 7.51 (t, J=9.1Hz, 1H), 7.60 (dd, J=6.7, 2.5Hz, 1H), 7.99 (d, J=1.8Hz, 1H), 9.26 (s, 1H);
HPLC retention time (minutes): 3.78; Mass data: 607 $(2M+H)^+$, 306, 304 $(M+H)^+$.

Examples 2(1) and 2(2)

The following compounds of the present invention were obtained according to the same procedure as Example 2 using a corresponding chromen compound in stead of 4-chloro-6-methyl-2H-chromen-2-one.

Example 2(1):

4-[(3-chloro-4-fluorophenyl)amino]-8-methyl-2H-chromen-2-one
NMR($d_6$-DMSO): $\delta$ 2.36 (s, 3H), 5.29 (s, 1H), 7.30 (t, J=7.7Hz, 1H), 7,40 (ddd, J=9.0, 4.4, 2.6Hz, 1H), 7.52 (t, J=9.0Hz, 1H), 7.54 (m, 1H), 7.61 (dd, J=6.6, 2.6Hz, 1H), 8.00 (d, J=7.7Hz, 1H), 9.26 (s, 1H);
HPLC retention time (minutes) : 3. 86; Mass data: 609, 607 $(2M+H)^+$, 306, 304 $(M+H)^+$.

Example 2(2) :

4-[(3-chloro-4-fluorophenyl)amino]-2H-chromen-2-one
NMR($d_6$-DMSO): $\delta$ 5.29 (s, 1H), 7.40 (m, 3H), 7.52 (t, J=9.2Hz, 1H), 7.62 (dd, J=6.5, 2.5Hz, 1H), 7.67 (m, 1H), 8.17 (m, 1H), 9.31 (s, 1H);
HPLC retention time (minutes): 3.69; Mass data: 579 $(2M+H)^+$, 292, 290 $(M+H)^+$.

Example 3:

5-thien-3-yl-N-(3,4,5-trimethoxybenzyl)pyrazolo[1,5-a]pyrimidin-7-amine

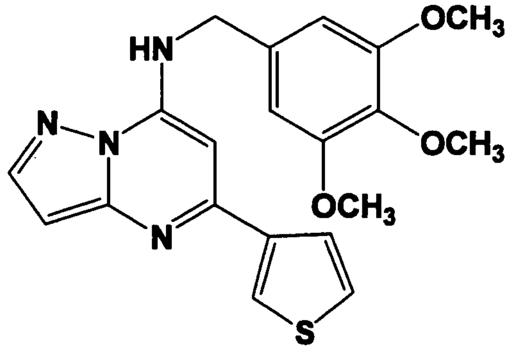

5-chloro-N-(3,4,5-trimethoxybenzyl)pyrazolo[1,5-a]pyrimidin-7-amine was obtained, using (3,4,5-trimethoxybenzyl)amine in stead of 3-chloroaniline by the same procedure as Example 1. The title compound having the following physical data was obtained by coupling between the obtained compound and 3-thienylboronic acid according to the same procedure as Suzuki coupling described in Tetrahedron Letters, 2002, 43, 5739.
HPLC retention time (minutes): 3.27; Mass data: 397 $(M+H)^+$. Examples 3(1) to 3(22)

The following compounds were obtained, using a corresponding amine compound instead of (3,4,5-trimethoxybenzyl)amine, and using a corresponding boronic acid compound instead of thienylboronic acid by the same procedure as Example 3.

Example 3(1):

N-(4-{7-[(4-methoxybenzyl)amino]pyrazolo[1,5-a]pyrimidin-5-yl}phenyl)acetamide
HPLC retention time (minutes): 3.20; Mass data: 388 $(M+H)^+$.

Example 3(2):

5-(2-furyl)-N-(3,4,5-trimethoxybenzyl)pyrazolo[1,5-a]pyrimidine-7-amine
HPLC retention time (minutes): 3.20; Mass data: 381 $(M+H)^+$.

Example 3(3):

5-(4-fluorophenyl)-N-(3,4,5-trimethoxybenzyl)pyrazolo[1,5-a]pyrimidin-7-amine
HPLC retention time (minutes): 3.31; Mass data: 409 $(M+H)^+$.

Example 3(4): 5-(5-methylthien-2-yl)-N-(3,4,5-trimethoxybenzyl)pyrazolo[1,5-a]pyrimidin-7-amine

HPLC retention time (minutes): 3.38; Mass data: 411 $(M+H)^+$.

Example 3(5):

5-(3,4-dimethylphenyl)-N-(pyridin-4-ylmethyl)pyrazolo[1,5-a]pyrimidin-7-amine
HPLC retention time (minutes): 2.99; Mass data: 330 $(M+H)^+$.

Example 3(6):

N-(pyridin-4-ylmethyl)-5-quinolin-3-ylpyrazolo[1,5-a]pyrimidin-7-amine
HPLC retention time (minutes): 2.94; Mass data: 353 $(M+H)^+$.

Example 3(7):

5-(3-fluorophenyl)-N-(3,4,5-trimethoxybenzyl)pyrazolo[1,5-a]pyrimidin-7-amine HPLC retention time (minutes): 3.35; Mass data: 409 $(M+H)^+$.

Example 3(8):

N-(pyridin-4-ylmethyl)-5-thien-3-ylpyrazolo[1,5-a]pyrimidin-7-amine HPLC retention time (minutes): 2.76; Mass data: 308 $(M+H)^+$.

Example 3(9):

N-(4-methoxybenzyl)-5-thien-3-ylpyrazolo[1,5-a]pyrimidin-7-amine HPLC retention time (minutes): 3.34; Mass data: 337 $(M+H)^+$.

Example 3(10):

1-(3-{7-[(4-methoxybenzyl)amino]pyrazolo[1,5-a]pyrimidin-5-yl}phenyl)ethanone HPLC retention time (minutes): 3.34; Mass data: 373 $(M+H)^+$.

Example 3(11):

5-pyridin-4-yl-N-(3,4,5-trimethoxybenzyl)pyrazolo[1,5-a]pyrimidin-7-amine HPLC retention time (minutes): 3.12; Mass data: 392 $(M+H)^+$.

Example 3(12):

5-(3-furyl)-N-(3,4,5-trimethoxybenzyl)pyrazolo[1,5-a]pyrimidin-7-amine HPLC retention time (minutes): 3.23; Mass data: 381 $(M+H)^+$.

Example 3(13):

5-(3-furyl)-N-(thien-2-ylmethyl)pyrazolo[1,5-a]pyrimidin-7-amine HPLC retention time (minutes): 3.23; Mass data: 297 $(M+H)^+$.

Example 3(14):

5-(3-furyl)-N-(3,4,5-trimethoxyphenyl)pyrazolo[1,5-a]pyrimidin-7-amine HPLC retention time (minutes): 3.23; Mass data: 367 $(M+H)^+$.

Example 3(15):

5-(4-methylphenyl)-N-(pyridin-4-ylmethyl)pyrazolo[1,5-a]pyrimidin-7-amine HPLC retention time (minutes): 2.94; Mass data: 316 $(M+H)^+$.

Example 3(16):

5-(3-methoxyphenyl)-N-(pyridin-4-ylmethyl)pyrazolo[1,5-a]pyrimidin-7-amine HPLC retention time (minutes): 2.90; Mass data: 332 $(M+H)^+$.

Example 3(17):

5-(3-furyl)-N-(pyridin-4-ylmethyl)pyrazolo[1,5-a]pyrimidin-7-amine HPLC retention time (minutes): 2.28; Mass data: 292 $(M+H)^+$.

Example 3(18):

N-(4-methoxybenzyl)-5-(4-methylphenyl)pyrazolo[1,5-a]pyrimidin-7-amine

HPLC retention time (minutes): 3.45; Mass data: 345 $(M+H)^+$.

Example 3(19):

N-(4-methoxybenzyl)-5-(3-methoxyphenyl)pyrazolo[1,5-a]pyrim idin-7-amine
HPLC retention time (minutes): 3.42; Mass data: 361 $(M+H)^+$.

Example 3(20):

5-(3-furyl)-N-(4-methoxybenzyl)pyrazolo[1,5-a]pyrimidin-7-amine
HPLC retention time (minutes): 3.31; Mass data: 321 $(M+H)^+$.

Example 3(21):

{1-[5-(3-methoxyphenyl)pyrazolo[1,5-a]pyrimidin-7-yl]pyrrolidin-2-yl}methanol
HPLC retention time (minutes): 3.13; Mass data: 325 $(M+H)^+$.

Example 3(22):

5-(4-methylthien-2-yl)-N-(pyridin-4-ylmethyl)pyrazolo[1,5-a ]pyrimidin-7-amine
HPLC retention time (minutes): 3.01; Mass data: 322 $(M+H)^+$.

Example 4:

$N^5$-[4-(dimethylamino)phenyl]-$N^7$-propylpyrazolo[1,5-a]pyrimidine-5,7-diamine

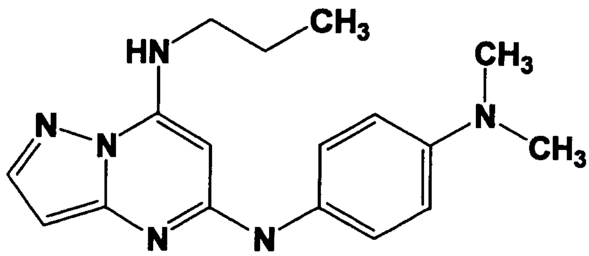

Using propylamine in stead of 3-chloroaniline, 5-chloro-N-propylpyrazolo[1,5-a]pyrimidin-7-amine was obtained by the same procedure as Example 1,. The obtained compound and N,N-dimethylbenzene-1,4-diamine were subjected to nucleophilic aromatic substitution according to the procedure as described in Tetrahedron Letters, 2002, 43, 5739, thereby to give the compound of the present invention having the following physical data.
HPLC retention time (minutes): 2.98; Mass data: 311 $(M+H)^+$.

Example 5:

5-chloro-N-(4-methoxybenzyl)pyrazolo[1,5-a]pyrimidin-7-amine

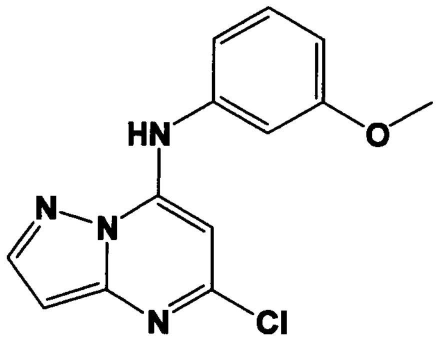

To a solution of 5,7-dichloropyrazolo[1,5- a]pyrimidine (1.8 g) in acetonitrile (30mL) were added p-methoxybenzylamine (1.3 g) and potassium carbonate (2.6 g), and the mixture was stirred overnight at room temperature. After the reaction mixture was concentrated, the residue was extracted with ethyl acetate and water. The extract was washed with a saturated aqueous solution of sodium chloride, dried over anhydrous magnesium sulfate and concentrated. The residue was purified by chromatography on silica gel (hexane : ethyl acetate = 5 : 1 → 3 : 1) to give the compound (2.4 g) of the present invention having the following physical data.
TLC: Rf 0.41 (hexane:ethyl acetate = 2:1);
NMR($CDCl_3$) : δ 3.82 (s, 3 H), 4.51 (d, J=5.49 Hz, 2 H), 5.98 (s, 1H), 6.44 (d, J=2.20 Hz, 1 H), 6.64 - 6.77 (m, 1H), 6.89 - 6.96 (m, 2 H), 7.24 - 7.33 (m, 2 H), 7.96 (d, J=2.20 Hz, 1 H).

Examples 5(1) and 5(2)

Using a corresponding halogeno compound in stead of 5,7-dichloropyrazolo[1,5-a]pyrimidine, the following compounds of the present invention were obtained by the same procedure as Example 5.

Example 5(1):

5-chloro-N-(4-methoxybenzyl)-2-methylpyrazolo[1,5-a]pyrimidin-7-amine
TLC: Rf 0.42 (hexane:ethyl acetate = 2:1);
NMR($CDCl_3$): δ 2.43 (s, 3 H), 3.82 (s, 3 H), 4.49 (d, J=5.68 Hz, 2 H), 5.91 (s, 1 H), 6.22 (s, 1 H), 6.55 - 6.67 (m, 1 H), 6.88 - 6.96 (m, 2 H), 7.23 - 7.34 (m, 2 H).

Example 5(2) :

5-chloro-N-(4-methoxybenzyl)-3-methylpyrazolo[1,5-a]pyrimidin-7-amine
TLC: Rf 0.35 (hexane:ethyl acetate = 4:1);
NMR ($CDCl_3$) : δ 2.29 (d, J=0.73 Hz, 3 H), 3.82 (s, 3 H), 4.50 (d, J=5.67 Hz, 2 H), 5.92 (s, 1 H), 6.58 - 6.67 (m, 1 H), 6.88 - 6.95 (m, 2 H), 7.24 - 7.32 (m, 2 H), 7.79 - 7.83 (m, 1 H).

Example 6:

N-(4-methoxybenzyl)-5-methylpyrazolo[1,5-a]pyrimidin-7-amine

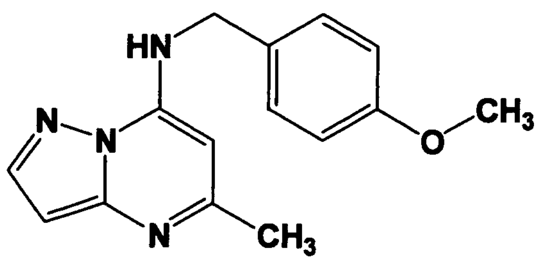

[1,3-bis(diphenylphosphino)propane]dichloronickel(II) (0.1 g) was added to a tetrahydrofuran solution (20 mL) of the

compound (0.5 g) obtained in Example 5, and the mixture was stirred overnight at room temperature. Then a solution of 0. 93 M methylmagnesium bromide in tetrahydrofuran (10 mL) was dropwise added thereto at room temperature and heated under reflux for 2 hours. The reaction mixture was cooled to room temperature, and extracted with a saturated aqueous solution of ammonium chloride and ethyl acetate. The extract was dried over anhydrous magnesium sulfate and concentrated. The residue was purified by chromatography on silica gel (chloroform) to give the compound of the present invention (340 mg) having the following physical data.
TLC: Rf 0.20 (hexane:ethyl acetate = 1:1);
NMR($CDCl_3$) : δ 2. 48 (s, 3 H), 3. 81 (s, 3 H), 4. 50 (d, J=5. 67 Hz, 2 H), 5.83 (s, 1 H), 6.38 (d, J=2.20 Hz, 1H), 6.46 - 6.65 (m, 1 H), 6.90 (d, J=8.60 Hz, 2 H), 7.28 (d, J=8.60 Hz, 2 H), 7.92 (d, J=2.20 Hz, 1 H) .

Examples 6(1) and 6(2)

Using the compounds obtained in Example 5(1) and Example 5(2) instead of the compound obtained in Example 5, the following compounds were obtained by the same procedure as Example 6.

Example 6(1):

N-(4-methoxybenzyl)-2,5-dimethylpyrazolo[1,5-a]pyrimidin-7-amine
TLC: Rf 0.20 (hexane:ethyl acetate = 1:1);
NMR($CDCl_3$) : δ 2. 43 (s, 3 H), 2. 45 (s, 3 H), 3. 81 (s, 3 H), 4.48 (d, J=5.67 Hz, 2 H), 5.76 (s, 1 H), 6.16 (s, 1 H), 6.38 - 6.50 (m, 1 H), 6.90 (d, J=8.78 Hz, 2 H), 7.28 (d, J=8.78 Hz, 2 H).

Example 6(2):

N-(4-methoxybenzyl)-3,5-dimethylpyrazolo[1,5-a]pyrimidin-7-amine
TLC: Rf 0.32 (hexane:ethyl acetate = 2:1);
NMR($CDCl_3$) : δ 2.33 (d, J=0.55 Hz, 3 H), 2.51 (s, 3 H), 3.81 (s, 3 H), 4.50 (d, J=5.67 Hz, 2 H), 5.80 (s, 1 H), 6.40 - 6.50 (m, 1 H), 6.86 - 6.94 (m, 2 H), 7.25 - 7.33 (m, 2 H), 7.77 - 7.81 (m, 1 H).

Example 7:

N-(4-methoxybenzyl)-5-phenylpyrazolo[1,5-a]pyrimidin-7-amine

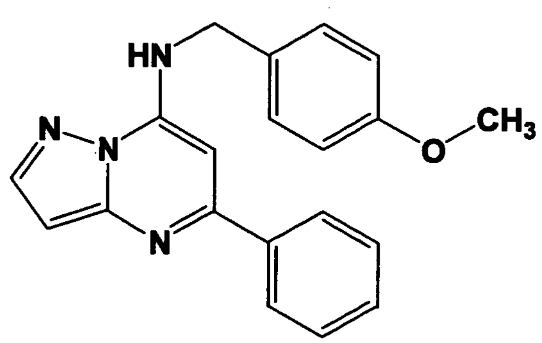

The compound (0.5 g) obtained in Example 5, sodium carbonate (0.34 g), tetrakis(triphenylphosphine)palladium (0) (0.26 g) and phenylbronic acid (0.29 g) were added to toluene (10 mL), and the mixture was heated under reflux overnight at 85°C. After the reaction mixture was cooled, ehyl ether and water were added to the reaction mixture and extracted. The extract was washed with a saturated aqueous solution of sodium chloride, dried over anhydrous magnesium sulfate and concentrated. The resulting residue was purified by chromatography on silica gel (chloroform:ethyl acetate = 50:1) to give the compound of the present invention having the following physical data.
TLC: Rf 0.36 (hexane:ethyl acetate = 2:1);
NMR($CDCl_3$) : δ 3.82 (s, 3 H), 4.61 (d, J=5.68 Hz, 2 H), 6.39 (s, 1 H), 6.57 (d, J=2.20 Hz, 1 H), 6.61 - 6.73 (m, 1H), 6.92 (d, J=8.60 Hz, 2 H), 7.31 - 7.37 (m, J=8.60 Hz, 2 H), 7.39 - 7.52 (m, 3 H), 7.95 - 8.02 (m, 3 H).

Examples 7(1) and 7(2)

Using the compound prepared in Examples 5 (1) and 5 (2) in stead of the compound prepared in Example 5, the compound of the present invention having the following physical data was prepared by the same procedure as Example 7.

Example 7(1):

N-(4-methoxybenzyl)-2-methyl-5-phenylpyrazolo[1,5-a]pyrimidin-7-amine
TLC: Rf 0.34 (hexane:ethyl acetate = 3:1);
NMR($CDCl_3$): δ 2.47 (s, 3 H), 3.81 (s, 3 H), 4.58 (d, J=5.67 Hz, 2 H), 6.31 (s, 1H), 6.33 (s, 1 H), 6.50 - 6.60 (m, 1 H), 6.90 (d, J=8.97 Hz, 2 H), 7.32 (d, J=8.97 Hz, 2 H), 7.39 - 7.50 (m, 3 H), 7.92 - 7.99 (m, 2 H).

Example 7(2):

N-(4-methoxybenzyl)-3-methyl-5-phenylpyrazolo[1,5-a]pyrimidin-7-amine
TLC: Rf 0.36 (hexane:ethyl acetate = 4:1);
NMR($CDCl_3$) : δ 2.40 (s, 3 H), 3. 81 (s, 3 H), 4. 60 (d, J=5. 49 Hz, 2 H), 6.35 (s, 1 H), 6.51 - 6.60 (m, 1 H), 6.88 - 6.95 (m, 2 H), 7.29 - 7.37 (m, 2 H), 7.40 - 7.52 (m, 3 H), 7.85 (s, 1 H), 8.00 - 8.07 (m, 2 H).

Biological Examples:

It was demonstrated, for example, by the following experiments that the compound of the present invention represented by formula (I) has JNK inhibitory activity.
All the procedures were conducted by conventionally used method on the basis of basic biological methods. Furthermore, the measuring method of the present invention was modified to improve the accuracy and/or sensitivity of measurement for evaluating the compound of the present invention. The detailed experimental method was as follows.

Experimental Method

5 μL of kinase buffer (25 mM Tris-HCl (pH 7.5), 5mM β-glycerophosphoric acid, 2 mM dithiothreitol, 0.1 mM $Na_3VO_4$, 10 mM $MgCl_2$) including recombinant human JNK (JNK1: Upstate Biotechnology#14-327, JNK2:Upstate Biotechnology#14-329) was added to well of 384-well plate for fluorescence assay (5 μL) (JNK1: 20 μU/well, JNK2: 50 μU/well). Then, kinase buffer including the compound of the present invention was added into it to carry out incubation for 20 minutes at room temperature. Subsequently, substrate mixture prepared with kinase buffer [biotinylated ATF2 (5 μg/mL) (Upstate Biotechnology #14-432), adenosine triphosphate (JNK1 activity measurement: 2 μmol/L, JNK2 activity measurement: 5μmol/L) (Cell Signaling Technology #9804), anti-phosphorylated ATF2 antibody (20-fold dilution) (Cell Signaling Technology #9221L)] was added to carry out enzyme reaction for 30 minutes at 30°C. After the end of reaction, 5 μL of Hepes buffer (pH7.4) including 0.25% bovine serum albumin (BSA) and 100 mM EDTA was added to carry out stopping of enzyme reaction. The amount of complex between phosphorylated ATF2 and anti-phosphorylated ATF2 was measured with Alpha Screen™ Rabbit Detection Kit (Packard #6760607).
JNK inhibitory activity which is the effect of the compound of the present invention was calculated as inhibition ratio (%) by the following equation:

$$\text{Inhibition ratio (\%)} = [(A_C - A_X)/(A_C - A_B)] \times 100$$

$A_B$: measured value without enzyme;
$A_c$: measured value with enzyme and without a test compound;
$A_x$: measured value with enzyme and a test compound
As a result, all the compound of the present invention showed an inhibition ratio of 50% or more at 10 μmol/L. For example, the compound of Example 1(1) showed an $IC_{50}$ value of 0.33 μM, and the compound of Example 3 showed an $IC_{50}$ value of 0.74 μM.

Formulation Example

Formulation Example 1:

N-(1,3-benzodioxol-5-ylmethyl)-5-chloropyrazolo[1,5-a]pyrimidin-7-amine (5.0 g), carboxymethylcellulose calcium (0.2g), magnesium stearate (0.1 g) and microcrystalline cellulose (4.7 g) were admixed according to a conventional method and punched out to obtain 10,000 tablets each containing 50 mg of the active ingredient.

Formulation Example 2:

N-(1,3-benzodioxol-5-ylmethyl)-5-chloropyrazolo[1,5-a] pyrimidin-7-amine (2.0g), mannitol (20 g) and distilled water (1000 mL) were admixed according to a conventional method, and filtered through a dust filter. The resulting solution was placed 5 mL portions into ampoules, heat sterilized in an autoclave, and then freeze-dried to obtain 10,000 ampoules each containing 20 mg of the active ingredient.

**INDUSTRIAL APPLICABILITY**

Since the compound represented by the general formula (I), a salt thereof, a N-oxide thereof, a solvate thereof or a prodrug thereof, has a kinase (especially c-Jun N-terminal kinase) inhibitory activity, an inhibitory activity of a function of AP-1 as a transcription factor and are furthermore safe, it is useful as a preventive and/or therapeutic agent for, for example, a diabetes of metabolic disease, etc., a rheumatoid arthritis of inflammatory, etc.

**Claims**

1. A compound represented by the formula (I):

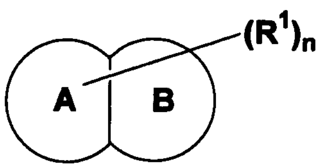

(I)

wherein

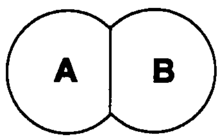

represents a 8- to 10-membered fused heterocyclic ring; $R^1$ represents (1) a hydrogen atom, (2) a halogen atom, (3) a cyano group, (4) an oxo group, (5) an optionally protected hydroxy group, (6) an optionally protected carboxyl group, (7) an optionally protected amino group, (8) a cyclic group which may have a substituent (s), (9) an aliphatic hydrocarbon group which may have a substituent (s), or (10) an optionally protected thiol group; n represents 0 or an integer of 1 to 8; provided that if n represents an integer of not less than 2, the plural $R^1$s are the same or different; or a salt thereof, a solvate thereof or a prodrug thereof.

2. The compound according to claim 1, wherein

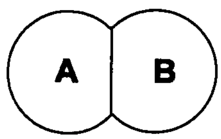

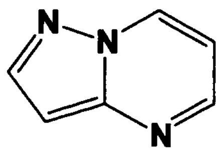 , 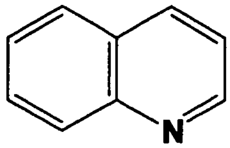 or 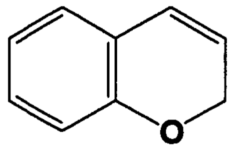 .

3. The compound according to claim 1, wherein the formula (I) is represented by the formula (I-1):

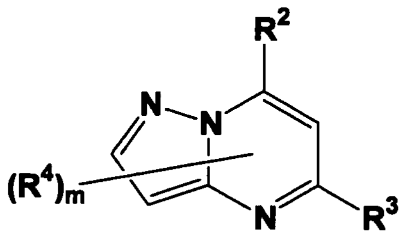

(I-1)

wherein $R^2$ represents an optionally protected amino group or a cyclic group which may have a substituent(s); $R^3$ represents a halogen atom, an optionally protected hydroxy group, an optionally protected thiol group or a cyclic group which may have a substituent (s) ; $R^4$ represents a hydrogen atom, a halogen atom, an optionally protected amino group, an optionally protected hydroxy group, an optionally protected thiol group, a cyclic group which may have a substituent (s) or an aliphatic hydrocarbon group which may have a substituent (s) ; m represents 0 or an integer of 1 to 3; provided that if m represents an integer of not less than 2, the plural $R^4$s are the same or different.

4. The compound according to claim 1, wherein the formula (I) is represented by the formula (I-2):

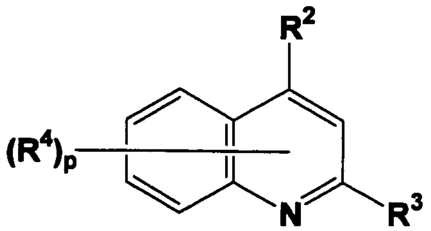

(I-2)

wherein p represents 0 or an integer of 1 to 5; the other symbols represent the same meanings as in claim 3; provided that if p represents an integer of not less than 2, the plural $R^4$s are the same or different.

5. The compound according to claim 1, wherein the formula (I) is represented by the formula (I-3):

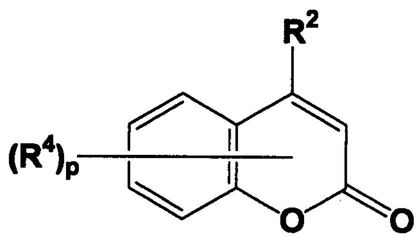

(I-3)

wherein all the symbols represent the same meanings as in claim 3 or 4.

**6.** The compound according to claim 3, 4, or 5, wherein $R^2$ is a protected amino group.

**7.** The compound according to claim 1 selected from the group consisting of

(1) N-(1,3-benzodioxol-5-ylmethyl)-5-chloropyrazolo[1,5-a]pyrimidin-7-amine,
(2) 5-chloro-N-(3-chlorophenyl)pyrazolo[1,5-a]pyrimidin-7-amine,
(3) 5-thien-3-yl-N-(3,4,5-trimethoxybenzyl)pyrazolo[1,5-a]pyrimidin-7-amine,
(4) N-(4-{7-[(4-methoxybenzyl)amino]pyrazolo[1,5-a]pyrimidin-5-yl}phenyl)acetamide,
(5) 2-{4-[(5-chloropyrazolo[1,5-a]pyrimidin-7-yl)amino]phenyl}ethanol,
(6) 5-(2-furyl)-N-(3,4,5-trimethoxybenzyl)pyrazolo[1,5-a]pyrimidin-7-amine,
(7) 5-(4-fluorophenyl)-N-(3,4,5-trimethoxybenzyl)pyrazolo[1,5-a]pyrimidin-7-amine,
(8) 5-(5-methylthien-2-yl)-N-(3,4,5-trimethoxybenzyl)pyrazolo[1,5-a]pyrimidin-7-amine,
(9) 5-(3,4-dimethylphenyl)-N-(pyridin-4-ylmethyl)pyrazolo[1,5-a]pyrimidin-7-amine,
(10) N-(pyridin-4-ylmethyl)-5-quinolin-3-ylpyrazolo[1,5-a]pyrimidin-7-amine,
(11) 5-(3-fluorophenyl)-N-(3,4,5-trimethoxybenzyl)pyrazolo[1,5-a]pyrimidin-7-amine,
(12) N-(pyridin-4-ylmethyl)-5-thien-3-ylpyrazolo[1,5-a]pyrimidin-7-amine,
(13) N-(4-methoxybenzyl)-5-thien-3-ylpyrazolo[1,5-a]pyrimidin-7-amine,
(14) 1-(3-{7-[(4-methoxybenzyl)amino]pyrazolo[1,5-a]pyrimidin-5-yl}phenyl)ethanone,
(15) 5-pyridin-4-yl-N-(3,4,5-trimethoxybenzyl)pyrazolo[1,5-a]pyrimidin-7-amine,
(16) $N^5$-[4-(dimethylamino)phenyl]-$N^{7-}$propylpyrazolo[1,5-a]pyrimidin-5,7-diamine,
(17) 5-(3-furyl)-N-(3,4,5-trimethoxybenzyl)pyrazolo[1,5-a]pyrimidin-7-amine,
(18) 5-(3-furyl)-N-(thien-2-ylmethyl)pyrazolo[1,5-a]pyrimidin-7-amine,
(19) 5-(3-furyl)-N-(3,4,5-trimethoxyphenyl)pyrazolo[1,5-a]pyrimidin-7-amine,
(20) 5-(4-methylphenyl)-N-(4-pyridinylmethyl)pyrazolo[1,5-a]pyrimidin-7-amine,
(21) 5-(3-methoxyphenyl)-N-(4-pyridinylmethyl)pyrazolo[1,5-a]pyrimidin-7-amine,
(22) 5-(3-furyl)-N-(4-pyridinylmethyl)pyrazolo[1,5-a]pyrimidin-7-amine,
(23) N-(4-methoxybenzyl)-5-(4-methylphenyl)pyrazolo[1,5-a]pyrimidin-7-amine,
(24) N-(4-methoxybenzyl)-5-(3-methoxyphenyl)pyrazolo[1,5-a]pyrimidin-7-amine,
(25) 5-(3-furyl)-N-(4-methoxybenzyl)pyrazolo[1,5-a]pyrimidin-7-amine,
(26) {1-[5-(3-methoxyphenyl)pyrazolo[1,5-a]pyrimidin-7-yl]-2-pyrrolidinyl}methanol,
(27) 5-(4-methyl-2-thienyl)-N-(4-pyridinylmethyl)pyrazolo[1,5-a]pyrimidin-7-amine,
(28) 4-[(3-chloro-4-fluorophenyl)amino]-6-methyl-2H-chromen-2-one,
(29) 4-[(3-chloro-4-fluorophenyl)amino]-8-methyl-2H-chromen-2-one,
(30) 4-[(3-chloro-4-fluorophenyl)amino]-2H-chromen-2-one,
(31) 5-chloro-N-(4-methoxybenzyl)pyrazolo[1,5-a]pyrimidin-7-amine,
(32) 5-chloro-N-(4-methoxybenzyl)-2-methylpyrazolo[1,5-a]pyrimidin-7-amine,
(33) 5-chloro-N-(4-methoxybenzyl)-3-methylpyrazolo[1,5-a]pyrimidin-7-amine,
(34) N-(4-methoxybenzyl)-5-methylpyrazolo[1,5-a]pyrimidin-7-amine,
(35) N-(4-methoxybenzyl)-2,5-dimethylpyrazolo[1,5-a]pyrimidin-7-amine,
(36) N-(4-methoxybenzyl)-3,5-dimethylpyrazolo[1,5-a]pyrimidin-7-amine,
(37) N-(4-methoxybenzyl)-5-phenylpyrazolo[1,5-a]pyrimidin-7-amine,
(38) N-(4-methoxybenzyl)-2-methyl-5-phenylpyrazolo[1,5-a]pyrimidin-7-amine, and
(39) N-(4-methoxybenzyl)-3-methyl-5-phenylpyrazolo[1,5-a]pyrimidin-7-amine.

**8.** A pharmaceutical composition which comprises the compound represented by the formula (I) according to claim 1, a salt thereof, a solvate thereof, or a prodrug thereof.

**9.** The pharmaceutical composition according to claim 8, which is a kinase inhibitor.

**10.** The pharmaceutical composition according to claim 9, wherein the kinase is c-Jun N-terminal kinase.

**11.** The pharmaceutical composition according to claim 10, wherein the c-Jun N-terminal kinase is JNK1.

**12.** The pharmaceutical composition according to claim 8, wherein the compound is represented by the formula (I-1), (I-2), or (I-3):

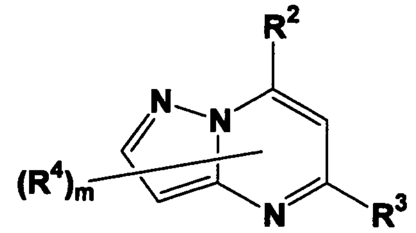

(I-1)

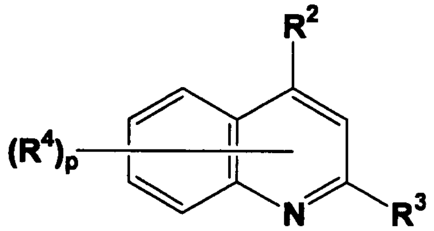

(I-2)

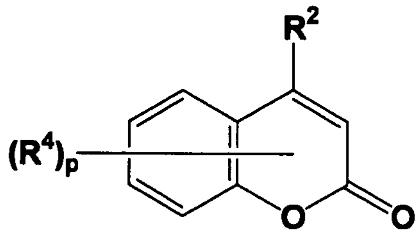

(I-3)

wherein all the symbols represent the same meanings as in claim 3 or 4.

**13.** The composition according to claim 8, wherein the compound is selected from the group consisting of

(1) N-(1,3-benzodioxol-5-ylmethyl)-5-chloropyrazolo[1,5-a]pyrimidin-7-amine,
(2) 5-chloro-N-(3-chlorophenyl)pyrazolo[1,5-a]pyrimidin-7-amine,
(3) 5-thien-3-yl-N-(3,4,5-trimethoxybenzyl)pyrazolo[1,5-a]pyrimidin-7-amine,
(4) N-(4-{7-[(4-methoxybenzyl)amino]pyrazolo[1,5-a]pyrimidin-5-yl}phenyl)acetamide,
(5) 2-{4-[(5-chloropyrazolo[1,5-a]pyrimidin-7-yl)amino]phenyl}ethanol,
(6) 5-(2-furyl)-N-(3,4,5-trimethoxybenzyl)pyrazolo[1,5-a]pyrimidin-7-amine,
(7) 5-(4-fluorophenyl)-N-(3,4,5-trimethoxybenzyl)pyrazolo[1,5-a]pyrimidin-7-amine,
(8) 5-(5-methylthien-2-yl)-N-(3,4,5-trimethoxybenzyl)pyrazolo[1,5-a]pyrimidin-7-amine,
(9) 5-(3,4-dimethylphenyl)-N-(pyridin-4-ylmethyl)pyrazolo[1,5-a]pyrimidin-7-amine,
(10) N-(pyridin-4-ylmethyl)-5-quinolin-3-ylpyrazolo[1,5-a]pyrimidin-7-amine,
(11) 5-(3-fluorophenyl)-N-(3,4,5-trimethoxybenzyl)pyrazolo[1,5-a]pyrimidin-7-amine,
(12) N-(pyridin-4-ylmethyl)-5-thien-3-ylpyrazolo[1,5-a]pyrimidin-7-amine,
(13) N-(4-methoxybenzyl)-5-thien-3-ylpyrazolo[1,5-a]pyrimidin-7-amine,
(14) 1-(3-{7-[(4-methoxybenzyl)amino]pyrazolo[1,5-a]pyrimidin-5-yl}phenyl)ethanone,
(15) 5-pyridin-4-yl-N-(3,4,5-trimethoxybenzyl)pyrazolo[1,5-a]pyrimidin-7-amine,
(16) N$^{5}$-[4-(dimethylamino)phenyl]-N$^{7}$-propylpyrazolo[1,5-a]pyrimidin-5,7-diamine,
(17) 5-(3-furyl)-N-(3,4,5-trimethoxybenzyl)pyrazolo[1,5-a]pyrimidin-7-amine,
(18) 5-(3-furyl)-N-(thien-2-ylmethyl)pyrazolo[1,5-a]pyrimidin-7-amine,
(19) 5-(3-furyl)-N-(3,4,5-trimethoxyphenyl)pyrazolo[1,5-a]pyrimidin-7-amine,
(20) 5-(4-methylphenyl)-N-(4-pyridinylmethyl)pyrazolo[1,5-a]pyrimidin-7-amine,
(21) 5-(3-methoxyphenyl)-N-(4-pyridinylmethyl)pyrazolo[1,5-a]pyrimidin-7-amine,
(22) 5-(3-furyl)-N-(4-pyridinylmethyl)pyrazolo[1,5-a]pyrimidin-7-amine,
(23) N-(4-methoxybenzyl)-5-(4-methylphenyl)pyrazolo[1,5-a]pyrimidin-7-amine,
(24) N-(4-methoxybenzyl)-5-(3-methoxyphenyl)pyrazolo[1,5-a]pyrimidin-7-amine,
(25) 5-(3-furyl)-N-(4-methoxybenzyl)pyrazolo[1,5-a]pyrimidin-7-amine,

(26) {1-[5-(3-methoxyphenyl)pyrazolo[1,5-a]pyrimidin-7-yl]-2-pyrrolidinyl}methanol,
(27) 5-(4-methyl-2-thienyl)-N-(4-pyridinylmethyl)pyrazolo[1,5-a]pyrimidin-7-amine,
(28) 4-[(3-chloro-4-fluorophenyl)amino]-6-methyl-2H-chromen-2-one,
(29) 4-[(3-chloro-4-fluorophenyl)amino]-8-methyl-2H-chromen-2-one,
(30) 4-[(3-chloro-4-fluorophenyl)amino]-2H-chromen-2-one,
(31) 5-chloro-N-(4-methoxybenzyl)pyrazolo[1,5-a]pyrimidin-7-amine,
(32) 5-chloro-N-(4-methoxybenzyl)-2-methylpyrazolo[1,5-a]pyrimidin-7-amine,
(33) 5-chloro-N-(4-methoxybenzyl)-3-methylpyrazolo[1,5-a]pyrimidin-7-amine,
(34) N-(4-methoxybenzyl)-5-methylpyrazolo[1,5-a]pyrimidin-7-amine,
(35) N-(4-methoxybenzyl)-2,5-dimethylpyrazolo[1,5-a]pyrimidin-7-amine,
(36) N-(4-methoxybenzyl)-3,5-dimethylpyrazolo[1,5-a]pyrimidin-7-amine,
(37) N-(4-methoxybenzyl)-5-phenylpyrazolo[1,5-a]pyrimidin-7-amine,
(38) N-(4-methoxybenzyl)-2-methyl-5-phenylpyrazolo[1,5-a]pyrimidin-7-amine, and
(39) N- (4-methoxybenzyl) -3-methyl-5-phenylpyrazolo[1,5-a]pyrimidin-7-amine.

**14.** The pharmaceutical composition according to claim 10, which is a preventive and/or therapeutic agent for c-Jun N-terminal kinase-mediated diseases.

**15.** The pharmaceutical composition according to claim 14, wherein the c-Jun N-terminal kinase-mediated diseases are metabolic diseases or inflammatory diseases.

**16.** The pharmaceutical composition according to claim 15, wherein the metabolic disease is diabetes mellitus.

**17.** The pharmaceutical composition according to claim 16, wherein the diabetes mellitus is insulin-resistant diabetes mellitus.

**18.** The pharmaceutical composition according to claim 15, wherein the inflammatory diseases are osteitis.

**19.** The pharmaceutical composition according to claim 18, wherein the osteitis is arthritis.

**20.** A method for inhibiting c-Jun N-terminal kinase, which comprises administering to a mammal an effective amount of the compound according to claim 1, a salt thereof, a solvate thereof or a prodrug thereof.
method for preventing and/or treating c-Jun kinase-mediated diseases in a mammal, which administering to a mammal an effective amount of the according to claim 1, a salt thereof, a solvate thereof ug thereof.
e of the compound according to claim 1, a salt thereof, hereof or a prodrug thereof, for the manufacture of ve and/or therapeutic agent for c-Jun N-terminal iated diseases.
pharmaceutical composition which comprises a a of the compound according to claim 1, a salt thereof, thereof or a prodrug thereof and one or two or more is selected from the group consisting of an MTP an HMG-CoA reductase inhibitor, a squalene inhibitor, a fibrate preparation, an ACAT inhibitor, enase inhibitor, a cholesterol absorption inhibitor, id absorption inhibitor, a ileum $Na^+$/bile acid ter inhibitor, an LDL receptor activator/expression a lipase inhibitor, a probucol preparation, a cid preparation, a hypoglycemic sulfonylurea agent, ie preparation, an $\alpha$-glucosidase inhibitor, a ng insulin secretagogue, an insulin preparation, a tor, a PTP1B inhibitor, a $\beta$3 adrenoceptor agonist, gonist, and a therapeutic agent for diabetes ons.

**INTERNATIONAL SEARCH REPORT**

International application No.
PCT/JP2004/014941

A. CLASSIFICATION OF SUBJECT MATTER
Int.Cl[7] C07D311/08, 487/04, A61K45/00, 31/519, A61P43/00, 3/10, 29/00, 19/08, 19/02

According to International Patent Classification (IPC) or to both national classification and IPC

B. FIELDS SEARCHED

Minimum documentation searched (classification system followed by classification symbols)
Int.Cl[7] C07D311/08, 487/04, A61K45/00, 31/519, A61P43/00, 3/10, 29/00, 19/08, 19/02

Documentation searched other than minimum documentation to the extent that such documents are included in the fields searched

Electronic data base consulted during the international search (name of data base and, where practicable, search terms used)
REGISTRY, CAPLUS, BIOSIS, MEDLINE, EMBASE(STN)

C. DOCUMENTS CONSIDERED TO BE RELEVANT

| Category* | Citation of document, with indication, where appropriate, of the relevant passages | Relevant to claim No. |
|---|---|---|
| X | WO 2002/083648 A1 (Eisai Co., Ltd.), 24 October, 2002 (24.10.02), Full text; particularly, Claims; example I-173 & US 2004/127538 A1 | 1,2,8-11, 14-16,18,19, 22 |
| X | Darren Krause et al., 'Transient activation of Jun N-terminal kinases and protection from apoptosis by the insulin-like growth factor I receptor can be suppressed by dicumarol.', J.Biol.Chem., 01 June, 2001 (01.06.01), Vol.276, No.22, pages 19244 to 19252 | 1,2,8-10,14, 22 |

[X] Further documents are listed in the continuation of Box C.

[ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 December, 2004 (06.12.04) | 21 December, 2004 (21.12.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/014941

C (Continuation). DOCUMENTS CONSIDERED TO BE RELEVANT

| Category* | Citation of document, with indication, where appropriate, of the relevant passages | Relevant to claim No. |
|---|---|---|
| X<br><br>Y<br>A | WO 2000/064872 A1 (VERTEX PHARMACEUTICALS INC.),<br>02 November, 2000 (02.11.00),<br>Full text; particularly, Claims; examples 170, 175; pages 108 to 109, 116 to 117<br>& US 2003/153560 A1 & EP 1175399 A1 | 1,2,8-10,<br>14-19,22<br>23<br>3,5-7,11-13 |
| X | WO 2003/059900 A1 (Takeda Chemical Industries, Ltd.),<br>24 July, 2003 (24.07.03),<br>Full text; particularly, Claims; examples<br>& JP 2003-267965 A | 1,2,5,8,12,<br>23 |
| P,X | WO 2004/081013 A1 (TEIJIN PHARMA LTD.),<br>23 September, 2004 (23.09.04),<br>Full text; particularly, Claims; examples<br>(Family: none) | 1,2,8,9,<br>16-19 |
| P,X | WO 2004/076458 A1 (TEIJIN PHARMA LTD.),<br>10 September, 2004 (10.09.04),<br>Full text; particularly, Claims; examples<br>(Family: none) | 1,2,8,9,<br>16-19 |
| P,X | WO 2004/087707 A1 (VERNALIS (CAMPRIDGE) LTD.),<br>14 October, 2004 (14.10.04),<br>Full text; particularly, Claims; examples<br>(Family: none) | 1-3,6,8,9,12 |
| P,X | US 2004/209878 A1 (Timothy J. Guzi),<br>21 October, 2004 (21.10.04),<br>Full text; particularly, Claims; examples<br>(Family: none) | 1-3,6,8,9,12 |
| P,X | WO 2004/026229 A2 (SCHERING CORP.),<br>01 April, 2004 (01.04.04),<br>Full text; particularly, Claims; examples<br>& US 2004/106624 A1 | 1-3,6,8,9,12 |

Form PCT/ISA/210 (continuation of second sheet) (January 2004)

**INTERNATIONAL SEARCH REPORT**

International application No.
PCT/JP2004/014941

**Box No. II Observations where certain claims were found unsearchable (Continuation of item 2 of first sheet)**

This international search report has not been established in respect of certain claims under Article 17(2)(a) for the following reasons:

1. [X] Claims Nos.: 20, 21
because they relate to subject matter not required to be searched by this Authority, namely:
Claims 20 and 21 pertain to methods for treatment of the human body by surgery or therapy.

2. [X] Claims Nos.: 4
because they relate to parts of the international application that do not comply with the prescribed requirements to such an extent that no meaningful international search can be carried out, specifically:
The compound of claim 4 is not sufficiently supported by the description.

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
A matter common to the subject matters of claims 1-19, 22, and 23 is a compound having an 8- to 10-membered fused heterocyclic structure. However, the structure itself has conventionally been known as a basic skeleton for heterocyclic compounds and is shown in, e.g., the documents 1 to 4 which will be given later. It is hence obvious that the matter is not novel.
As a result, that common matter is not a special technical feature in the meaning of the second sentence of Rule 13.2 of the Regulations under the PCT. Any other common matter is not considered to be a special technical feature. No technical relationship in the meaning of Rule 13 of the Regulations under (continued to extra sheet)

1. [ ] As all required additional search fees were timely paid by the applicant, this international search report covers all searchable claims.

2. [X] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (2)) (January 2004)

**INTERNATIONAL SEARCH REPORT**

International application No.
PCT/JP2004/014941

Continuation of Box No.III of continuation of first sheet(2)

the PCT can be found among the different subject matters. Consequently, claims 2-19, 22, and 23 do not comply with the requirement of unity of invention.

With respect to claims 1-3, 5, 8-12, 14-19, 22, and 23, these claims relate to compounds themselves including a wide range of compounds, a use of the compounds, a composition thereof, etc. However, as stated above with respect to claim 4, the compounds represented by the general formula I-2 are not sufficiently supported by the description. Consequently, the compounds having a quinazoline skeleton, of these claims, were excluded from the subject matters for search.

Furthermore, of the claimed compounds having other skeletons, the compounds which are supported by the description in the meaning of Article 6 of the PCT and are disclosed in the meaning of Article 5 of the PCT are limited to compounds in which the substituent R2 is bonded through a nitrogen atom. Consequently, a search was made mainly for compounds in which the substituent R2 is bonded through a nitrogen atom, of the compounds represented by the general formulae I-1 and I-3.

A complete search was made with respect to claims 7 and 13.

With respect to the medical applications of claims 14-19 and 23, no concrete data showing effectiveness in the medical applications are given in the description. These claims are not sufficiently supported by the description. In particular, persons skilled in the art cannot understand that all the compounds in a wide range shown in the claims are effective in the medical applications.

Form PCT/ISA/210 (extra sheet) (January 2004)